(19) Europäisches Patentamt
European Patent Office
Office européen des brevets

(11) Publication number: **0 322 862 A2**

# (12) EUROPEAN PATENT APPLICATION

(21) Application number: 88121725.1

(22) Date of filing: 27.12.88

(51) Int. Cl.4: C07C 69/92 , C07C 69/90 , C07C 69/708 , C07C 69/76 , C07C 69/24 , C07C 69/96 , C07C 69/82 , C07C 43/13 , C07C 43/205 , C07C 59/125 , C07D 239/34

(30) Priority: 24.12.87 JP 325464/87
01.04.88 JP 78039/88

(43) Date of publication of application:
05.07.89 Bulletin 89/27

(84) Designated Contracting States:
CH DE FR GB LI

(71) Applicant: **HITACHI, LTD.**
6, Kanda Surugadai 4-chome Chiyoda-ku
Tokyo(JP)

Applicant: **Takeda Chemical Industries, Ltd.**
27, Doshomachi 2-chome Higashi-ku
Osaka-shi Osaka, 541(JP)

(72) Inventor: Kitamura, Teruo
3600-234, Nakane
Katsuta-shi(JP)
Inventor: Kondo, Katsumi
19-21, Aobacho
Katsuta-shi(JP)
Inventor: Mukoh, Akio
498-21, Kasaharacho
Mito-shi(JP)
Inventor: Matsumura, Koichi
12-9, Teradacho
Ibaraki-shi(JP)
Inventor: Kawada, Mitsuru
25-3, Tsukaguchicho-4-chome
Amagasaki-shi(JP)
Inventor: Sugihara, Yoshihiro
4-5, Tamaicho-3-chome
Toyonaka-shi(JP)

(74) Representative: Patentanwälte Beetz sen. - Beetz jun. Timpe - Siegfried - Schmitt-Fumian
Steinsdorfstrasse 10
D-8000 München 22(DE)

## (54) Optically active compounds, their preparation and their use in liquid crystal compositions and liquid crystal optical modulators.

(57) The invention relates to optically active compounds represented by the general formula I

$$R_1-Q_1-M-Q_2-\underset{\substack{| \\ R_2}}{\overset{*}{C}H}-(CH_2)_n-\underset{\substack{| \\ R_3}}{\overset{*}{C}H}-Q_3-R_4 \qquad (I),$$

wherein n, $R_1$, $R_2$, $R_3$, $R_4$, $Q_1$, $Q_2$, $Q_3$, and M are defined as in the specification, methods and intermediates for their preparation, liquid crystal compositions comprising at least one optically active compound of formula I and their use in electrooptical display, switching and modulation devices.

EP 0 322 862 A2

# OPTICALLY ACTIVE COMPOUNDS, THEIR PREPARATION AND THEIR USE IN LIQUID CRYSTAL COMPOSITIONS AND LIQUID CRYSTAL OPTICAL MODULATORS

The present invention relates to optically active compounds, intermediates, methods for their preparation and to liquid crystal compositions containing these compounds, particularly for use in electrooptical display devices, switching devices and modulators. The compounds and compositions of the present invention show a ferroelectric liquid crystal phase, and accordingly are useful as electrooptic switching elements such as liquid crystal display devices or the like, and can be used in liquid crystal optical modulators.

Liquid crystal display devices have various excellent features such as low-voltage operability, low power consumption, being thin and light-weight, good recognizability etc. Accordingly, they are in wide use as various display devices.

Liquid crystal display devices using a nematic liquid crystal operating in the so-called twisted nematic mode (TN mode) are in use currently. However, display devices using this kind of nematic liquid crystals have the drawback of being very slow in response as compared to luminescent type display devices such as CRT, EL and the like. Consequently, when such display devices are applied in a display device, particularly a large scale display device capable of displaying a large amount of information, it is impossible to obtain a display of good contrast. Thus the liquid crystal display devices using a nematic liquid crystal have only a very limited applicability. There has recently been developed a liquid crystal device using a nematic liquid crystal operating in the so-called super twisted nematic type (STN mode) or SBE and capable of giving a display of improved contrast. Even in this STN type liquid crystal display device, however, the response is not sufficient, and therefore said device finds also only a very limited application, because it cannot be used for displays capable of displaying a still larger amount of information. Hence, various attempts have been made to develop a new liquid crystal display system giving an excellent response.

Ferroelectric liquid crystals have a memory function and give a high speed response, and accordingly they are suited for large scale displays.

As liquid crystals having ferroelectric properties, there are known those showing a chiral smectic C phase, a chiral smectic H phase, a chiral smectic J phase, etc. Of these ferroelectric liquid crystals, those showing a chiral smectic C phase are thought to have highest practical utility.

Ferroelectric liquid crystals showing a chiral smectic C phase were first synthesized in 1975 by R. B. Meyer et al.; one typical example thereof is 2-methylbutyl-4-(4′-n-decyloxybenzylideneamino)-cinnamate (hereinafter abbreviated to DOBAMBC) [J. Physique, 36, L-69 (1975)].

A thin film liquid crystal cell was prepared using DOBAMBC and was found to have a high speed response in the order of $\mu$s [N. A. Clark et al., Appl. Phys. Lett., 36, 89 (1980)]. Since that time, there was started the development of optical modulation devices (e.g. liquid crystal devices, photo-printer heads) using a ferroelectric liquid crystal showing a chiral smectic C phase (hereinafter may be referred to simply as "ferroelectric liquid crystal").

As a result, a number of ferroelectric liquid crystal compounds showing a chiral smectic C phase have been developed since then, and various ferroelectric liquid crystal compounds are already known. However, no ferroelectric liquid crystal compound is found yet which has satisfactory reliability and capability for use in display devices, particularly large scale displays, etc.

In order for a ferroelectric liquid crystal to be practically used in a liquid crystal display device, etc., the liquid crystal must be superior in high speed response, orientation, memory function, threshold, temperature dependences of these properties, etc. Also, the ferroelectric liquid crystal is required to show a chiral smectic C phase over a wide temperature range so that it can operate within a sufficiently wide temperature range including room temperature, and further to have excellent physical and chemical stability.

In order for a ferroelectric liquid crystal to have, in particular, excellent physical and chemical stability, good high speed response and good memory function, the liquid crystal must have a large spontaneous polarization.

Among the so far developed ferroelectric liquid crystals, no compound is found yet which satisfies the above requirements. For example, the above mentioned DOBAMBC, being a liquid crystal of Schiff's base type, is insufficient in chemical stability to water, light, etc., and moreover, has a small spontaneous polarization of 4 $nC/cm^2$ or below.

Ester type liquid crystals are reported as being ferroelectric liquid crystals which are chemically stable. However, these liquid crystals are not satisfactory because they have no sufficiently large spontaneous

polarization and no sufficiently wide temperature range of chiral smectic C phase.

In order to obtain a large spontaneous polarization, there were synthesized compounds having two asymmetric carbon atoms as an optically active group essential for the expression of a chiral smectic C phase.

These compounds include, for example, liquid crystal compounds having a dichiral epoxide side chain [David M. Walba et al., Journal of American Chemical Society, 108, 7424 (1986)], and liquid crystal compounds having a halogen atom and a methyl group on two adjacent asymmetric carbon atoms [cf. eg. JP- A- 168780/1985, 218358/1985, 68449/1986, 40/1987, 46/1987, 103043/1987, 111950/1987, 142131/1987, 175443/1987].

A typical example of the above liquid crystal compounds is (s)-3-methyl-2-chlorobutyl-4-(4′-octylcarbonyloxy)-diphenylcarboxylate JP-A-68449/1986]. This liquid crystal compound has a very large spontaneous polarization of 180 $nC/cm^2$ but, being an aliphatic chloro compound, has poor chemical stability. Hence, there was synthesized 4′-octylcarbonyloxy-4-[ (s)-2-methoxy-(s)-3-methylpentyloxycarbonyl]-diphenyl [JP-A-228036/1987]. This compound has excellent chemical stability but has an insufficient spontaneous polarization of 17 $nC/cm^2$.

It is the object of the present invention to provide ferroelectric optically active liquid crystal compounds having excellent physical and chemical stability and a large spontaneous polarization which can be used in electrooptical display, switching and/or modulation devices, methods and intermediates for their preparation, and corresponding liquid crystal compositions and their use. This object is achieved according to claims 1 and 12 to 17. The dependent claims relate to preferred embodiments.

According to the concept of the present invention a chemically stable ester compound is combined with an optically active group having two asymmetric carbon atoms.

Specifically, the present invention has been achieved by using, as an optically active group having two asymmetric carbon atoms, a structure containing no halogen-carbon bond which is contained in conventional liquid crystal compounds and gives poor chemical stability, and bonding said structure to (a) a six-membered ring (e.g. benzene) constituting the skeleton of the liquid crystal compounds and also to (b) an alkyl group constituting one terminal group of said liquid crystal compound, in a particular pattern.

The figure is a schematic illustration of an example of a liquid crystal display device using the liquid crystal compositions of the present invention, which comprises as may be seen from the drawing, a polarizing plate, a backglass substrate, a transparent electrode provided thereon, a front glass provided with a transparent signal electrode, a ferroelectric liquid crystal sandwiched between the backglass substrate and the front glass which are sealed together, and a polarizing plate provided on the front glass.

The optically active compounds according to the present invention are represented by the general formula I:

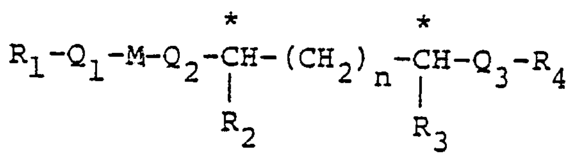

[I]

wherein n is 0 or 1 (when n is 0, $-(CH_2)_n-$ is a single bond), $R_1$ is an alkyl group of 3-14 carbon atoms, $R_2$ and $R_3$ are independently a lower alkyl group of 1-3 carbon atoms, $R_4$ is an alkyl group of 1-10 carbon atoms, $Q_1$, $Q_2$ and $Q_3$ are independently a single bond, an ether bond, a carboxylic acid ester bond, a carbonyl bond or a carbonyldioxy bond, M is

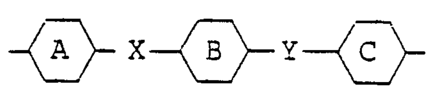

or

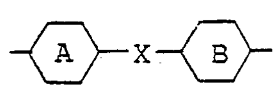

(X and Y are independently a single bond, a carboxylic acid ester bond, a methyleneoxy bond or an

ethylene bond, and

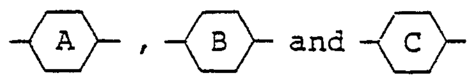

are independently a homocyclic or heterocyclic six-membered ring-1,4-diyl group which may contain 1-2 oxygen or nitrogen atoms as ring-forming atoms.

The *-marked carbon atoms are asymmetric carbon atoms. The liquid crystal compositions of the present invention comprise at least one optically active compound of the general formula I.

The concept of the present invention further comprises the use of the optically actice compounds of formula I and the respective liquid crystal compositions in electrooptical devices, particularly display devices, switching devices and modulator devices. It is particularly directed to liquid crystal optical modulators using a liquid crystal composition according to the invention.

The compounds I according to the present invention can be classified into the following compounds I′ and I″ depending upon the basic skeleton M.

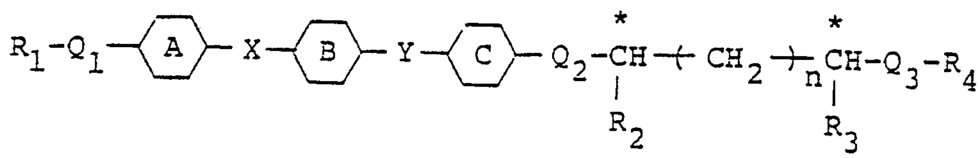

[I']

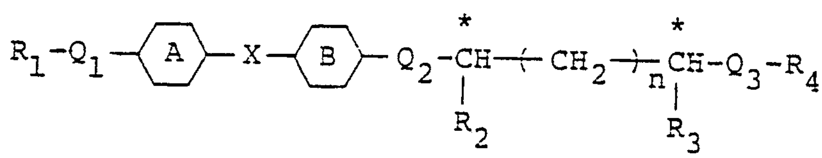

[I"]

In the above compounds, I, I′ and I″, the alkyl group of 3-14 carbon atoms represented by $R_1$ can be of straight chain or branched chain. Specifically, there can be mentioned straight alkyl chains such as propyl, butyl, pentyl, hexyl, heptyl, octyl, nonyl, decyl, undecyl, dodecyl, tridecyl, tetradecyl and the like, as well as branched alkyl chains such as isopropyl, isobutyl, sec-butyl, tert-butyl, isopentyl, neopentyl, tert-pentyl, isohexyl, 1-methylpentyl, 2-methylpentyl, 5-methylhexyl, 2,3,5-trimethylhexyl, 2,7,8-trimethyldecyl, 4-ethyl-5-methylnonyl and the like. Of these, preferable are straight alkyl chain of 6-12 carbon atoms, such as hexyl, heptyl, octyl, decyl, undecyl, dodecyl and the like. As the lower alkyl groups of 1-3 carbon atoms represented by $R_2$ and $R_3$, there can be mentioned straight alkyl chains or branched alkyl chains such as methyl, ethyl, propyl and isopropyl. Of these, methyl is preferable. The alkyl group of 1-10 carbon atoms represented by $R_4$ can be of straight chain or branched chain. Specifically, there can be mentioned straight chain alkyl groups such as methyl, ethyl, propyl, butyl, pentyl, hexyl, heptyl, octyl, nonyl, decyl and the like, as well as branched chain alkyl groups such as isopropyl, isobutyl, sec-butyl, tert-butyl, isopentyl, neopentyl, tert-pentyl, isohexyl, 1-methylpentyl, 2-methylpentyl, 5-methylhexyl, 4-ethylhexyl, 2,3,5-trimethylhexyl, 4-ethyl-5-methylhexyl and the like. Of these, preferable are straight alkyl chain of 1-8 carbon atoms such as methyl, ethyl, propyl, butyl, pentyl, heptyl and octyl.

It is generally preferable that $Q_1$ be a single bond, an ether groups or a carboxylic acid ester group, and $Q_2$ and $Q_3$ be independently an ether group or a carboxylic acid ester group. As carboxylic acid ester groups represented by $Q_1$, $Q_2$ and $Q_3$, there can be mentioned the

$-\overset{O}{\overset{\|}{C}}-O-$ ester group and the

$O-\overset{O}{\overset{\|}{C}}-$ ester group $Q_1$ is preferably a single bond, an ether group or a

$-\overset{O}{\overset{\|}{C}}-O-$ ester group; $Q_2$ is preferably a

$-\overset{O}{\overset{\|}{C}}-O-$ ester group, a

$-O-\overset{O}{\overset{\|}{C}}-$ ester group or an ether group, $Q_3$ is preferably an ether group, or an ester group $-O-\overset{O}{\overset{\|}{C}}-$ or $-\overset{O}{\overset{\|}{C}}-O-$.

The carboxylic acid ester groups represented by X and Y can be a $-\overset{O}{\overset{\|}{C}}-O-$ ester group and a $-O-\overset{O}{\overset{\|}{C}}-$ ester group; and as the ethyleneoxy group represented by X and Y, there can be mentioned $-CH_2O-$ and $-OCH_2-$.

The six-membered ring-1,4-diyl group represented by

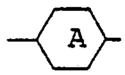,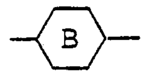 and 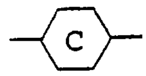

refers to a benzene ring having two bonds at the para-positions. As specific examples thereof there can be mentioned, for example, p-phenylene, 1,4-cyclohexylene, 2,5-(1,3-dioxane)-diyl, 2,5-pyridinediyl, 2,5-pyrimidinediyl, 2,5-(1,4-pyrazine)-diyl and 3,6-(1,2-pyridazine)-diyl. These rings can be substituted with a halogen, cyano, methyl and/or methoxy group.

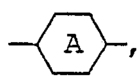, 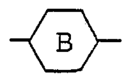 and 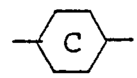

may be the same or different. 2,5-(1,3-dioxane)-diyl can be

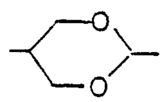

or

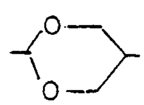;

2,5-pyridinediyl can be

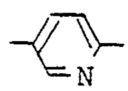 or 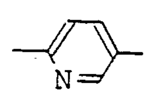

and 2,5-pyrimidinediyl can be

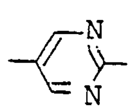 or 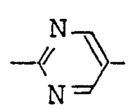.

When M is

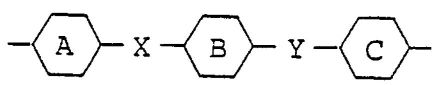,

preferable combinations of A, B, C, X and Y include the case where one of X and Y is a single bond, the

other of them is a carboxylic acid ester group, and all of

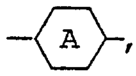, 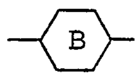 and 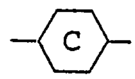

are p-phenylene or at least one of them is 2,5-pyrimidinediyl. When M is

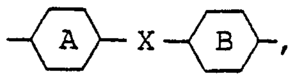,

preferable combinations of A, B and X include the case where X is a single bond or a carboxylic acid ester bond, and both of

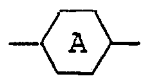 and 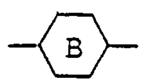

are p-phenylene or either of them is 2,5-pyrimidinediyl.

The compounds I have two asymmetric carbon atoms within the molecule and therefore have four different optical isomers, that is, (R,R) type, (R,S) type, (S,R) type and (S,S) type.

The optically active compounds I of the present invention have a structure in which each asymmetric carbon atom is bonded to oxygen or carbonyl, and therefore the compounds generally show high spontaneous polarization. In addition, most of the compounds I show a chiral smectic C (Sc*) phase which is a liquid crystal phase suitable for display methods utilizing the ferroelectric properties of liquid crystals, and the temperature range of the chiral smectic C phase is low and wide.

The optically active compounds of the present invention are very stable to heat, light, water and air. Accordingly, in putting the compounds to practical use as or in liquid crystal materials, there can be eliminated inconveniences such as arrangements of an apparatus for prevention of overheating, a glass frit seal for prevention of moisture absorption or permeation, etc.

The optically active compounds I of the present invention have excellent compatibility with conventionally known liquid crystal compounds such as those of Schiff's base type, biphenyl type, phenylcyclohexane type, heterocyclic type and the like. Therefore, the compounds can be made into liquid crystal compositions having excellent properties, by incorporating them into said liquid crystal compounds.

As the liquid crystal compounds into which the optically active compounds I of the present invention can be incorporated, there can be mentioned, for example, ferroelectric liquid crystal compounds as well as liquid crystal compounds showing a smectic C$^*$ phase. The ferroelectric liquid crystal compounds include, for example, biphenyl type liquid crystals described in JP-A-118744/1984 and 13729/1985, ester type liquid crystals described in JP-A-128357/1984, 51147/1985, 22051/1986 and 249953/1986, and pyrimidine type liquid crystals described in JP-A-260564/1985, 24756/1986, 85368/1986 and 215373/1986. The liquid crystal compounds showing a smectic C phase include, for example, ester type liquid crystals compounds described in JP-A-228036/1987, and cyclohexane type liquid crystals and heterocyclic type liquid crystals described in the materials of the 16th Friburg Liquid Crystal Forum (Mar. 21, 1986) and the materials of the First International Symposium on Ferroelectric Liquid Crystals (Sept. 21, 1987).

The ferroelectric liquid crystal compounds of the present invention can also be incorporated into the nematic or cholesteric liquid crystals described in "Flüssige Kristalle in Tabellen" I & II, VEB-Verlag, Leipzig, and further can be mixed with any of commercially available nematic liquid crystal compounds. When the ferroelectric liquid crystal compounds of the present invention are incorporated into nematic liquid crystals, the chirality of the present compounds is effectively utilized, and the twisting direction of the cholesteric pitch and the pitch length of the nematic liquid crystal compositions obtained (can be freely) controlled via the amount added.

The liquid crystal display devices according to the present invention can be produced from the above mentioned liquid crystal compositions of the present invention according to a conventional process. The present compositions can be used in, for example, liquid crystal display devices having a structure as shown in the Figure. The device can be designed as a guest host type display device by the use of a

dichroic dye.

In the following, the preparation of the compounds I according to the present invention is explained, whereby reference is made to the following literature:

*1) A. Stempel and F. W. Landgraf, J. Org. Chem., 27, 4675 (1962); A. Hassner and V. Alexanian, Tetrahedron Lett., 1978, 4475.

*2) T. Mukaiyama et al., Chemistry Lett., 1975, 1045; T. Mukaiyama et al., Chemistry Lett., 1976, 13; K. Saigo et al., Bull. Chem. Soc. Japan, 50, 1863 (1977).

*3) O. Mitsunobu and M. Yamada, Bull. Chem. Soc. Japan, 40, 2380 (1967).

*4) M. Saroja and T.N.B. Kaimal, Syn. Commun., 16, 1423 (1986).

*5) S. Kim and W. J. Lee, Syn. Commun., 16, 659 (1986).

*6) S. Bittner and Y. Assaf, Chem. & Ind., 1975, 281.

*7) "Asymmetric Synthesis, "vol. 1 (1983) - Vol. 5 (1985), ed. by J. D. Morrison, Academic Press, Orland; "Asymmetric Catalysis," ed. by B. Bosnich, Martinus Nijhoff Publ., Dordecht (1986); M. A. Sutter and D. Seebach, Ann., 1983, 939.

*8) "Applications of Biochemical Systems in Organic Chemistry", ed. by J. B. Jones, C. J. Sih and D. Perlman, John, Wiley, New York (1976); G. Frater et al., Tetrahedron, 40, 1269 (1984); R. W. Hoffmann et al., Chem. Ber., 114, 2786 (1981); K. Nakamura et al., Tetrahedron Lett., 27, 3155 (1986).

*9) J. Jacques, A. Collet, S. H. Wilen, "Enantiomers, Racemates and Resolutions." John Wiley & Sons (1981); A. W. Ingersoll, Org. Synth., Coll. Vo., 2,506 (1943); H. Nohira et al., Chemistry Lett., 1981, 857, 951.

*10) E. J. Corey et al., Tetrahedron Lett., 1975, 3183; D. T. Sawyer and M. J. Gibian, Tetrahedron, 35, 1471 (1979).

*11) J. Kaulen, Angew. Chem, 99, 800 (1987).

*12) O. Mitsunobu and E. Eguchi, Bull. Chem. Soc. Japan, 44, 3427 (1971); Review: O. Mitsunobu, Synthesis, 1981, 1.

The optically active compounds I of the present invention can be produced according to the following processes.

Process 1 [Case wherein in the general formula I, the group $Q_2$ between the skeletal component and the dichiral side chain component is a

$-\overset{O}{\overset{\|}{C}}-O-$ group (scheme 1)

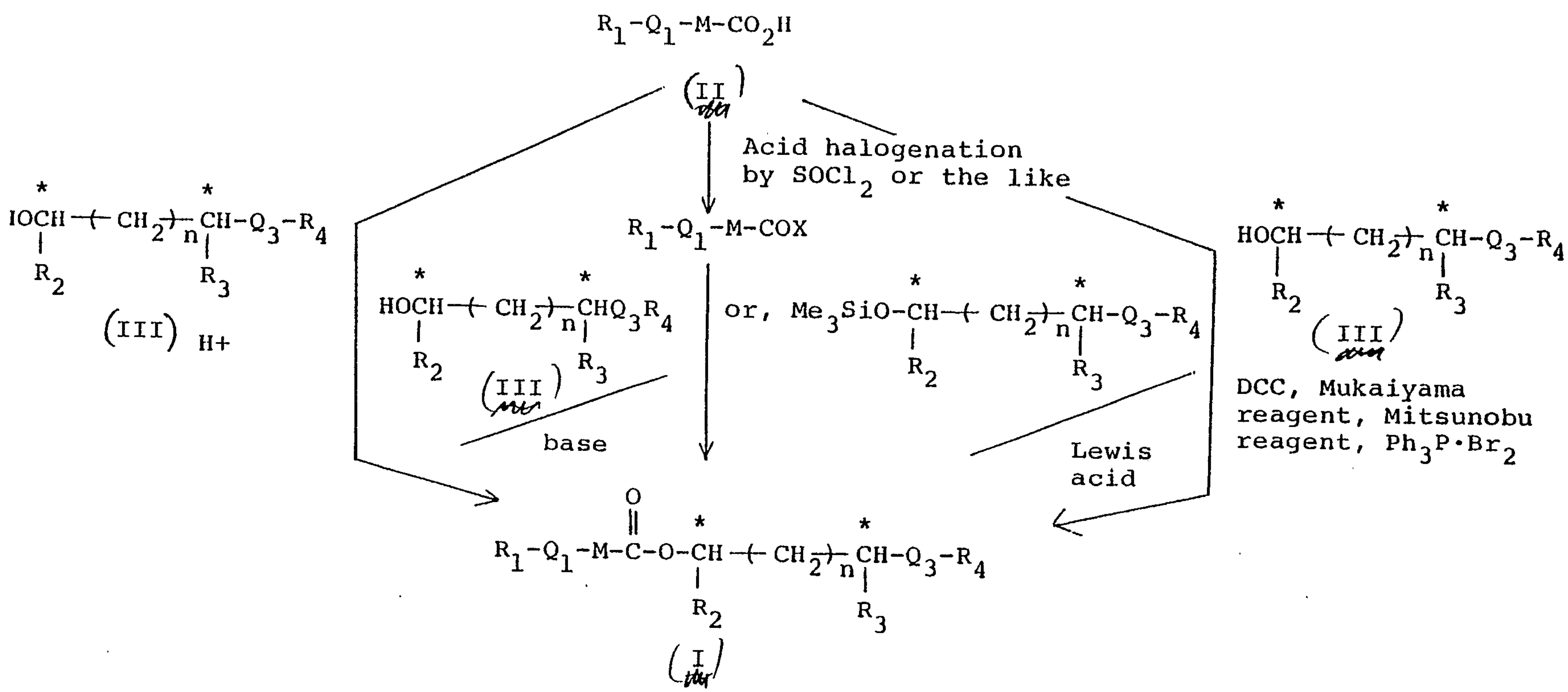


Scheme 1

In the scheme 1, n, $R_1$, $R_2$, $R_3$, $R_4$, $Q_1$, $Q_3$, M and the asterisk * mark have the same definitions as given previously, and X is a halogen atom.

As shown in the scheme 1, the compounds I can be obtained from a carboxylic acid II as the basic skeleton of the compound I and an optically active dichiral secondary alcohol III. This condensation reaction can be carried out according to a known conventional method. That is, the basic skeletal carboxylic acid II and the optically active dichiral secondary alcohol III are subjected to a dehydration and condensation reaction in an organic solvent in the presence of a proton acid to produce the compound I. As the proton acid, there can be used, for example, inorganic acids such as sulfuric acid, hydrochloric acid, perchloric acid and the like, organic sulfonic acids such as p-toluenesulfonic acid, benzenesulfonic acid, trifluoromethanesulfonic acid, methanesulfonic acid and the like, and strongly acidic ion exchange resins such as Amberlise ® and the like. As the organic solvent used in this condensation reaction, there can be mentioned, for example, hydrocarbons such as hexane, benzene, toluene and the like, halogenated hydrocarbons such as chloroform, methylene chloride, carbon tetrachloride, 1,2-dichloroethane and the like, ethers such as diethyl ether, tetrahydrofuran, dioxane and the like, ethyl acetate, acetonitrile and dimethylformamide.

The desired optically active compounds I thus produced can be isolated from the reaction mixture and purified by an ordinary separation and purification procedures (e.g. extraction, solvent operation, column chromatography, liquid chromatography, recrystallization, fractional crystallization).

The desired optically active compounds I can also be obtained in a pure form by converting the basic skeletal carboxylic acid II into a corresponding acid halide with thionyl chloride, thionyl bromide or the like, subjecting the acid halide and an optically active dichiral secondary alcohol III to an esterification reaction in an appropriate organic solvent in the presence of an organic base (e.g. pyridine, triethylamine) at a low temperature, room temperature or an elevated temperature for a few to several hours, optionally a few to several days, and carrying out an ordinary separation and purification.

The condensation reaction of scheme 1 can also be carried out by appropriately selecting a known condensation method other than those mentioned above, such as (a) a method using an activating reagent [e.g. N,N′-dicyclohexylcarbodiimide (DCC)[*1], Mukaiyama reagent [*2] rin.e. a 1-methyl-2-halopyridinium iodide, diethyl azodicarboxylate DEAD and triphenylphosphite ($Ph_3P$) (Mitsunobu's reagent)[*3], triphenylphosphine dibromide[*4]] or (b) a method[*5] comprising converting the sekletal carboxylic acid II into a corresponding acid halide with thionyl chloride or thionyl bromide or the like, and then condensing the acid halide with a trimethylsilyl ether of an optically active dichiral secondary alcohol III in the presence of a catalytic amount of a Lewis acid (e.g. zinc chloride).

Process 2 [Case wherein in the general formula I, the group $Q_2$ between the skeletal component and the dichiral side chain component is an ether (-O-) group] (scheme 2)

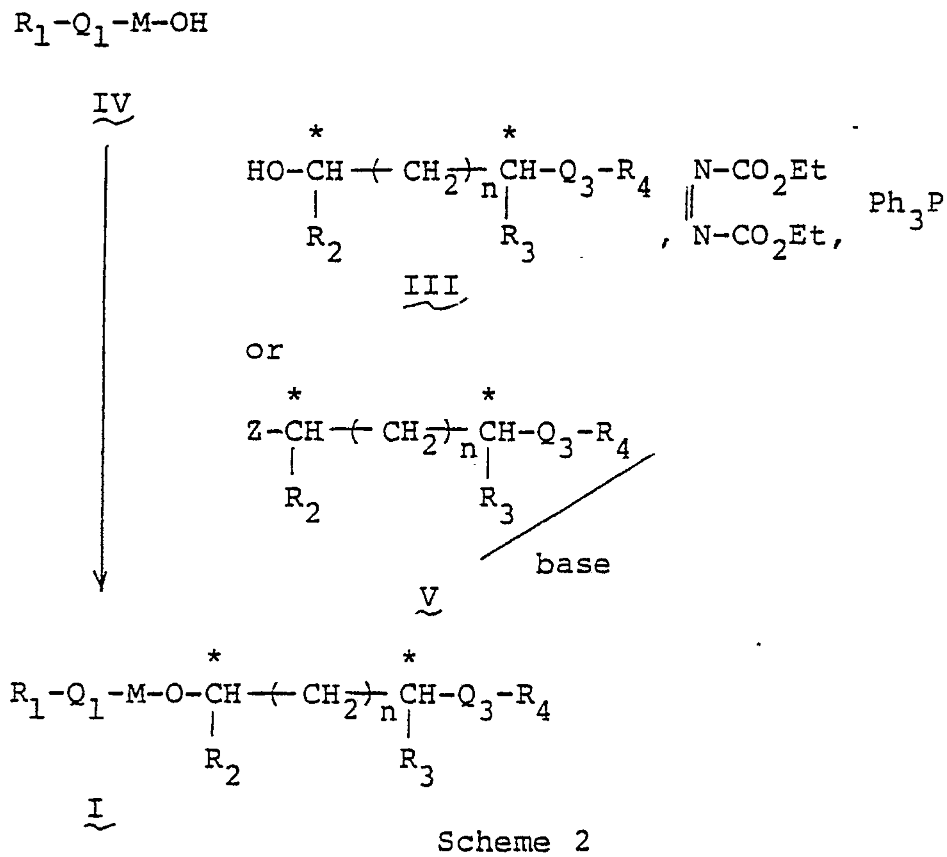


Scheme 2

In the above scheme 2, n, $R_1$, $R_2$, $R_3$, $R_4$, $Q_1$, $Q_3$, M and * mark have the same definitions as given previously. In the formula V, Z is a halogen atom, an organic sulfonyloxy group or the like.

The compounds I can also be obtained by a known condensation reaction between a basic skeletal alcohol or phenolic hydroxy compound IV and an optically active dichiral secondary alcohol III. For example, the compounds I can be obtained by a condensation reaction[*5] using the above mentioned diethyl azodicarboxylate (DEAD) and triphenylphosphine ($Ph_3P$), or by reacting a dichiral secondary alcohol III with an organic sulfonyl chloride in the presence of an organic base (e.g. pyridine, triethylamine) or an inorganic base (e.g. sodium hydride) in an appropriate organic solvent to convert the alcohol into a corresponding organic sulfonic acid ester and then subjecting the ester to an etherification reaction with a basic skeletal phenolic hydroxy compound or alcohol IV in the presence of an inorganic base (e.g. potassium carbonate, sodium hydride) or a strong organic base. As the organic sulfonyl chloride referred to herein, there can be mentioned, for example, aromatic sulfonic acid chlorides such as p-toluenesulfonyl chloride, o-toluenesulfonyl chloride, p-chlorobenzenesulfonyl chloride, benzenesulfonyl chloride, α-naphthalenesulfonyl chloride, β-naphthalenesulfonyl chloride and the like; methanesulfonyl chloride; and trifluoromethanesulfonyl chloride. As the organic solvent used in the etherification reaction of the scheme 2, there can be mentioned hydrocarbons, halogenated hydrocarbons, ethers (e.g. diethyl ether, tetrahydrofuran, dioxane), ethyl acetate, acetonitrile, benzene, toluene, dimethylformamide (DMF), dimethyl sulfoxide (DMSO), hexamethylphosphoric triamide (HMPA), etc. The activation of the optically active dichiral secondary alcohol III includes, besides the above mentioned conversion into an organic sulfonic acid ester, conversions into a halide. The conversion of the optically active dichiral secondary alcohol III into a halide can be effected by a method in which said organic sulfonic acid ester is reacted with a metal halide (e.g. sodium iodide, potassium iodide) or a method in which the dichiral secondary alcohol III is directly reacted with a halogenating agent (e.g. thionyl chloride, thionyl bromide). The thus synthesized halide of the dichiral secondary alcohol III can be subjected to an etherification reaction with the basic skeletal phenolic hydroxy compound or alcohol IV in the presence of an inorganic base (e.g. potassium carbonate, sodium hydroxide) or a strong organic base in an organic solvent. The desired ether compound I thus produced can be isolated from the reaction mixture and purified by an ordinary separation and purification procedures (e.g. extraction, solvent operation, column chromatography, liquid chromatography, recrystallization).

Process 3 [Case wherein in the general formula I, the group $Q_2$ between the skeletal component and the dichiral side chain component is a

$-O-\overset{O}{\overset{\|}{C}}-$ group

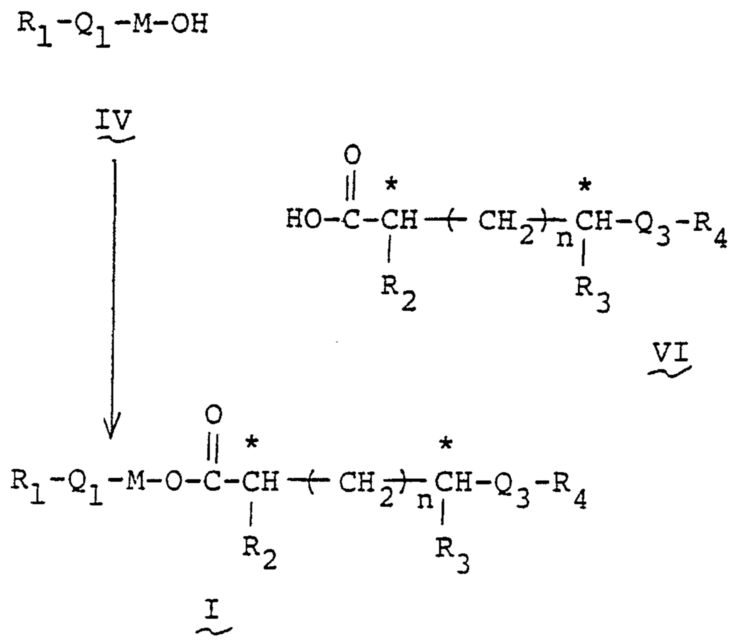


Scheme 3

In scheme 3, n, $R_1$, $R_2$, $R_3$, $R_4$, $Q_1$, $Q_3$, M and * mark have the same definitions as given previously.

The compounds I can also be obtained by a known condensation reaction between a basic skeletal alcohol or phenolic hydroxy compound IV and an optically active dichiral carboxylic acid VI as shown in Scheme 3. This condensation reaction can be carried out according to a known conventional method.

In the above, representative processes for producing the liquid crystal ester compounds I and the liquid crystal ether compounds I of the present invention are described. However, the process for producing the liquid crystal compounds I of the present invention is not restricted to the above processes in which the basic skeletal compound II or IV and the optically active dichiral secondary alcohol III, the optically active dichiral derivative V or the optically active dichiral carboxylic acid VI are condensed. The liquid crystal compounds I can be also be produced, for example, by condensing an alcohol or phenolic hydroxy compound containing an optically active dichiral component as a basic skeleton and a carboxylic acid or a phenolic hydroxy compound as another skeleton, or by condensing a carboxylic acid or phenolic hydroxy compound containing an optically active dichiral component as a basic skeleton and an alcohol or phenolic hydroxy compound as another skeleton.

When $Q_2$ is different from the above-mentioned groups, e.g., a carbonyl group, a single bond, or a carbonyldioxy group, the compounds I can also be produced according to conventional methods.

The optically active dichiral alcohol III and the optically active dichiral derivative V, both of which are materials for the important dichiral component of the present liquid crystal compounds I, can be derived from optically active dichiral compounds which are easily available as a reagent, and can also be obtained by a chemical asymmetric synthesis[*7], a biological asymmetric synthesis[*8] using an enzyme or a microorganism, or an optical resolution[*9]. The thus obtained optically active dichiral secondary alcohol III can be subjected to inversion of configuration on asymmetric carbon by a chemical or biological method to convert it into other optical isomer(s). As the typical methods for inverting the hydroxyl group of optically active secondary alcohol, there are known, for example, a method[*10] in which the hydroxyl group is converted into an organic sulfonic acid ester and then subjected to an intramolecular nucleophilic substitution reaction to effect inversion, a method[*11] in which an optically active secondary alcohol is activated by N,N′-dicyclohexylcarbodiimide (DCC) in the presence of cuprous chloride and then reacted with an appropriate carboxylic acid to effect inversion, and a method[*12] in which an optically active secondary alcohol is reacted with diethyl azodicarboxylic acid (DEAD), triphenylphosphine ($Ph_3P$) and an

appropriate carboxylic acid to effect inversion.

As a typical example of the optically active dichiral secondary alcohol III which is a material for the important dichiral component of the optically active compounds I of the present invention, there can be mentioned compounds represented by the following formula:

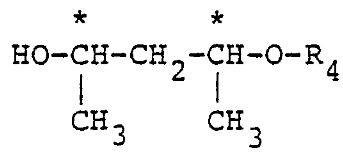

wherein $R_4$ is a straight chain or branched chain alkyl group of 1-10 carbon atoms. This is a compound of formula III in which $R_2$ and $R_3$ are each $CH_3$, n is 1, and $Q_3$ is O (oxygen), and specifically is an optically active 3-alkyloxy-2-pentanol.

When $R_4$ is a straight chain alkyl group of 1-10 carbon atoms, the compound includes optically active 3-methoxy-2-pentanol, 3-ethoxy-2-pentanol, 3-propoxy-2-pentanol, 3-butoxy-2-pentanol, 3-pentyloxy-2-pentanol, 3-hexyloxy-2-pentanol, 3-heptyloxy-2-pentanol, 3-octyloxy-2-pentanol, 3-nonyloxy-2-pentanol, 3-decyloxy-2-pentanol, etc.

When $R_4$ is a branched chain alkyl group of 1-10 carbon atoms, the above compound includes optically active 3-isopropoxy-2-pentanol, 3-isobutoxy-2-pentanol, 3-tert-butoxy-2-pentanol, 3-(2-methylpentyloxy)-2-pentanol, 3-(3-methylpentyloxy)-2-pentanol, etc. Of these, preferable are compounds having, as $R_4$, a straight chain alkyl group of 3-6 carbon atoms, that is, 3-propoxy-2-pentanol, 3-butoxy-2-pentanol, 3-pentyloxy-2-pentanol, and 3-hexyloxy-2-pentanol.

The alkyl ester of 4-hydroxy-2-methylvaleric acid shown below are also typical examples of the optically active dichiral secondary alcohol III:

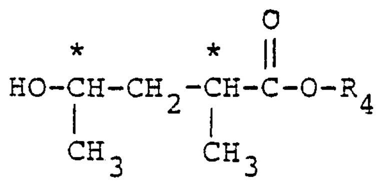

wherein $R_4$ is a straight or branched chain alkyl group of 1-10 carbon atoms. This is a compound of formula III in which $R_2$ and $R_3$ are each $CH_3$, n is 1, and $Q_3$ is $-\overset{O}{\overset{\|}{C}}-O-$, and specifically is an alkyl ester of optically active 4-hydroxy-2-methylvaleric acid.

When $R_4$ is a straight chain alkyl group of 1-10 carbon atoms, the compound includes methyl ester, ethyl ester, propyl ester, butyl ester, pentyl ester, hexyl ester, heptyl ester, octyl ester, nonyl ester and decyl ester of optically active 4-hydroxy-2-methylvaleric acid. Of these, preferable are compounds of a straight alkyl chain of 3-6 carbon atoms, that is, propyl ester, butyl ester, pentyl ester and hexyl ester of optically active 4-hydroxy-2-methylvaleric acid.

The 3-acyloxy-2-pentanols shown below are also typical examples of the optically active dichiral secondary alcohol III:

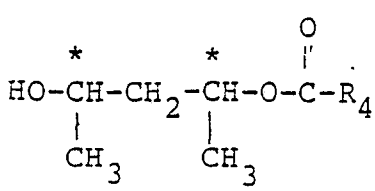

wherein $R_4$ is a straight chain or branched chain alkyl group of 1-10 carbon atoms. This is a compound of formula III in which $R_2$ and $R_3$ are each $CH_3$, n is 1, and $Q_3$ is $-O-\overset{O}{\overset{\|}{C}}-$, and specifically is an optically active 3-acyloxy-2-pentanol.

When $R_4$ is a straight chain alkyl group of 1-10 carbon atoms, the compound includes optically active 3-acetyloxy-2-pentanol, optically active 3-propionyloxy-2-pentanol, optically active 3-butyryloxy-2-pentanol,

optically active 3-pentanoyloxy-2-pentanol, optically active 3-hexanoyloxy-2-pentanol, optically active 3-heptanoyloxy-2-pentanol, optically active 3-octanoyloxy-2-pentanol, optically active 3-nonanoyloxy-2-pentanol and optically active 3-decyloxy-2-pentanol. When $R_4$ is a branched chain alkyl group of 1-10 carbon atoms, the compound includes optically active 3-isobutyryloxy-2-pentanol, 3-isovaleryloxy-2-pentanol, 3-pyvaroyloxy-1-methylpropanol, etc.

When the radical $\lbrace CH_2 \rbrace_n$ of the optically active compounds I of the present invention is a single bond (n=0), the optically active dichiral secondary alcohol III which is an important material for constituting the dichiral portion of the compounds I includes, as a typical example, a 2-alkoxy-1-methylpropanol represented by the following formula:

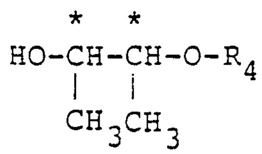

wherein $R_4$ is an alkyl group of 1-10 carbon atoms.

This alcohol specifically includes 3-methoxy-2-butanol, 3-ethoxy-2-butanol, 3-propoxy-2-butanol, 3-butoxy-2-butanol, 3-pentyloxy-2-butanol, 3-hexyloxy-2-butanol, 3-heptyloxy-2-butanol, 3-octyloxy-2-butanol, 3-nonyloxy-2-butanol, 3-decyloxy-2-butanol, 3-isopropoxy-2-butanol, 3-isobutoxy-2-butanol, 3-tert-butoxy-2-butanol, 3-(2-methylpentyloxy)-2-butanol, 3-(3-methylpentyloxy)-2-butanol, etc.

The optically active dichiral secondary alcohol III further includes alkyl esters of a 3-hydroxy-2-methylbutyric acid represented by the following formula:

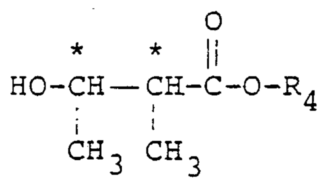

wherein $R_4$ is an alkyl group of 1-10 carbon atoms.

This compound specifically includes methyl ester, ethyl ester, propyl ester, butyl ester, pentyl ester, hexyl ester, heptyl ester, octyl ester, nonyl ester, decyl ester, isopropyl ester, isobutyl ester, tert-butyl ester, 2-methylpentyl ester, 3-methylpentyl ester of 3-hydroxy-2-methylbutyric acid, etc.

The optically active dichiral secondary alcohol III furthermore includes 3-acyloxy-2-butanols represented by the following formula:

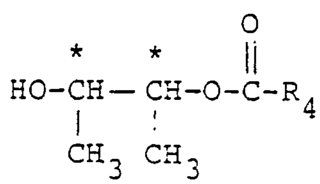

wherein $R_4$ is an alkyl group of 1-10 carbon atoms.

This compound specifically includes 3-acetyloxy-2-butanol, 3-propionyloxy-2-butanol, 3-butyryloxy-2-butanol, 3-pentanoyloxy-2-butanol, 3-hexanoyloxy-2-butanol, 3-heptanoyloxy-2-butanol, 3-octanoyloxy-2-butanol, 3-nonanoyloxy-2-butanol, 3-decyloxy-2-butanol, 3-isobutyryloxy-2-butanol, 3-isovaleryloxy-2-butanol, 3-pyvaroyloxy-2-butanol, etc.

The optically active dichiral carboxylic acid VI which is also an important material for constituting the dichiral portion of the optically active compound I of the present invention includes 3-alkyloxy-2-methylbutyric acids represented by the following formula:

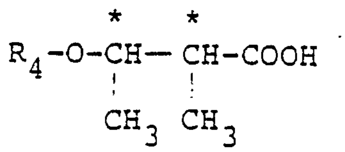

wherein $R_4$ is an alkyl group of 1-10 carbon atoms.

This acid specifically includes 3-methoxy-2-methyl-butyric acid, 3-ethoxy-2-methylbutyric acid, 3-propoxy-2-methylbutyric acid, 3-butoxy-2-methylbutyric acid, pentyloxy-2-methylbutyric acid, 3-hexyloxy-2-methylbutyric acid, 3-heptyloxy-2-methylbutyric acid, 3-nonyloxy-2-methylbutyric acid, 3-decyloxy-2-butyric acid, 3-isopropoxy-2-methylbutyric acid, 3-isobutoxy-2-methylbutyric acid, 3-tert-butoxy-2-methylbutyric acid, 3-(2-methylpentyloxy)-2-methylbutyric acid, 3-(3-methylpentyloxy)-2-methylbutyric acid, etc.

The skeletal carboxylic acid, which is a material for the sekletal component of the optically active compound of the present invention and which is represented by the general formula II, more specifically by the general formulae II′ and II″, is explained specifically:

$R_1$-$Q_1$-M-$CO_2$H [II]

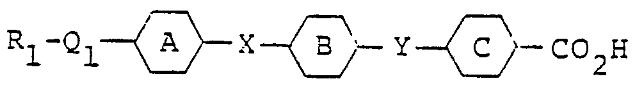 [II′]

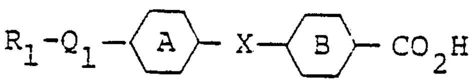 [II″]

When in the general formula II′, $R_1$ is a straight or branched chain alkyl group of 3-14 carbon atoms, $Q_1$ is a single bond or an ether group, X and Y are independently an ester group

($-\overset{O}{\overset{\|}{C}}-O-$ or $-O-\overset{O}{\overset{\|}{C}}-$) or a single bond , and

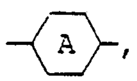, 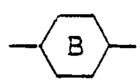 and 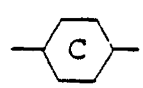

are a phenyl ring, the skeletal carboxylic acid II includes 4-(4′-alkyloxy or alkyl-4-biphenylcarbonyloxy) benzoic acids, 4-(4′-alkyloxy or alkyl-4-biphenyloxycarbonyl)-benzoic acids 4′-(4-alkyloxy or alkylphenylcarbonyloxy)-4-biphenylcarboxylic acids, 4′-(4-alkyloxy or alkylphenyloxycarbonyl)-4-biphenylcarboxylic acids, 4-{4-[4-(alkyloxy or alkyl)phenylcarbonyloxy]-4′-phenylcarbonyloxy}benzoic acids, 4-{4-[4-(alkyloxy or alkyl)phenyloxycarbonyl-4′-phenylcarbonyloxy}benzoic acids, 4-{4-[4-(alkyloxy or alkyl)phenyloxycarbonyl]-4′-phenyloxycarbonyl}benzoic acids, 4-{4-[4-(alkyloxy or alkyl)phenylcarbonyloxy]-4′-phenyloxycarbonyl}benzoic acids, etc. When in the general formula II″, $R_1$ is a straight or branched chain alkyl group of 3-14 carbon atoms, $Q_1$ is a single bond or an ether group, X is an ester group

($-\overset{O}{\overset{\|}{C}}-O-$ or $-O-\overset{O}{\overset{\|}{C}}-$) or a single bond, and

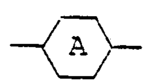 and 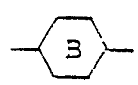

are a phenyl ring, the skeletal carboxylic acid II includes 4′-alkyloxy or alkyl-4-biphenylcarboxylic acids, 4-[4-(alkyloxy or alkyl)phenylcarbonyloxy)benzoic acids and 4-[4-(alkyloxy or alkyl)phenyloxycarbonyl]benzoic acids. As other typical examples of the skeletal carboxylic acid II, there can be mentioned 4″-alkyloxy(or alkyl)-4-terphenylcarboxylic acids, 4′-(trans-4-alkyloxy or alkylcyclohexylcarbonyloxy)-4-biphenylcarboxylic acids, trans-4-(4′-alkyloxy or alkyl-4-biphenylcarbonyloxy)cyclohexanecarboxylic acids, 2-[4-(4-alkyloxy or alkylphenylcarbonyloxy)phenyl]pyridimidinyl-5-carboxylic acids, 2-(4′-alkyloxy or alkyl-4-biphenyl)-pyrimidinyl-5-carboxylic acids, 4′-(5-alkyloxy or alkylpyrimidinyl-2-oxycarbonyl)biphenyl-4-carboxylic acids, 4′-[2-(5-alkyloxy or alkyl)-2-(pyridyl)ethyl]biphenyl-4-carboxylic acids, 4-[4-(trans-5-alkyloxy or alkyl-1,3-dioxane-2-yl)phenylcarbonyloxy]benzoic acids 4′-[4-(trans-5-alkyloxy or alkyl-1,3-dioxane-2-yl)]biphenyl-4-carboxylic acids, 2-[4-(4-alkyloxy or alkylphenylcarbonyloxy)phenyl]pyrazinyl-5-carboxylic acids, 2-(4′-alkyloxy or alkyl-4-biphenyl)pyrazinyl-5-carboxylic acids, 4′-(5-alkyloxy or alkylpyrazinyl-2-oxycarbonyl)-biphenyl-4-carboxylic acids, etc.

The skeletal alcohol or phenolic hydroxy compound which is a material for the skeletal component of the liquid crystal compound of the present invention is represented by the general formula IV, more preferably the general formulas [IV′] and [IV″]:

$R_1$-$Q_1$-M-OH [IV]

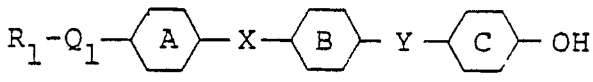 [IV']

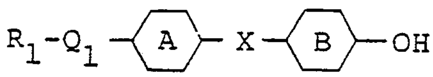 [IV"] ,

The skeletal alcohol or phenolic hydroxy compound is an alcohol or hydroxyl group derived from the above mentioned carboxylic acid and obtained by replacing the carboxyl group of the latter with a hydroxyl group. Specific examples of the alcohol or phenolic hydroxy compounds include 4-hydroxyphenyl esters of 4′-alkyloxy or alkylbiphenyl-4-carboxylic acids, 4′-alkyloxy or alkyl-4-biphenyl esters of 4′-hydroxybenzoic acids, 4′-hydroxy-4-biphenyl esters of 4-alkyloxy or alkylbenzoic acids, 4-alkyloxy or alkylphenyl esters of 4′-hydroxybiphenyl-4-carboxylic acids, 4′-hydroxy-4-biphenyl esters of trans-4-alkyloxy or alkylcyclohexanecarboxylic acids, trans-4-hydroxycyclohexyl esters of 4′-alkyloxy or alkyl-4-biphenylcarboxylic acids, 4-(5-hydroxy-2-pyrimidinyl)phenyl esters of 4-alkyloxy or alkylbenzoic acids, 2-(4′-alkyloxy or alkyl-4-biphenyl)pyrimidine-5-ol, 5-alkyloxy or alkyl-2-pyridinyl esters of 4′-hydroxy-4-biphenylcarboxylic acids, 4′-[2-(5-alkyloxy or alkyl-2-pyridyl)ethyl]biphenyl-4-ol, 4-hydroxyphenyl esters of 4-[4-(trans-5-alkyloxy or alkyl)-1,3-dioxane-2-yl]benzoic acids, and 5-alkyloxy or 5-alkyl-2-pyrazinyl esters of 4′-hydroxy-4-biphenylcarboxylic acids.

In the above, there have been listed typical examples of the skeletal carboxylic acid II and the skeletal alcohol or phenolic hydroxy compound IV which are contained in the optically active of the present invention, as well as the typical examples of the optically active dichiral alcohol II which is a material for the optically active dichiral side chain component contained in the optically active compound of the present invention. While the optically active compounds I of the present invention can be produced by appropriately combining one of the skeletal components and one of the optically active dichiral components, the properties of the optically active compounds I of the present invention are dependent upon both of the skeleton containing the nonchiral alkyl side chain and the optically active dichiral component used. The skeletal component and the optically active dichiral component are not restricted to the above mentioned examples.

The present invention is explained in more detail by way of Examples.

Example 1 Preparation of 4-[(1R, 2R)-2-butoxy-1-methylpropoxycarbonyl]phenyl ester of 4′-octyloxy-4-biphenylcarboxylic acid (a compound of the general formula I′ in which n is 0, $R_1$ is n-$C_8H_{17}$, $R_2$ and $R_3$ are each $CH_3$, $R_4$ is n-$C_4H_9$, $Q_1$ and $Q_3$ are each -O-, X is a single bond, $Q_2$ and Y are each $-\overset{O}{\overset{\|}{C}}$-O-and

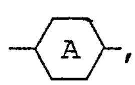, 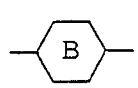 and 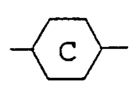 are each 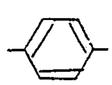).

i) Preparation of (2R, 3R)-3-butoxy-2-butanol

The title alcohol can be produced according to the following scheme.

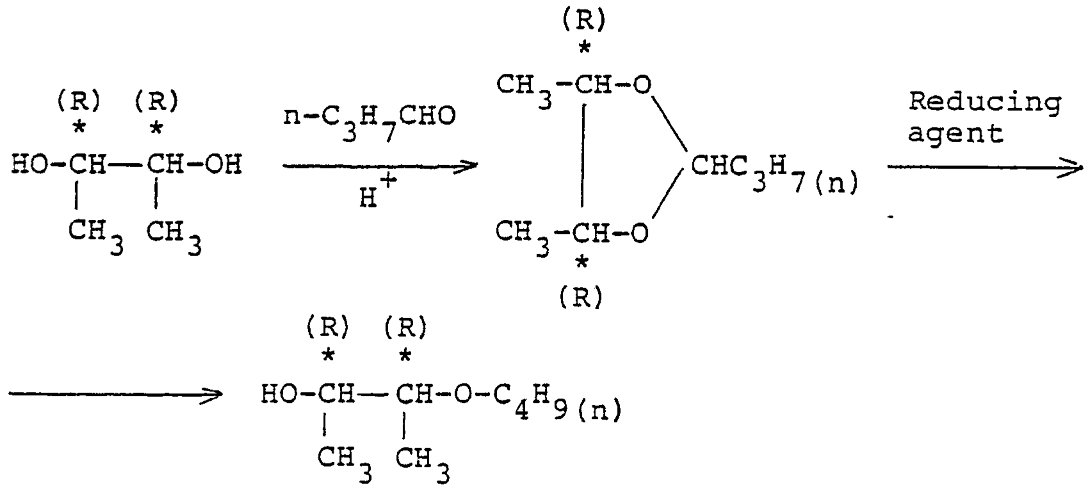


10.0 g of (2R, 3R)-(-)-2,3-butanediol and 10.4 g of n-butyl aldehyde were dissolved in 100 ml of benzene. Thereto was added 0.2 g of p-toluenesulfonic acid monohydrate. The mixture was refluxed for 2 hours with removing water according to a conventional method. After the completion of the reaction, 0.1 ml of pyridine was added and the mixture was concentrated under reduced pressure. The residue was subjected to distillation under reduced pressure (b.p. 58-59° C/30 mmHg) to obtain 14.5 g of a butylidene derivative of (2R, 3R)-(-)-2,3-butanediol.

Under ice-cooling, 26.7 g of aluminum chloride were dissolved in 200 ml of dry ether, and thereto was added 1.90 g of lithium aluminum hydride in small portions. Thereto was dropwise added 14.5 g of the above obtained butylidene derivatives under ice-cooling. The mixture was stirred overnight with cooling to room temperature. After the completion of the reaction, 150 ml of 10% sulfuric acid was added dropwise with stirring under ice-cooling. The ether layer was separated and the aqueous layer was extracted with ether twice. The combined ether layer was dried and concentrated. The residue was subjected to distillation under reduced pressure (b.p. 84-86° C/33 mmHg) to obtain 13.4 g of (2R, 3R)-3-butoxy-2-butanol.

The $^1$H-NMR and IR spectrum data of the above compound are shown below.

$^1$H-NMR (90 MHz, $CDCl_3$)

δ: 0.93 (3H, t), 1.09 (3H, d, J = 5.9Hz), 1.14 (3H, d, J = 6.1Hz), 1.20-1.73 (4H, m), 2.76 (1H, d, OH), 3.16 (1H, m), 3.30-3.76 (3H, m)

IR $\nu_{max}^{neat}$ cm$^{-1}$: 3150-3600

ii) Esterification

2.49 g of the (2R, 3R)-3-butoxy-2-butanol produced in the above i) was dissolved in 25 ml of dry tetrahydrofuran. Thereto were added 3.30 g of 1,1,1,3,3,3-hexamethyldisilazane and one drop of trimethylsilyl chloride, and the mixture was refluxed for 12 hours. The mixture was concentrated under reduced pressure. The residue was subjected to distillation under reduced pressure b.p. 92-97° C/30 mmHg) to obtain 2.93 g of trimethylsilyl ether of (2R, 3R)-3-butoxy-2-butanol.

Separately, 40 ml of thionyl chloride was added to 3.00 g of 4-(4′-octyloxy-4-biphenylcarboxyoxy)-benzoic acid. The mixture was refluxed for 3 hours. Excessive thionyl chloride was removed by concentration under reduced pressure to obtain a corresponding acid chloride. Thereto were added 50 ml of dry acetonitrile, 1.47 g of the above obtained trimethylsilyl ether of (2R,3R)-3-butoxy-2-butanol and 0.09 g of zinc chloride, and the mixture was refluxed for 1 hour. After the completion of the reaction, the mixture was concentrated, and the residue was subjected to column chromatography [silica gel, developing solvent = dichloromethane) and then to recrystallization from ethanol to obtain 42 g of the title compound.

The $^1$H-NMR, IR spectrum, elemental analysis and specific rotation power of the compound are shown below.

$^1$H-NMR (90 MHz, $CDCl_3$)

δ: 0.89 (6H, t), 1.20 (3H, d, J = 6.4Hz), 1.32 (3H, d, J = 6.4Hz), 1.15-1.95 (16H, m), 3.52 (3H, m), 4.02 (2H, t), 5.22 (1H, m), 6.90-8.30 (12H, m)

IR $\nu_{max}^{KBr}$ cm$^{-1}$: 1735, 1710, 1600, 1500, 825, 760

| Elemental analysis | | | | | |
|---|---|---|---|---|---|
| Calcd. for $C_{36}H_{46}O_6$ | : | C, | 75.23; | H, | 8.07 |
| Found | : | C, | 75.30; | H, | 8.07 |

$[\alpha]_D^{28}$ : -17.1° (c = 1.09, chloroform)

Example 2 Preparation of 4-[(1S,2R)-2-butoxy-1-methylpropoxycarbonyl]phenyl ester of 4′-octyloxy-4-biphenylcarboxylic acid (a compound of the general formula I′ in which n is 0, $R_1$ is n-$C_8H_{17}$, $R_2$ and $R_3$ are each $CH_3$, $R_4$ is n-$C_4H_9$, $Q_1$ and $Q_3$ are each -O-, X is a single bond, $Q_2$ and Y are each

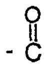 -O-, and

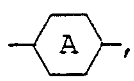, 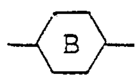 and 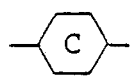 are each 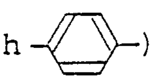)

In 50 ml of dry tetrahydrofuran were dissolved 0.72 g of the (2R,3R)-3-butoxy-2-butanol produced in Example 1 i), 2.00 g of 4-(4′-octyloxy-4-biphenylcarbonyloxy)benzoic acid and 2.3 g of triphenylphosphine. Thereto was dropwise added 1.56 g of diethyl azodicarboxylate. The mixture was stirred for 30 minutes at room temperature. The mixture was concentrated under reduced pressure. The residue was subjected to column chromatography [silica gel, developing solvent = hexane-ethyl acetate (5:1)] and then to recrystallization from ethanol to obtain 1.28 g of the title compound.

The $^1$H-NMR, IR spectrum, elemental analysis and specific rotation are shown below.

$^1$H-NMR (90 MHz, $CDCl_3$)

δ: 0.90 (3H, t), 0.91 (3H, t), 1.22 (3H, d, J = 6.4 Hz), 1.35 (3H, d, J = 6.5Hz), 1.15-2.00 (16H, m), 3.53 (3H, m), 4.02 (2H, t), 5.16 (1H, m), 6.90-8.30 (12H, m)

IR $\nu_{max}^{KBr}$ $cm^{-1}$: 1735, 1710, 1600, 1500, 830, 760

| Elemental analysis | | | | | |
|---|---|---|---|---|---|
| Calcd. for $C_{38}H_{46}O_6$ | : | C, | 75.23; | H, | 8.07 |
| Found | : | C, | 75.11; | H, | 7.87 |

$[\alpha]_D^{23}$ : + 11.7° (c = 1.04, chloroform)

Example 3 Preparation of 4′-octyloxy-4-biphenyl ester of 4[(1R,2R)-2-butoxy-1-methylpropoxy]benzoic acid (a compound of the general formula I′ in which n is 0, $R_1$ is n-$C_8H_{17}$, $R_2$ and $R_3$ are each $CH_3$, $R_4$ is n-$C_4H_9$, $Q_1$, $Q_2$ and $Q_3$ are each -O-, X is a single bond, Y is

-O- 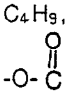 -, and

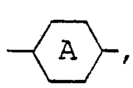, 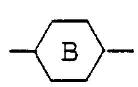 and 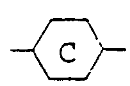 are each 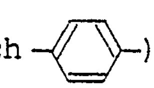).

i) Preparation of (2S,3R)-3-butoxy-2-butanol

This optically active alcohol can be produced by subjecting the (2R,3R)-3-butoxy-2-butanol produced in Example 1 i) to the inversion of hydroxyl group according to the following scheme.

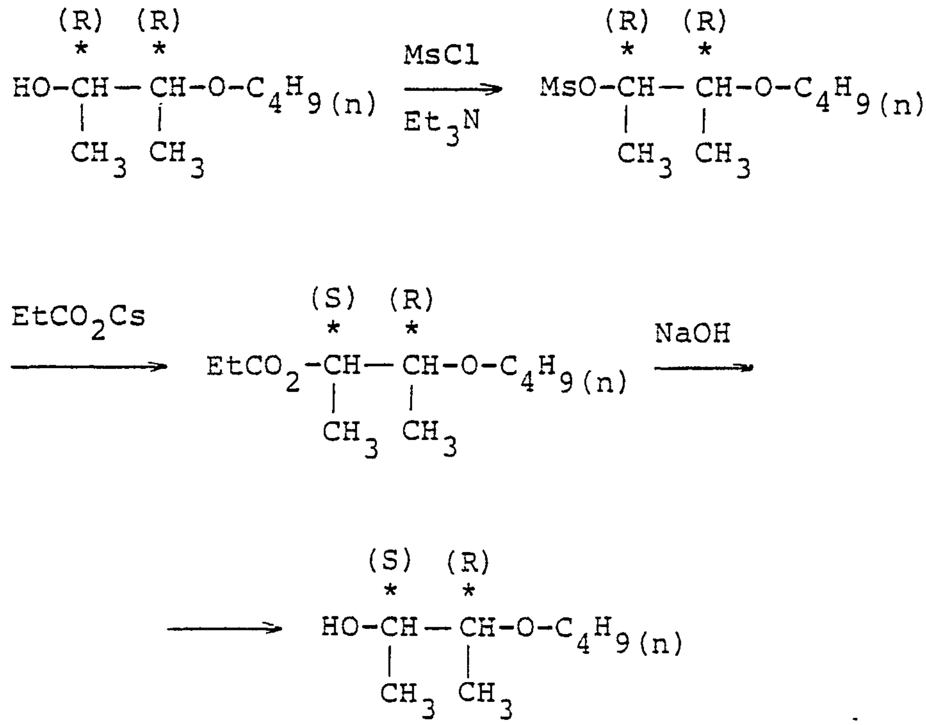


1.00 g of (2R,3R)-3-butoxy-2-butanol and 0.83 g of triethylamine were dissolved in 10 ml of dichloromethane. Thereto was dropwise added 0.94 g of methanesulfonyl chloride with ice-cooling. The mixture was stirred overnight at room temperature. The mixture was poured into water and extracted with dichloromethane. The organic layer was washed with dilute hydrochloric acid and an aqueous sodium bicarbonate solution in this order, and then dried and concentrated under reduced pressure. The residue was subjected to Kugel-rohr distillation (1 mmHg) to obtain 1.30 g of (2R,3R)-3-butoxy-2-butyl methanesulfonate.

Independently, 1.97 g of cesium carbonate was dissolved in 50 ml of methanol. Thereto was dropwise added 1.34 g of propionic acid. The mixture was stirred for 1 hour at room temperature. The mixture was concentrated under reduced pressure removed. The residue was mixed with toluene and the mixture was concentrated under reduced pressure (addition of 50 ml of toluene and concentration was carried out three times) to obtain a white powder of cesium propionate. This powder and 0.68 g of the above obtained (2R,3R)-3-butoxy-2-butyl methanesulfonate were dissolved in 50 ml of dry N,N-dimethylformamide. The mixture was heated overnight at 100° C. After the completion of the reaction, the mixture was poured into water. The resulting mixture was extracted with ether three times. The extract was washed with water (twice), dilute hydrochloric acid (once) and an aqueous sodium bicarbonate solution (once), and dried and concentrated. The residue was subjected to Kugelrohr distillation (23 mmHg) to obtain 0.37 g of (2S,3R)-3-butoxy-2-butyl propionate.

0.37 g of the compound was dissolved in 8 ml of methanol. Thereto was added 2 ml of a 1 N methanol solution of sodium hydroxide. The mixture was stirred overnight at room temperature. After the completion of the reaction, the mixture was concentrated under reduced pressure. To the residue was added water and the mixture was extracted with dichloromethane three times. The extract was dried and concentrated. The residue was subjected to Kugelrohr distillation (22 mmHg) to obtain 0.22 g of (2S,3R)-3-butoxy-2-butanol.

The $^1$H-NMR and IR spectrum data of the compound are shown below.

$^1$H-NMR (90 MHz, $CDCl_3$)

δ: 0.92 (3H, t), 1.09 (3H, d, J=6.3Hz), 1.11 (3H, d, J=6.5Hz), 1.20-1.66 (4H, m), 2.08 (1H, d, OH), 3.13-3.62 (3H, m), 3.83 (1H, m)

IR $\nu_{max}^{neat}$ $cm^{-1}$: 3150-3650

ii) Condensation

In 10 ml of dry tetrahydrofuran were dissolved 0.22 g of the (2S,3R)-3-butoxy-2-butanol produced in the above i), 0.62 g of 4′-octyloxy-4-biphenyl 4-hydroxybenzoate and 0.59 g of triphenylphosphine. Thereto was

dropwise added 0.39 g of diethyl azodicarboxylate. The mixture was stirred for 1 hour at room temperature. The mixture was concentrated under reduced pressure. The residue was subjected to column chromatography (silica gel, developing solvent = dichloromethane) and then to recrystallization from ehtanol to obtain 0.43 g of the title compound.

The $^1$H-NMR, IR spectrum, elementary analysis and specific rotation of the compound are shown below.

$^1$H-NMR (90 MHz, $CDCl_3$)

δ: 0.90 (6H, t), 1.20 (3H, d, J = 6.4Hz), 1.31 (3H, d, J = 6.2Hz), 1.10-1.97 (16H, m), 3.54 (3H, m), 4.00 (2H, t), 4.52 (1H, m), 6.80-8.20 (12H, m)

IR $\nu_{max}^{KBr}$ $cm^{-1}$: 1730, 1605, 1500, 840, 810, 760

| Elementary analysis | | | | | |
|---|---|---|---|---|---|
| Calcd. for $C_{35}H_{46}O_5$ | : | C, | 76.89; | H, | 8.48 |
| Found | : | C, | 77.16; | H, | 8.53 |

$[\alpha]_D^{22}$ : 0 (c = 0.612, chloroform)

Example 4 Preparation of 4′-octyloxy-4-biphenyl ester of 4-[(1S,2R)-2-butoxy-1-methylpropoxy]benzoic acid (a compound of the general formula I′ in which n is 0, $R_1$ is n-$C_8H_{17}$, $R_2$ and $R_3$ are each $CH_3$, $R_4$ is n-$C_4H_9$, $Q_1$, $Q_2$ and $Q_3$ are each -O-, X is a single bond, Y is

$-O-\overset{O}{\overset{\|}{C}}-$,

and 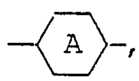, 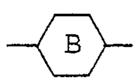 and 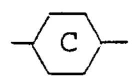 are each 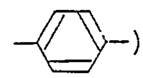).

0.84 g of the (2R,3R)-3-butoxy-2-butanol produced in Example 1 i) and 2.00 g of 4-octyloxy-4-biphenyl ester of 4-hydroxybenzoic acid were subjected to the same procedure as in Example 3 ii) to obtain 1.22 g of the title compound.

The $^1$H-NMR, IR spectrum, elementary analysis and specific rotation of the compound are shown below.

$^1$H-NMR (90 MHz, $CDCl_3$)

α: 0.89 (3H, t), 0.92 (3H, t), 1.23 (3H, d, J = 6.3 Hz), 1.36 (3H, d, J = 6.2Hz), 1.10-2.00 (16H, m), 3.54 (3H, m), 4.00 (2H, t), 4.40 (1H, m), 6.80-8.20 (12H, m)

IR $\nu_{max}^{KBr}$ $cm^{-1}$: 1730, 1610, 1500, 840, 810, 760

| Elementary analysis | | | | | |
|---|---|---|---|---|---|
| Calcd. for $C_{35}H_{46}O_5$ | : | C, | 76.89; | H, | 8.48 |
| Found | : | C, | 77.11; | H, | 8.53 |

$[\alpha]_D^{30}$ : + 6.63° (c = 1.10, chloroform)

Example 5 Preparation of 4-[(1S,2R)-2-butoxy-1-methylpropoxy]phenyl ester of 4′-octyloxy-4-biphenylcarboxylic acid (a compound of the general formula I′ in which n is 0, $R_1$ is n-$C_8H_{17}$, $R_2$ and $R_3$ are each $CH_3$, $R_4$ is n-$C_4H_9$, $Q_1$, $Q_2$ and $Q_3$ are each -O-, X is a single bond, Y is

$-\overset{O}{\overset{\|}{C}}-O-$, and

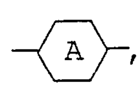, 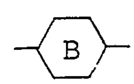

and 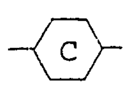 are each 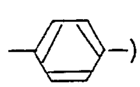).

0.45 g of the (2R,3R)-3-butoxy-2-butanol produced in Example 1 i) and 1.50 g of 4-hydroxyphenyl ester of 4′-octyloxy-4-biphenylcarboxylic acid were subjected to the same procedure as in Example 3 ii) to obtain 0.63 g of the title compound.

The elementary analysis and specific rotation of the compound are shown below.

| Elementary analysis | | | | | |
|---|---|---|---|---|---|
| Calcd. for $C_{35}H_{48}O_5$ | : | C, | 76.89; | H, | 8.48 |
| Found | : | C, | 77.05; | H, | 8.53 |

$[\alpha]_D^{23}$ : + 7.05° (c = 1.02, chloroform)

Example 6 Preparation of 4-octyloxyphenyl ester of 4′-[(1S,2R)-2-butoxy-1-methylpropoxy]-4-biphenylcarboxylic acid (a compound of the general formula I′ in which N is 0, $R_1$ is n-$C_8H_{17}$, $R_2$ and $R_3$ are each $CH_3$, $R_4$ is n-$C_4H_9$, $Q_1$, $Q_2$ and $Q_3$ are each -O-, X is

$-O-\overset{O}{\overset{\|}{C}}-$, Y is a single bond, and

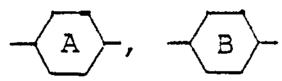,

and 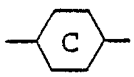 are each 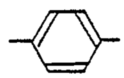).

0.53 g of the (2R, 3R)-3-butoxy-2-butanol produced in Example 1 i) and 1.50 g of 4-octyloxyphenyl ester of 4′-hydroxy-4-biphenylcarboxylic acid were subjected to the same procedure as in Example 3 ii) to obtain 0.57 g of the title compound.

The elementary analysis and specific rotation of the compound are shown below.

| Elementary analysis | | | | | |
|---|---|---|---|---|---|
| Calcd. for $C_{35}H_{48}O_5$ | : | C, | 76.89; | H, | 8.48 |
| Found | : | C, | 76.83; | H, | 8.48 |

$[\alpha]_D^{25}$ : + 4.44° (c = 0.844, chloroform)

Example 7 Preparation of 4′-[(1S,2R)-2-butoxy-1-methylpropoxy]-4-biphenyl ester of 4-octyloxybenzoic acid (a compound of the general formula I′ in which n is 0, $R_1$ is n-$C_8H_{17}$, $R_2$ and $R_3$ are each $CH_3$, $R_4$ is n-$C_4H_9$, $Q_1$, $Q_2$ and $Q_3$ are each -O-, X is

$-\overset{O}{\overset{\|}{C}}-O-$, Y is a single bond, and

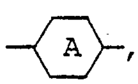, 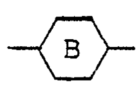 and 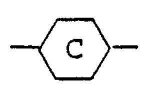 are

each 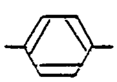).

0.53 g of the (2R,3R)-3-butoxy-2-butanol and 1.50 g of 4′-hydroxy-4-biphenyl ester of 4-octyloxybenzoic acid were subjected to the same procedure as in Example 3 ii) to obtain 0.61 g of the title compound.

The elementary analysis and specific rotation of the compound are shown below.

| Elementary analysis | | | | | |
|---|---|---|---|---|---|
| Calcd. for $C_{35}H_{48}O_5$ | : | C, | 76.89; | H, | 8.48 |
| Found | : | C, | 76.74; | H, | 8.59 |

$[\alpha]_D^{26}$ : + 4.75° (c = 1.02, chloroform)

Example 8 Preparation of 4′-octyloxy-4-biphenyl ester of 4-[(1S,2R)-2-octyloxy-1-methylpropoxy]benzoic acid (a compound of the general formula I′ in which n is 0, $R_1$ and $R_4$ are each n-$C_8H_{17}$, $R_2$ and $R_3$ are each $CH_3$, $Q_1$, $Q_2$ and $Q_3$ are each -O-, X is a single bond, Y is

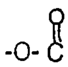 -, and

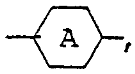, 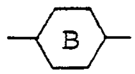 and 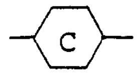 are

each 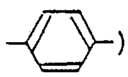).

i) preparation of (2R,3R)-3-octyloxy-2-butanol

5.34 g of (2R,3R)-(-)-2,3-butanediol and 7.59 g of n-octyl aldehyde were subjected to the same procedure as in Example 1 i) to obtain 11.20 g of (2R,3R)-3-octyloxy-2-butanol (b.p. 71-72° C/1 mmHg).

The $^1$H-NMR and IR spectrum data of the compound are shown below.

$^1$H-NMR (90 MHz, $CDCl_3$)

δ: 0.88 (3H, t), 1.09 (3H, d, J = 6.2Hz), 1.14 (3H, d, J = 6.1Hz), 1.00-1.75 (12H, m), 2.76 (1H, d, OH), 3.12 (1H, m), 3.25-3.72 (3H, m)

IR $\nu_{max}^{neat}$ cm$^{-1}$: 3150-3650

ii) Condensation

0.33 g of the (2R,3R)-3-octyloxy-2-butanol produced in the above i) and 0.62 g of 4′-octyloxy-4-biphenyl ester of 4-hydroxybenzoic acid were subjected to the same procedure as in Example 3 ii) to obtain 0.49 g of the title compound.

The $^1$H-NMR, IR spectrum, elementary analysis and specific rotation of the compound are shown below.

$^1$H-NMR (90 MHz, $CDCl_3$)

δ: 0.89 (6H, t), 1.23 (3H, d, J = 6.4Hz), 1.36 (3H, d, J = 6.2Hz), 1.06-1.96 (24H, m), 3.50 (3H, m), 4.00 (2H, t), 4.40 (1H, m), 6.80-8.20 (12H, m)

IR $\nu_{max}^{KBr}$ cm$^{-1}$: 1730, 1600, 1500, 840, 760

| Elementary analysis | | | | | |
|---|---|---|---|---|---|
| Calcd. for $C_{35}H_{54}O_5$ | : | C, | 77.70; | H, | 9.03 |
| Found | : | C, | 77,82; | H, | 8.96 |

$[\alpha]_D^{25}$ : + 5.37° (c = 0.614, chloroform)

Example 9 Preparation of 4-[(1S,2R)-2-ethoxycarbonyl-1-methylpropoxycarbonyl]phenyl ester of 4′-octyloxy-4-biphenylcarboxylic acid (a compound of the general formula I′ in which n is 0, $R_1$ is n-$C_8H_{17}$, $R_2$ and $R_3$ are each $CH_3$, $R_4$ is $C_2H_5$, $Q_1$ is -O-, X is a single bond, $Q_2$, $Q_3$ and Y are each

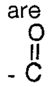 -O-, and

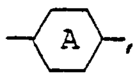, 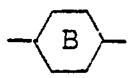 and 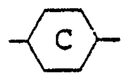 are each 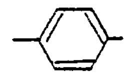).

i) Preparation of ethyl ester of (2R,3S)-3-hydroxy-2-methylbutyric acid

The title compound can be obtained according to a known method [R. W. Hoffman et al., Chem. Ber., 114, 2786 (1981)]. In this method, ethyl 2-methyl-3-oxobutyrate was subjected to asymmetric reduction using a baker's yeast to obtain the title compound and its diastereomer, that is, ethyl ester of (2S,3S)-3-hydroxy-2-methylbutyric acid at a ratio of (82:18) to (87:13). In the following Example 9 ii), Examples 10-18 and 21-26, this mixture was used without separation, and the final mixture obtained by condensation with a skeletal compound was subjected to purification such as column chromatography, recrystallization or the like to obtain a single diastereomer.

ii) Esterification

5.00 g of the ethyl ester of (2R,3S)-3-hydroxy-2-methylbutyric acid produced in the above i) was dissolved in 70 ml of dry tetrahydrofuran. Thereto were added 4.31 g of 1,1,1,3,3,3-hexamethyldisilazane and one drop of trimethylsilyl chloride, and the mixture was refluxed for 5 hours. The reaction mixture was concentrated and the residue was subjected to distillation under reduced pressure (b.p. 96 °C/30 mmHg) to obtain 5.69 g of trimethylsilyl ether of ethyl ester of (2R,3S)-3-hydroxy-2-methylbutyric acid.

Independently, 2.50 g of 4-(4′-octyloxy-4-biphenylcarbonyloxy)benzoic acid was mixed with 40 ml of thionyl chloride, and the mixture was refluxed for 7 hours. Excessive thionyl chloride was removed by distillation to obtain the corresponding acid chloride. Thereto were added 50 ml of dry acetonitrile, 1.21 g of the above obtained trimethylsilyl ether of ethyl ester of (2R,3S)-3-hydroxy-2-methylbutyric acid and 0.08 g of zinc chloride. The mixture was refluxed for 1.5 hours. After the completion of the reaction, the reaction mixture was concentrated. The residue was subjected to column chromatography [silica gel, developing solvent = chloroform-n-hexane (9:1)] and then to recrystallization from ethanol to obtain 0.51 g of the title compound.

The $^1$H-NMR, IR spectrum, elementary analysis and specific rotation are shown below.

$^1$H-NMR (90 MHz, $CDCl_3$)

δ: 0.89 (3H, t), 1.15-1.7 (19H,m), 1.75-1.9 (2H, m), 2.7-2.95 (1H, m), 3.95-4.29 (4H, m), 5.2-5.5 (1H, m), 6.95-8.26 (12H, m)

IR $\nu_{max}^{KBr}$ $cm^{-1}$: 1740, 1710, 1600, 830, 760

| Elementary analysis | | | | | |
|---|---|---|---|---|---|
| Calcd. for $C_{35}H_{42}O_7$ | : | C, | 73.15; | H, | 7.37 |
| Found | : | C, | 73.31; | H, | 7.41 |

$[\alpha]_D^{27}$ : + 11.6° (c = 0.876, chloroform)

Example 10 Preparation of 4-[(1S,2R)-2-butoxycarbonyl-1-methylpropoxycarbonyl]phenyl ester of 4′-octyloxy-4-biphenylcarboxylic acid (a compound of the general formula I′ in which n is 0, $R_1$ is n-$C_8H_{17}$, $R_2$ and $R_3$ are each $CH_3$, $R_4$ is n-$C_4H_9$, $Q_1$ is -O-, X is a single bond, $Q_2$, $Q_3$ and Y are each

$-\overset{O}{\overset{\|}{C}}-O-$, and

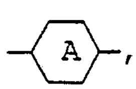, 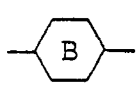 and 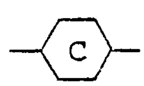 are each 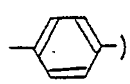).

i) Preparation of butyl ester of (2R,3S)-3-hydroxy-2-methyl-butyric acid

This compound can be produced by the following scheme.

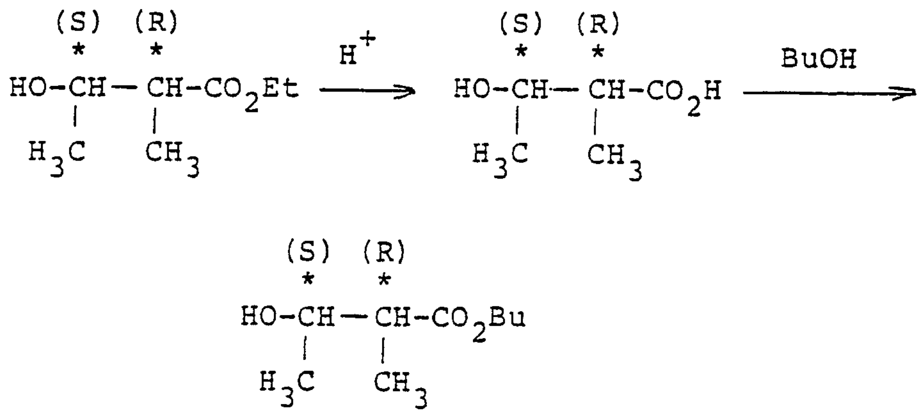


To 160 ml of water was added 8.00 g of the ethyl ester of (2R,3S)-3-hydroxy-2-methylbutyric acid produced in Example 9 i). Further, 40 ml of 36% hydrochloric acid was added. The mixture was heated for 6 hours at 80 °C. The reaction mixture was extracted with ethyl acetate. The extract was dried and concentrated. The residue was subjected to distillation under reduced pressure (b.p. 90 °C/1 mmHg) to obtain 3.34 g of (2R,3S)-3-hydroxy-2-methylbutyric acid. This compound was dissolved in 150 ml of n-butanol, and 0.2 ml of concentrated sulfuric acid was added. The mixture was refluxed for 7 hours with removing water produced by a molecular sieve (3A). The mixture was concentrated under reduced pressure and the residue was subjected to distillation under reduced pressure (b.p. 65 °C/1 mmHg) to obtain 3.28 g of butyl ester of (2R,3S)-3-hydroxy-2-methylbutyric acid.

The $^1$H-NMR and IR spectrum of this compound are shown below.

$^1$H-NMR (90 MHz, $CDCl_3$)

δ: 0.86-1.71 (13H, m), 2.37-2.57 (2H, m), 3.85-4.2 (3H, m)

IR $\nu_{max}^{neat}$ cm$^{-1}$: 3400, 1730, 1460

ii) Esterification

The butyl ester of (2R,3S)-3-hydroxy-2-methylbutyric acid produced in the above i) was dissolved in 50 ml of dry tetrahydrofuran. Thereto were added 18.2 g of 1,1,1,3,3,3-hexamethyldisilazane and one drop of trimethylsilyl chloride, and the mixture was refluxed for 6 hours. The mixture was concentrated and the residue was subjected to distillation under reduced pressure (b.p. 130 °C/47 mmHg) to obtain 4.15 g of trimethylsilyl ether of butyl ester of (2R,3S)-3-hydroxy-2-methylbutyric acid.

Independently, 2.50 g of 4-(4′-octyloxy-4-biphenylcarbonyloxy)benzoic acid was mixed with 40 ml of thionyl chloride, and the mixture was refluxed for 5 hours. Excessive thionyl chloride was removed under reduced pressure to obtain an acid chloride. This acid chloride was dissolved in 50 ml of dry acetonitrile, 1.42 g of the above obtained trimethylsilyl ether of butyl ester of (2R,3S)-3-hydroxy-2-methylbutyric acid and 0.08 g of zinc chloride. The mixture was refluxed for 3 hours. After the completion of the reaction, the mixture was concentrated under reduced pressure and the residue was subjected to rough purification by column chromatography [silica gel, developing solvent = dichloromethane-n-hexane (9:1)] and then to column chromatography (silica gel, developing solvent = dichloromethane) and further to recrystallization from ethyl acetate-n-hexane to obtain 1.52 g of the title compound.

The $^1$H-NMR, IR spectrum, elementary analysis and specific rotation of the compound are shown below.

$^1$H-NMR (90 MHz, $CDCl_3$)

δ: 0.8-1.0 (6H, m), 1.25-1.5 (20H, m), 1.6-1.95 (2H, m), 1.6-1.95 (1H, m), 4.02 (2H, t), 4.10 (2H, t), 5.2-5.55 (1H, m), 6.92-8.25 (12H, m)

IR $\nu_{max}^{KBr}$ cm$^{-1}$: 1740, 1730, 1720, 1605, 830, 760

| Elementary analysis | | | | | |
|---|---|---|---|---|---|
| Calcd. for $C_{37}H_{48}O_7$<br>Found | :<br>: | C,<br>C, | 73.73;<br>73.90; | H,<br>H, | 7.69<br>7.76 |

$[\alpha]_D^{25}$ : + 13.7° (c = 1.25, chloroform)

Example 11 Preparation of 4-[(2R,3S)-3-methoxy-2-methylbutylcarbonyloxy]phenyl ester of 4′-octyloxy-4-biphenylcarboxylic acid (a compound of the general formula I′ in which n is 0, $R_1$ is n-$C_8H_{17}$, $R_2$, $R_3$ and $R_4$ are each $CH_3$, $Q_1$ and $Q_3$ are each -O-, X is a single bond, Y is

$-\overset{O}{\overset{\|}{C}}$ -O-, $Q_2$ is -O-$\overset{O}{\overset{\|}{C}}$ - and

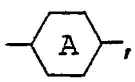, 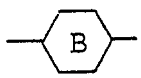 and 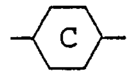 are each 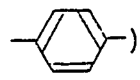).

i) Preparation of (2R,3S)-3-methoxy-2-methylbutyric acid

This optically active carboxylic acid can be produced by the following scheme.

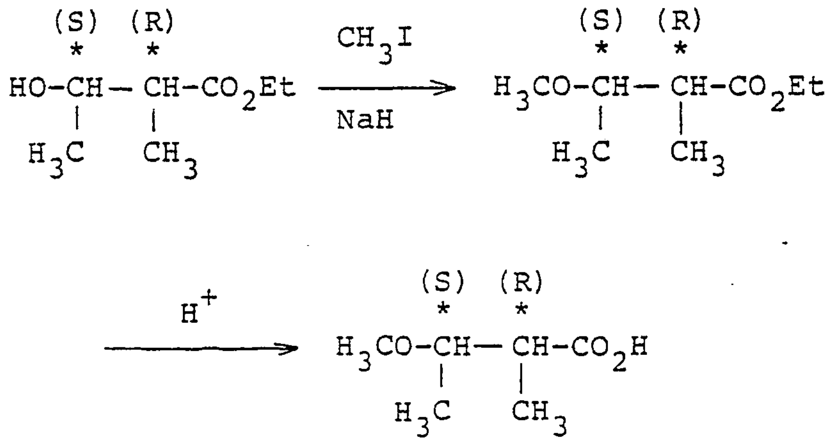


In 150 ml of N,N-dimethylformamide were dissolved 8.20 g of the ethyl ester of (2R,3S)-3-hydroxy-2-methylbutyric acid produced in Example 9 i) and 78.0 g of methyl iodide. To the solution was added in small portions 2.46 g of about 60% oily sodium hydride with ice cooling, and the mixture was stirred for 1 hour with ice cooling. The mixture was poured into 300 ml of ethyl acetate and the mixture was stirred for 10 minutes. The insoluble materials were removed by filtration and the filtrate was washed with water three times. The organic layer was dried and concentrated. The residue was subjected to distillation under reduced pressure (b.p. 80° C/30 mmHg) to obtain 6.87 g of ethyl ester of (2R,3S)-3-methoxy-2-methylbutyric acid.

2.55 g of this methyl ether compound was dissolved in 50 ml of dioxane. To the solution were added 10 ml of concentrated (36%) hydrochloric acid and 50 ml of water. The mixture was heated for 5 hours at 90° C. The mixture was poured into 100 ml of water. The mixture was extracted with ethyl acetate four times. The extract was dried and concentrated and the residue was subjected to distillation under reduced pressure (b.p. 105° C/1 mmHg) to obtain 1.49 g of (2R,3S)-3-methoxy-2-methylbutyric acid.

The $^1$H-NMR and IR spectrum of this compound are shown below.

$^1$H-NMR (90 MHz, $CDCl_3$)

δ: 1.15 (3H, s), 1.23 (3H, s), 2.5-2.8 (1H, m), 3.39 (3H, s), 3.47 - 3.74 (1H, m), 6.0 (1H, b)

IR $\nu_{max}^{neat}$ $cm^{-1}$: 3450, 1710, 1100

ii) Esterification

In 40 ml of dry tetrahydrofuran were dissolved 0.79 g of the (2R,3S)-3-methoxy-2-methylbutyric acid produced in the above i), 2.5 g of 4-hydroxyphenyl ester of 4′-octyloxy-4-biphenylcarboxylic acid and 2.66 g of tributylamine. Thereto was added 1.84 g of 2-chloro-1-methylpyridium iodide, and the mixture was refluxed for 7 hours. The insoluble materials were removed by filtration and the filtrate was concentrated under reduced pressure. The residue was subjected to column chromatography [silica gel, developing solvent = chloroform-n-hexane (4:1)] and then to recrystallization from ethanol to obtain 0.36 g of the title compound.

The $^1$H-NMR, IR spectrum, elementary analysis and specific rotation of the compound are shown below.

$^1$H-NMR (90 MHz, $CDCl_3$)

δ: 0.90 (3H, t), 1.05-1.6 (16H, m), 1.8 (2H, b), 2.65-2.9 (1H, m), 3.40 (3H, s), 3.6-3.82 (1H, m), 4.01 (2H, t), 6.94-8.25 (12H, m)

IR $\nu_{max}^{KBr}$ $cm^{-1}$: 1760, 1730, 1610, 1510, 1100, 830

| Elementary analysis | | | | | |
|---|---|---|---|---|---|
| Calcd. for $C_{33}H_{40}O_6$ | : | C, | 74.41; | H, | 7.57 |
| Found | : | C, | 74.69; | H, | 7.52 |

$[\alpha]_D^{28}$ : - 5.69° (c = 1.14, chloroform)

Example 12 Preparation of 4′-octyloxy-4-biphenyl ester of 4-[(2R,3S)-3-methoxy-2-methylbutyloxy]-benzoic acid (a compound of the general formula I′ in which n is 0, $R_1$ is n-$C_8H_{17}$, $R_2$, $R_3$ and $R_4$ are each $CH_3$, $Q_1$ and $Q_3$ are each -O-, X is a single bond, Y and $Q_2$ are each

$-O-\overset{O}{\overset{\|}{C}}-$, and

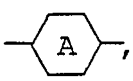, 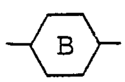 and 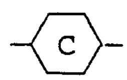 are each 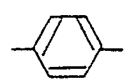).

0.70 g of the (2R,3S)-3-methoxy-2-methylbutyric acid produced in Example 11 i) and 2.22 g of 4′-octyloxy-4-biphenyl ester of 4-hydroxybenzoic acid were subjected to the same procedure as in Example 11 ii) to obtain 0.52 g of the title compound.

The elementary analysis and specific rotation of the compound are shown below.

| Elementary analysis | | | | | |
|---|---|---|---|---|---|
| Calcd. for $C_{33}H_{40}O_6$ | : | C, | 74.41; | H, | 7.57 |
| Found | : | C, | 74.58; | H, | 7.59 |

$[\alpha]_D^{27}$ : - 5.75° (c = 0.828, chloroform)

Example 13 Preparation of 4′-[(1R,2S)-2-methoxy-1-methylpropionylcarbonyloxy]-4-biphenyl ester of 4-octyloxybenzoic acid (a compound of the general formula I′ in which n is 0, $R_1$ is n-$C_8H_{17}$, $R_2$, $R_3$ and $R_4$ are each $CH_3$, $Q_1$ and $Q_3$ are each -O-, X is

$-\overset{O}{\overset{\|}{C}}-O-$, Y is a single bond, $Q_2$ is

$-O-\overset{O}{\overset{\|}{C}}-$,

and 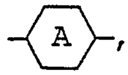, 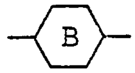 and 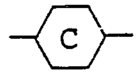 are each 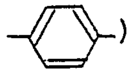).

0.48 g of the (2R,3S)-3-methoxy-2-methylbutyric acid produced in Example 11 i) and 1.0 g of 4′-hydroxy-4-biphenyl ester of 4-octyloxybenzoic acid were mixed with 150 ml of dichloromethane. Thereto was added 1.35 g of triphenylphosphine bromide, and the mixture was refluxed for 7 hours. The reaction mixture was concentrated under reduced pressure. The residue was subjected to column chromatography [silica gel, developing solvent: chloroform-n-hexane (9:1)] and then to recrystallization from ethanol to obtain 0.63 g of the title compound.

The elementary analysis and specific rotation of the compound are shown below.

| Elementary analysis | | | | | |
|---|---|---|---|---|---|
| Calcd. for $C_{33}H_{40}O_6$ | : | C, | 74.41; | H, | 7.57 |
| Found | : | C, | 74.52; | H, | 7.59 |

$[\alpha]_D^{23}$ : - 8.99° (c = 1.06, chloroform)

Example 14 Preparation of 4-octyloxyphenyl ester of 4′-[(2R,3S)-3-methoxy-2-methylbutyryloxy-4-biphenyl-carboxylic acid (a compound of the general formula I′ in which n is 0, $R_1$ is n-$C_8H_{17}$, $R_2$, $R_3$ and $R_4$ are each $CH_3$, $Q_1$ and $Q_3$ are each -O-, X and $Q_2$ are each

$\text{-O-}\overset{O}{\overset{\|}{C}}\text{-}$, Y is a single bond, and

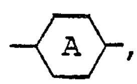, 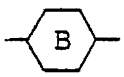 and 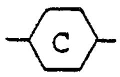 are each 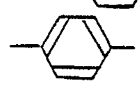).

0.48 g of the (2R,3S)-3-methoxy-2-methylbutyric acid produced in Example 11 i) and 1.0 g of 4-octyloxyphenyl ester of 4′-hydroxy-4-biphenylcarboxylic acid were subjected to the same procedure as in Example 13 to obtain 0.22 g of the title compound.

The elementary analysis and specific rotation of the compound are shown below.

| Elementary analysis | | | | | |
|---|---|---|---|---|---|
| Calcd. for $C_{33}H_{40}O_6$ | : | C, | 74.41; | H, | 7.57 |
| Found | : | C, | 74.66; | H, | 7.65 |

$[\alpha]_D^{24}$ : - 93.7° (c = 0.354, chloroform)

Example 15 Preparation of 4-[2R,3S)-3-butoxy-2-methylbutyryloxy]phenyl ester of 4′-octyloxy-4-biphenyl-carboxylic acid (a compound of the general formula I′ in which n is 0, $R_1$ is n-$C_8H_{17}$, $R_2$ and $R_3$ are each $CH_3$, $R_4$ is n-$C_4H_9$, $Q_1$ and $Q_3$ are each -O-, X is a single bond, Y is

$\text{-}\overset{O}{\overset{\|}{C}}\text{-O-}$, $Q_2$ is

$\text{-O-}\overset{O}{\overset{\|}{C}}\text{-}$,

and 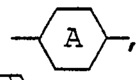, 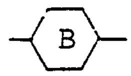 and 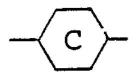 are each 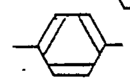 ).

i) Preparation of (2R,3S)-3-butoxy-2-methylbutyric acid

This optically active carboxylic acid can be produced by the following scheme.

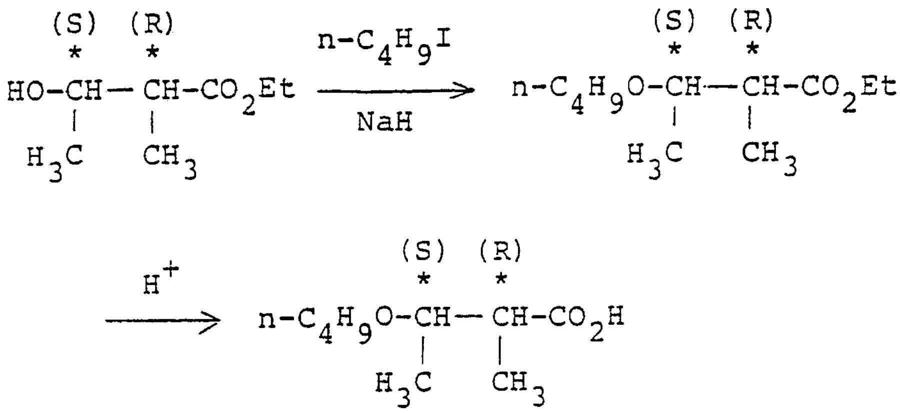


In 150 ml of N,N-dimethylformamide were dissolved 15.0 g of the ethyl ester of (2R,3S)-3-hydroxy-2-methylbutyric acid produced in Example 9 i) and 56.6 g of n-butyl iodide. To the solution was added in small portions 7.89 g of about 60% oily sodium hydride with ice cooling. The mixture was stirred for 2 hours with ice cooling and poured into 300 ml of ethyl acetate. The resulting mixture was stirred for 10 minutes. The insoluble materials were removed by filtration and the filtrate was washed with water four times. The organic layer was dried and concentrated. The residue was subjected to distillation under reduced pressure (b.p. 90-95°C/30 mmHg) to obtain 8.32 g of ethyl ester of (2R,3S)-3-butoxy-2-methylbutyric acid.

1.78 g of this compound was dissolved in 20 ml of dioxane. Thereto were added 5 ml of concentrated (36%) hydrochloric acid and 20 ml of water. The mixture was refluxed for 20 hours. The mixture was diluted with 20 ml of water, and the mixture was extracted with ethyl acetate five times. The extract was dried and concentrated. The residue was subjected to distillation under reduced pressure (b.p. 100-105°C/1 mmHg) to obtain 1.17 g of (2R,3S)-3-butoxy-2-methylbutyric acid.

The $^1$H-NMR spectrum of this compound is shown below.

$^1$H-NMR (90 MHz, $CDCl_3$)

δ: 0.91 (3H, t), 1.15 (3H, s), 1.22 (3H, s), 1.3-1.6 (4H, m), 2.47-2.75 (1H, m), 3.29-3.8 (3H, m), 8.9 (1H, b)

ii) Esterification

To 1.1 g of the (2R,3S)-3-butoxy-2-methylbutyric acid produced in the above i) was added in small portions 5 ml of oxalyl chloride at room temperature. The mixture was stirred for 2 hours at room temperature and then for 30 minutes at 50°C. Excess oxalyl chloride was removed by distillation under reduced pressure. The residue was subjected to Kugel-rohr distillation under reduced pressure to obtain 0.88 g of (2R,3S)-3-butoxy-2-methylbutyric acid chloride.

0.88 g of this acid chloride and 1.00 g of 4-hydroxyphenyl ester of 4′-octyloxy-4-biphenylcarboxylic acid were dissolved in 20 ml of dry tetrahydrofuran.

Thereto was added 0.38 g of dry pyridine, and the mixture was stirred overnight at room temperature. The reaction mixture was concentrated. The residue was subjected to column chromatography (silica gel, developing solvent = dichloromethane) and then to recrystallization from ethyl acetate-methanol to obtain 0.52 g of the title compound.

The $^1$H-NMR, IR spectrum, elementary analysis and specific rotation of the compound are shown below.

$^1$H-NMR (90 MHz, $CDCl_3$)
δ: 0.9 (3H, t), 0.93 (3H, t), 1.15-1.6 (20H, m), 1.7-1.95 (2H, m), 2.6-2.9 (1H, m), 3.35-3.9 (3H, m), 4.05 (2H, t), 6.85-8.25 (12H, m)
IR $\nu^{KBr}_{max}$ $cm^{-1}$: 1750, 1730, 1605, 1505

| Elementary analysis | | | | | |
|---|---|---|---|---|---|
| Calcd. for $C_{36}H_{46}O_6$<br>Found | :<br>: | C,<br>C, | 75.23;<br>75.02; | H,<br>H, | 8.07<br>8.00 |

$[\alpha]_D^{24}$ : + 6.63° (c = 0.532, chloroform)

Example 16 Preparation of 4′-octyloxy-4-biphenyl ester of 4-[(2R,3S)-3-butoxy-2-methylbutyryloxy]benzoic acid (a compound of the general formula I′ in which n is 0, $R_1$ is n-$C_8H_{17}$, $R_2$ and $R_3$ are each $CH_3$, $R_4$ is n-$C_4H_9$, $Q_1$ and $Q_3$ are each -O-, X is a single bond, $Q_2$ and Y are each
$-O-\overset{O}{\overset{\|}{C}}-$, and

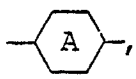, 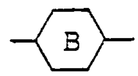
and 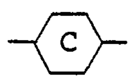 are each 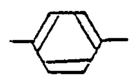).

0.75 g of the (2R,3S)-3-butoxy-2-methylbutyric acid chloride produced in Example 15 ii) and 1.00 g of 4′-octyloxy-4-biphenyl ester of 4-hydroxybenzoic acid were subjected to the same procedure as in Example 15 ii) to obtain 0.85 g of the title compound.

The elementary analysis and specific rotation of this compound are shown below.

| Elementary analysis | | | | | |
|---|---|---|---|---|---|
| Calcd. for $C_{36}H_{46}O_6$<br>Found | :<br>: | C,<br>C, | 75.23;<br>75.15; | H,<br>H, | 8.07<br>8.05 |

$[\alpha]_D^{24}$ : - 0.29° (c = 1.02, chloroform)

Example 17 Preparation of 4′-[(2R,3S)-3-butoxy-2-methylbutyryloxy]-4-biphenyl ester of 4-octyloxybenzoic acid (a compound of the general formula I′ in which n is 0, $R_1$ is n-$C_8H_{17}$, $R_2$ and $R_3$ are each $CH_3$, $R_4$ is n-$C_4H_9$, $Q_1$ and $Q_3$ are each -O-, X is
$-\overset{O}{\overset{\|}{C}}-O-$, Y is a single bond, $Q_2$ is
$-O-\overset{O}{\overset{\|}{C}}-$, and

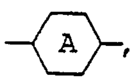, 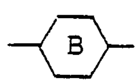 and 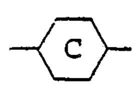 are each
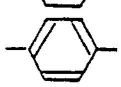).

0.75 g of the (2R,3S)-3-butoxy-2-methylbutyric acid chloride produced in Example 15 ii) and 1.00 g of 4′-hydroxy-4-biphenyl ester of 4-octyloxybenzoic acid were subjected to the same procedure as in Example 15 ii) to obtain 0.62 g of the title compound.

The elementary analysis and specific rotation of this compound are shown below.

| Elementary analysis | | | | | |
|---|---|---|---|---|---|
| Calcd. for $C_{36}H_{46}O_6$ | : | C, | 75.23; | H, | 8.07 |
| Found | : | C, | 75.02; | H, | 8.07 |

$[\alpha]_D^{24}$ : - 47.1° (c = 0.738, chloroform)

Example 18 Preparation of 4-octyloxyphenyl ester of 4′-[(2R,3S)-3-butoxy-2-methylbutyloxy]-4-biphenylcarboxylic acid (a compound of the general formula I′ in which n is 0, $R_1$ is n-$C_8H_{17}$, $R_2$ and $R_3$ are each $CH_3$, $R_4$ is n-$C_4H_9$, $Q_1$ and $Q_3$ are each -O-, X and $Q_2$ are each $-O-\overset{\overset{O}{\|}}{C}-$, Y is a single bond and

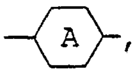, 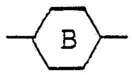 and 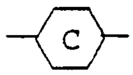 are each 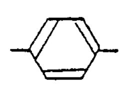).

0.75 g of the (2R,3S)-3-butoxy-2-methylbutyric acid chloride produced in Example 15 ii) and 1.0 g of 4-octyloxyphenyl ester of 4′-hydroxy-4-biphenylcarboxylic acid were subjected to the same procedure as in Example 15 ii) to obtain 0.65 g of the title compound.

The elementary analysis and specific rotation of this compound are shown below.

| Elementary analysis | | | | | |
|---|---|---|---|---|---|
| Calcd. for $C_{36}H_{46}O_6$ | : | C, | 75.23; | H, | 8.07 |
| Found | : | C, | 75.33; | H, | 7.97 |

$[\alpha]_D^{24}$ : + 0.40° (c = 0.646, chloroform)

Example 19 Preparation of (1S,2R)-2-butoxy-1-methylpropyl ester of 4′-octyloxy-4-biphenylcarboxylic acid (a compound of the general formula I′ in which n is 0, $R_1$ is n-$C_8H_{17}$, $R_2$ and $R_3$ are each $CH_3$, $R_4$ is n-$C_4H_9$, $Q_1$ and $Q_3$ are each -O-, $Q_2$ is $-\overset{\overset{O}{\|}}{C}-O-$, X is a single bond, and

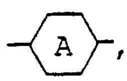, 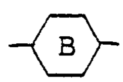 and 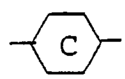 are each 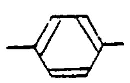).

0.45 g of the (2R,3R)-3-butoxy-2-butanol produced in Example 1 i) and 1.00 g of 4′-octyloxy-4-biphenylcarboxylic acid were subjected to the same procedure as in Example 2 to obtain 0.67 g of the title compound.

The elementary analysis and specific rotation of the compound are shown below.

| Elementary analysis | | | | | |
|---|---|---|---|---|---|
| Calcd. for $C_{29}H_{42}O_4$ | : | C, | 76.61; | H, | 9.31 |
| Found | : | C, | 76.52; | H, | 9.10 |

$[\alpha]_D^{23}$ : + 15.5° (c = 1.09, chloroform)

Example 20 Preparation of 4-[(1S,2R)-2-butoxy-1-methylpropoxy-4′-octyloxybiphenyl (a compound of the general formula I″ in which n is 0, $R_1$ is n-$C_8H_{17}$, $R_2$ and $R_3$ are each $CH_3$, $R_4$ is n-$C_4H_9$, $Q_1$, $Q_2$ and $Q_3$ are each -O-, X is a single bond, and

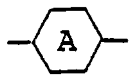 and 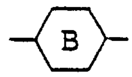 are

each 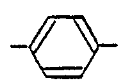 ).

0.49 g of the (2R,3R)-3-butoxy-2-butanol produced in Example 1 i) and 1.00 g of 4′-octyloxybiphenyl-4-ol were subjected to the same procedure as in Example 3 ii) to obtain 0.49 g of the title compound.

The elementary analysis and specific rotation of the compound are shown below.

| Elementary analysis | | | | | |
|---|---|---|---|---|---|
| Calcd. for $C_{28}H_{42}O_3$ | : | C, | 78.83; | H, | 9.92 |
| Found | : | C, | 78.87; | H, | 10.01 |

$[\alpha]_D^{23}$ : + 5.70° (c = 1.07, chloroform)

Example 21 Preparation of (1S,2R)-2-butoxycarbonyl-1-methylpropyl ester of 4′-octyloxy-4-biphenylcarboxylic acid (a compound of the general formula I″ in which n is 0, $R_1$ is n-$C_8H_{17}$, $R_2$ and $R_3$ are each $CH_3$, $R_4$ is n-$C_4H_9$, $Q_1$ is -O-, $Q_2$ and $Q_3$ are each

$-\overset{O}{\overset{\|}{C}}-O-$, X is a single bond, and

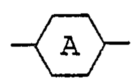 and

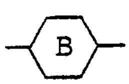 are each 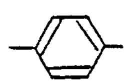 ).

1.0 g of 4′-octyloxy-4-biphenylcarboxylic acid was mixed with 5 ml of thionyl chloride. The mixture was refluxed for 5 hours. Excess thionyl chloride was removed by distillation under reduced pressure to obtain an acid chloride. This acid chloride was mixed with 0.86 g of the ethyl ester of (2R,3S)-2-methyl-3-trimethylsiloxybutyric acid produced in Example 10 ii), 25 ml of acetonitrile and 0.038 g of zinc chloride. The mixture was refluxed for 5 hours. After the completion of the reaction, the mixture was concentrated under reduced pressure. The residue was subjected to column chromatography (silica gel, developing solvent = dichloromethane) and then to recrystallization from ethanol to obtain 0.76 g of the title compound.

The specific rotation of the compound is shown below.

$[\alpha]_D^{22}$ : + 22.5° (C = 1.18, chloroform)

Example 22 Preparation of 4′-octyloxy-4-biphenyl ester of (2R,3S)-3-butoxy-2-methylbutyric acid (a compound of the general formula I″ in which n is 0, $R_1$ is n-$C_8H_{17}$, $R_2$ and $R_3$ are each $CH_3$, $R_4$ is n-$C_4H_9$, $Q_1$ and $Q_3$ are each -O-, $Q_2$ is

$-O-\overset{O}{\overset{\|}{C}}-$, X is a single bond, and

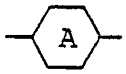 and 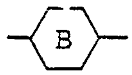 are each 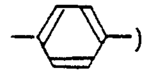).

1.27 g of the 3-butoxy-2-methylbutyric acid chloride produced in Example 15 ii) and 1.21 g of 4′-octyloxybiphenyl-4-ol were subjected to the same procedure as in Example 15 ii) to obtain 0.94 g of the title compound.

The elementary analysis and specific rotary power of the compound are shown below.

| Elementary analysis | | | | | |
|---|---|---|---|---|---|
| Calcd. for $C_{29}H_{42}O_4$ | : | C, | 76.61; | H, | 9.31 |
| Found | : | C, | 76.74; | H, | 9.48 |

$[\alpha]_D^{25}$ : + 41.7° (c = 1.18, chloroform)

Example 23 Preparation of 4′-octyloxy-4-biphenyl ester of (2R,3S)-3-methoxy-2-methylbutyric acid (a compound of the general formula I″ in which n is 0, $R_1$ is n-$C_8H_{17}$, $R_2$ and $R_3$ are each $CH_3$, $Q_1$ and $Q_3$ are each -O-, $Q_2$ is

$-O-\overset{O}{\overset{\|}{C}}-$, X is a single bond, and

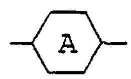 and 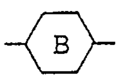 are each 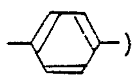).

0.58 g of the (2R,3S)-3-methoxy-2-methylbutyric acid produced in Example 11 i) and 1.0 g of 4′-octyloxybiphenyl-4-ol were subjected to the same procedure as in Example 13 to obtain 0.11 g of the title compound.

The elementary analysis and specific rotation of the compound are shown below.

| Elementary analysis | | | | | |
|---|---|---|---|---|---|
| Calcd. for $C_{26}H_{36}O_4$ | : | C, | 75.69; | H, | 8.80 |
| Found | : | C, | 75.84; | H, | 8.91 |

$[\alpha]_D^{26}$ : - 15.5° (c = 0.462, chloroform)

Example 24 Preparation of 4-octyloxyphenyl ester of 4′-[(2R,3S)-3-ethoxy-2-methylbutyryloxy]-4-biphenylcarboxylic acid (a compound of the general formula I′ in which n is 0, $R_1$ is n-$C_8H_{17}$, $R_2$ and $R_3$ are each $CH_3$, $R_4$ is $C_2H_5$, $Q_1$ and $Q_3$ are each -O-, X and $Q_2$ are each

$-O-\overset{O}{\overset{\|}{C}}-$, Y is a single bond, and

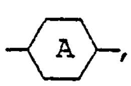, 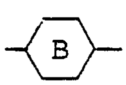 and 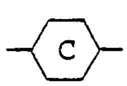 are each 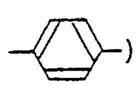).

i) Production of (2R,3S)-3-ethoxy-2-methylbutyric acid chloride

This optically active carboxylic acid can be produced by the following scheme.

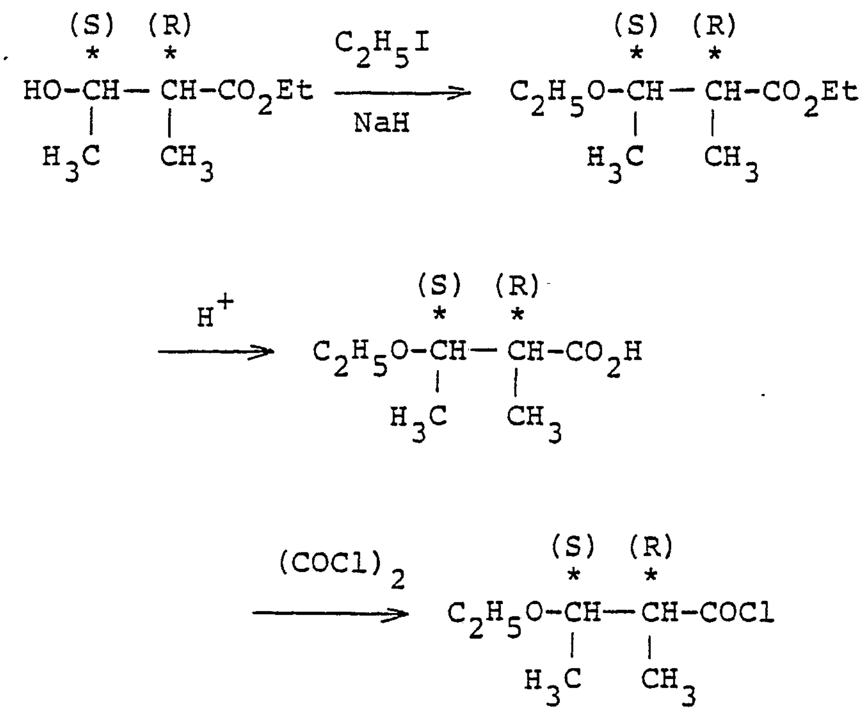


5.0 g of the (2R,3S)-3-hydroxy-2-methylbutyric acid ester produced in Example 9 i) and 15.0 g of ethyl iodide were dissolved in 50 ml of N,N-dimethylformamide. To this solution was added in small portions 2.05 g of about 60% oily sodium hydride with ice cooling. The mixture was stirred for 2 hours with ice cooling and then poured into 300 ml of ethyl acetate. The resulting mixture was stirred for 10 minutes. The insoluble materials were removed by filtration and the filtrate was washed with water four times. The organic layer was dried and concentrated. The residue was subjected to distillation under reduced pressure (b.p. 57-60 °C 28 mmHg) to obtain 2.38 g of ethyl ester of (2R,3S)-3-ethoxy-2-methylbutyric acid.

This compound was dissolved in 30 ml of dioxane. To the solution were added concentrated (36%) hydrochloric acid and 50 ml of water. The mixture was refluxed for 6 hours. After the completion of the reaction, the mixture was extracted with ethyl acetate five times. The extract was dried and concentrated. To the residue was added in small portions 7 ml of oxalyl chloride at room temperature. The mixture was stirred for 1 hour at 40 °C. Excess oxalyl chloride was removed by distillation under reduced pressure. The residue was subjected to Kugel-rohr distillation to obtain 1.18 g of (2R,3S)-3-ethoxy-2-methylbutyric acid chloride.

ii) Esterification

0.38 g of the (2R,3S)-3-ethoxy-2-methylbutyric acid chloride produced in the above i) and 100 g of 4-octyloxyphenyl ester of 4′-hydroxy-4-biphenylcarboxylic acid were dissolved in 20 ml of dry tetrahydrofuran. Thereto was added 0.38 g of dry pyridine, and the mixture was stirred overnight at room temperature. The mixture was concentrated. The residue was subjected to rough purification by column chromatography (silica gel, developing solvent = dichloromethane) and then to column chromatography [silica gel, developing solvent = n-hexane ethyl acetate (95:5)] and further to recrystallization from ethanol to obtain 0.29 g of the title compound.

The $^1$H-NMR, IR spectrum, elementary analysis and specific rotation of the compound are shown below.

IR $\nu_{max}^{KBr}$ cm$^{-1}$: 1760, 1740, 1520

$^1$H-NMR (90 MHz, $CDCl_3$)

δ: 0.89 (3H, t), 1.14-1.50 (19H, m), 1.8 (2H, b), 2.65-2.95 (1H, m), 3.35-4.03 (5H, m), 6.86-8.28 (12H, m)

| Elementary analysis | | | | | |
|---|---|---|---|---|---|
| Calcd. for $C_{34}H_{42}O_6$ | : | C, | 74.70; | H, | 7.74 |
| Found | : | C, | 74.87; | H, | 7.75 |

$[\alpha]_D^{26}$ : - 19.5° (c = 0.650, chloroform)

Example 25 Preparation of 4-propoxyphenyl ester of 4′-[(2R,3S)-3-ethoxy-2-methylbutyryloxy]-4-biphenylcarboxylic acid (a compound of the general formula I′ in which n is 0, $R_1$ is n-$C_3H_7$, $R_2$ and $R_3$ are each $CH_3$, $R_4$ is $C_2H_5$, $Q_1$ and $Q_3$ are each -O-, X and $Q_2$ are each

$-O-\overset{\overset{O}{\|}}{C}-$, Y is a single bond, and

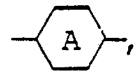,

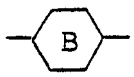 and 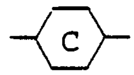 are each 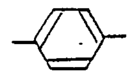).

0.39 g of the (2R,3S)-3-ethoxy-2-methylbutyric acid chloride produced in Example 24 i) and 0.83 g of 4-propoxyphenyl ester of 4′-hydroxy-4-biphenylcarboxylic acid were subjected to the same procedure as in Example 24 ii) to obtain 0.20 g of the title compound.

The elementary analysis and specific rotation of the compound are shown below.

| Elementary analysis | | | | | |
|---|---|---|---|---|---|
| Calcd. for $C_{29}H_{32}O_6$ | : | C, | 73.09; | H, | 6.77 |
| Found | : | C, | 73.17; | H, | 6.70 |

$[\alpha]_D^{25}$ : - 14.6° (c = 0.642, chloroform)

Example 26 Preparation of 4-octylphenyl ester of 4′-[(2R,3S)-3-ethoxy-2-methylbutyryloxy]-4-biphenylcarboxylic acid (a compound of the general formula I′ in which n is 0, $R_1$ is n-$C_8H_{17}$, $R_2$ and $R_3$ are each $CH_3$, $R_4$ is $C_2H_5$, $Q_1$ and Y are each a single bond, X and $Q_2$ are each

$-O-\overset{\overset{O}{\|}}{C}-$, $Q_3$ is -O-, and

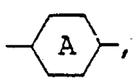, 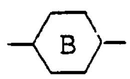 and 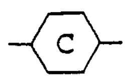 are each 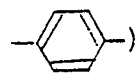).

0.39 g of the (2R,3S)-3-ethoxy-2-methylbutyric acid chloride produced in Example 24 i) and 0.95 g of 4-octylphenyl ester of 4′-hydroxy-4-biphenylcarboxylic acid were subjected to the same procedure as in Example 24 ii) to obtain 0.48 g of the title compound.

The elementary analysis and specific rotation of the compound are shown below.

| Elementary analysis | | | | | |
|---|---|---|---|---|---|
| Calcd. for $C_{34}H_{42}O_5$ | : | C, | 76.95; | H, | 7.98 |
| Found | : | C, | 76.66; | H, | 7.94 |

$[\alpha]_D^{26}$ : -16.6° (c = 0.866, chloroform)

Example 27 Preparation of 4′-octyloxy-4-biphenyl ester of 4-[(1R,2S)-2-butoxy-1-methylpropoxy]-benzoic acid (a compound of the general formula I′ in which n is 0, $R_1$ is n-$C_8H_{17}$, $R_2$ and $R_3$ are each $CH_3$, $R_4$ is n-$C_4H_9$, $Q_1$, $Q_2$ and $Q_3$ are each -O-, X is a single bond, Y is

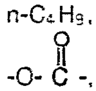 , and

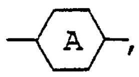 , 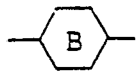 and 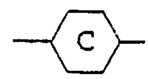

are each 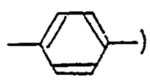 ).

i) Production of (2S,3S)-3-butoxy-2-butanol

4.00 g of (2S,3S)-(+)-2,3-butanediol and 4.04 g of n-butyl aldehyde were subjected to the same procedure as in Example 1 i) to obtain 4.14 g of (2S,3S)-3-butoxy-2-butanol (b.p. 68-71 °C/33 mmHg). The $^1$H-NMR and IR spectrum of this compound were completely agreed with those of (2R,3R)-3-butoxy-2-butanol.

ii) Condensation

0.35 g of the (2S,3S)-butoxy-2-butanol produced in the above i) and 1.00 g of 4′-octyloxy-4-biphenyl ester of 4-hydroxybenzoic acid were subjected to the same procedure as in Example 3 ii) to obtain 0.63 g of the title compound.

The elementary analysis and specific rotatory power of the compound are shown below.

| Elementary analysis | | | | | |
|---|---|---|---|---|---|
| Calcd. for $C_{35}H_{48}O_5$ | : | C, | 76.89; | H, | 8.48 |
| Found | : | C, | 76.89; | H, | 8.53 |

$[\alpha]_D^{25}$ : - 6.60° (c = 1.03, chloroform)

Example 28 Preparation of 4′-octyloxy-4-biphenyl ester of 4-[(1S,2S)-2-butoxy-1-methylpropoxy]-benzoic acid (a compound of the general formula I′ in which n is 0, $R_1$ is $C_8H_{17}$, $R_2$ and $R_3$ are each $CH_3$, $R_4$ is n-$C_4H_9$, $Q_1$, $Q_2$ and $Q_3$ are each -O-, X is a single bond, Y is

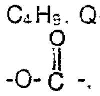 , and

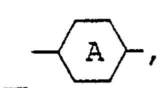 , 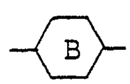 and

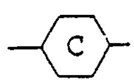 are each 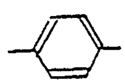 ).

i) Preparation of (2R, 3S)-3-butoxy-2-butanol

This optically active alcohol was produced by subjecting the (2S,3S)-3-butoxy-2-butanol produced in Example 27 i) to the inversion of hydroxyl group in the same manner as in Example 3 i).

The title alcohol can also be produced from (2R,3R)-(-)-2,3-butanediol in the following scheme.

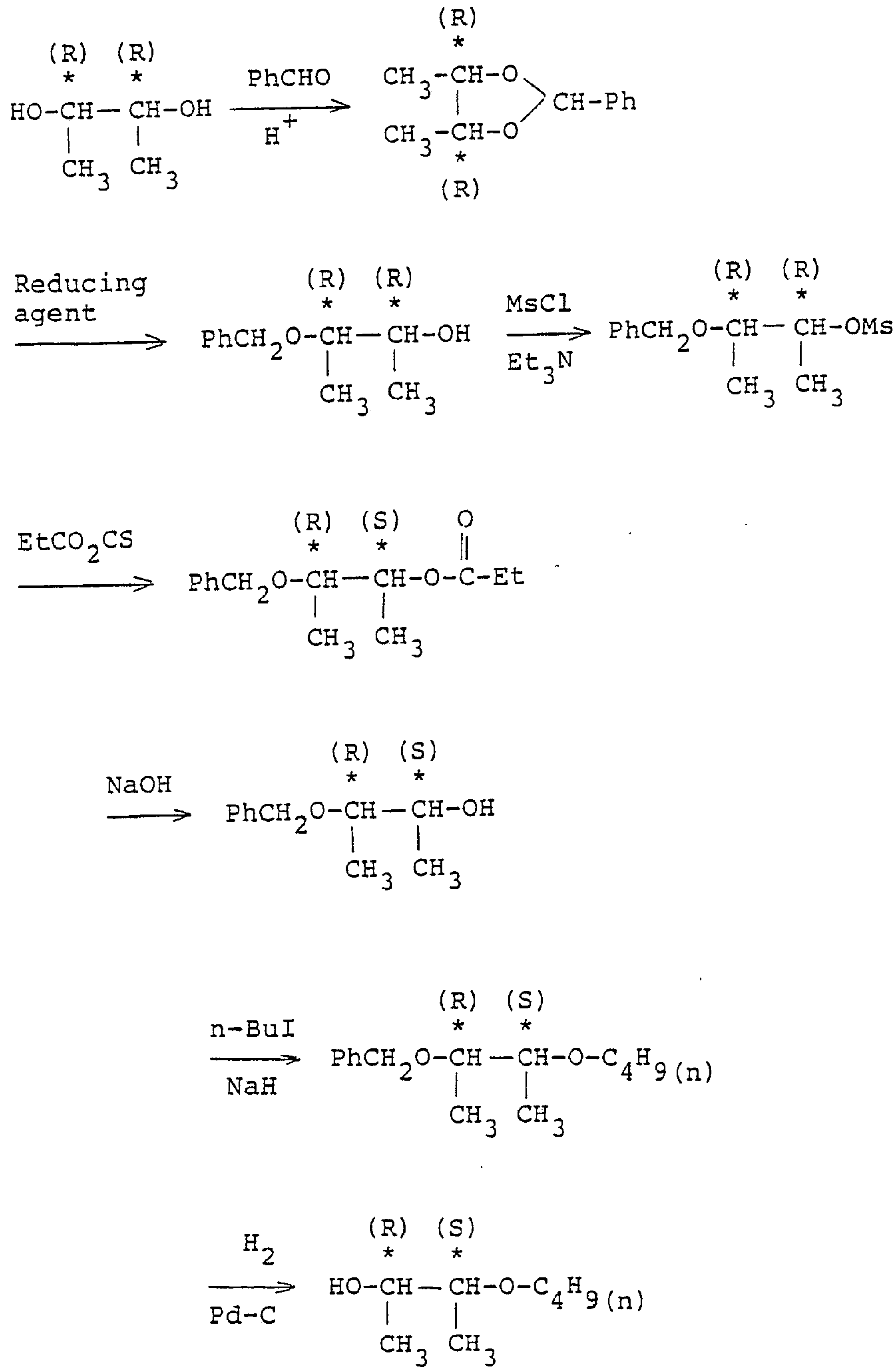


That is, 5.15 g of (2R,3R)-(-)-2,3-butanediol and 6.06 g of benzaldehyde were dissolved in 100 ml of benzene. Thereto was added 0.23 g of p-toluenesulfonic acid monohydrate. The mixture was refluxed for 3 hours with dehydrating according to a conventional method. After the completion of the reaction, 0.1 ml of pyridine was added. The solvent was removed by distillation under reduced pressure. The residue was subjected to distillation under reduced pressure (b.p. 56-59 °C/mmHg) to obtain 9.87 g of a benzylidene compound.

13.87 g of aluminum chloride was dissolved in 100 ml of dry ether with ice cooling. Thereto was added 0.99 g of lithium aluminum hydride in small portions. Thereto was dropwise added 9.27 g of the above

obtained benzylidene compound with ice cooling. The mixture was stirred overnight at room temperature. After the completion of the reaction, 100 ml of 10% sulfuric acid was added with stirring under ice cooling. The ether layer was separated and the aqueous layer was extracted with ether twice. The combined ether layer was dried and concentrated. The concentrate was subjected to distillation under reduced pressure (b.p. 72-74° C/1 mmHg) to obtain 9.03 g of (2R,3R)-3-benzyloxy-2-butanol.

In 30 ml of dichloromethane were dissolved 3.00 g of (2R,3R)-3-benzyloxy-2-butanol and 2.19 g of triethylamine. Thereto was dropwise added 2.48 g of methanesulfonyl chloride with stirring under ice cooling. The mixture was stirred for 3 hours at room temperature. The mixture was poured into water. The resulting dichloromethane layer was separated. The aqueous layer was extracted with dichloromethane twice. The combined dichloromethane solution was washed with dilute hydrochloric acid and an aqueous sodium bicarbonate solution in this order, dried and concentrated. The residue was subjected to column chromatography (silica gel, developing solvent = dichloromethane) to obtain 4.14 g of oily (2R,3R)-3-benzyloxy-2-butyl ester of methanesulfonic acid.

10.85 g of cesium carbonate was dissolved in 200 ml of methanol. 7.41 g of propionic acid was added dropwise. The mixture was stirred for 1 hour at room temperature. Methanol was removed by distillation. The residue was mixed with toluene and subjected to distillation to remove toluene (100 ml x three times), whereby a white powder of cesium propionate was obtained. This powder and 4.07 g of the above obtained (2R,3R)-3- benzyloxy-2-butyl ester of methanesulfonic acid were dissolved in 120 ml of dry N,N-dimethylformamide. The mixture was heated at 100° C overnight. After the completion of the reaction, the mixture was poured into water and extracted with ether three times. The extract was washed with water (two times), dilute hydrochloric acid (once) and an aqueous sodium bicarbonate solution (once) in this order, dried and concentrated. The residue was subjected to Kugel-rohr distillation (1 mmHg) to obtain 2.81 g of (2S,3R)-3-benzyloxy-2-butyl ester of propionic acid.

2.70 g of (2S,3R)-3-benzyloxy-2-butyl ester of propionic acid was dissolved in 40 ml of methanol. Thereto was added 14 ml of a 1 N solution of sodium hydroxide in methanol. The mixture was stirred overnight at room temperature. After the completion of the reaction, methanol was removed by distillation. The residue was diluted with water and extracted with dichloromethane three times. The extract was dried and concentrated. The residue was subjected to Kugel-rohr distillation (1 mmHg) to obtain 2.00 g of (2S,3R)-3-benzyloxy-2-butanol.

1.90 g of (2S,3R)-3-benzyloxy-2-butanol was dissolved in 50 ml of dry N,N-dimethylformamide. Thereto was added in small portions 2.53 g of about 60% oily sodium hydride. The mixture was stirred for 1 hour at room temperature. 11.63 g of butyl iodide was dropped with ice cooling. The mixture was stirred overnight at room temperature. The reaction mixture was poured into water. The resulting mixture was extracted with ether three times. The extract was washed with water three times, dried and concentrated. The residue was subjected to Kugel-rohr distillation (1 mmHg) to obtain 2.05 g of (2R,3S)-2-benzyloxy-3-butoxybutane.

1.94 g of (2R,3S)-2-benzyloxy-3-butoxybutane was dissolved in a mixture of 80 ml of ethanol and 8 ml of 2 N hydrochloric acid. Thereto was added 200 mg of 5% palladium carbon. The mixture was subjected to hydrogenation at the atmospheric pressure at room temperature. After the completion of the reaction, the catalyst was removed by filtration and the filtrate was subjected to distillation. The residue was diluted with water and extracted with dichloromethane three times. The extract was dried and concentrated. The residue was subjected to Kugel-rohr distillation (30 mmHg) to obtain 0.76 g of (2R,3S)-3-butoxy-2-butanol. The $^1H$-NMR and IR spectrum of this compound were completely agreed with those of (2S,3R)-3-butoxy-2-butanol.

ii) Condensation

0.35 g of the (2R,3S)-3-butoxy-2-butanol produced in the above i) and 1.00 g of 4′-octyloxy-4-biphenyl ester of 4-hydroxybenzoic acid were subjected to the same procedure as in Example 3 ii) to obtain 0.91 g of the title compound.

The elementary analysis and specific rotatory power of the compound are shown below.

| Elementary analysis | | | | | |
|---|---|---|---|---|---|
| Calcd. for $C_{35}H_{46}O_5$ | : | C, | 76.89; | H, | 8.48 |
| Found | : | C, | 76.93; | H, | 8.50 |

$[\alpha]_D^{24}$ : 0 (c = 1.03, chloroform)

Test Examples 1-28

The optically active compounds produced in Examples 1-28 were measured for phases, phase transition temperatures and spontaneous polarization. The results of the measurements of these properties are shown in Table 1.

Incidentally, the phases and phase transition temperatures were determined by using a polarizing microscope and DSC (differential scanning calorimetry).

The measurement of spontaneous polarization was conducted in accordance with the above mentioned Sowyer-Tower method. The spontaneous polarization is a value at a temperature which is 10 °C lower than the upper limit of chiral smectic C phase.

In Table 1, the phases (liquid crystal phases, etc.) are abbreviated as follows:

Iso: Isotropic liquid phase
$S_A$: Smectic A phase
Sc* Chiral smectic C phase
$S_1$, $S_2$: Smectic phase which is not identified.
Ch: Cholesteric phase
K: Crystalline phase .

Table 1

| Test No. | Optically active compound of general formula $n\text{-}C_8H_{17}-Q_1-M-Q_2-\overset{*}{C}H(CH_3)-\overset{*}{C}H(CH_3)-Q_3-R_4$ | | | | | Example No. |
|---|---|---|---|---|---|---|
| | $R_4$ | $Q_1$ | $Q_2$ | $Q_3$ | M | |
| 1 | $-C_4H_9$, | -O-, | $-\overset{\overset{O}{\|}}{C}-O-$, | -O-, | 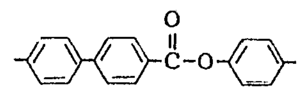 | 1 (1R, 2R) |
| 2 | $-C_4H_9$, | -O-, | $-\overset{\overset{O}{\|}}{C}-O-$, | -O-, | 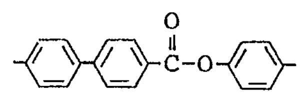 | 2 (1S, 2R) |
| 3 | $-C_2H_5$, | -O-, | $-\overset{\overset{O}{\|}}{C}-O-$, | $-\overset{\overset{O}{\|}}{C}-O-$, | 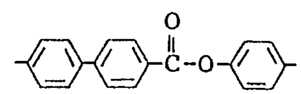 | 9 |
| 4 | $-C_4H_9$, | -O-, | $-\overset{\overset{O}{\|}}{C}-O-$, | $-\overset{\overset{O}{\|}}{C}-O-$, | 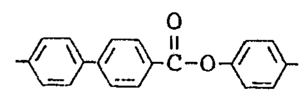 | 10 |
| 5 | $-C_4H_9$, | -O-, | -O-, | -O-, | 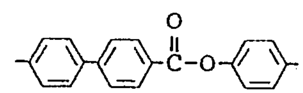 | 5 |
| 6 | $-C_4H_9$, | -O-, | -O-, | -O-, | 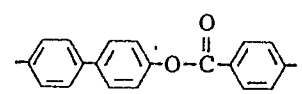 | 3 (1R, 2R) |
| 7 | $-C_4H_9$, | -O-, | -O-, | -O-, | 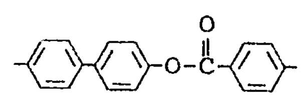 | 4 (1S, 2R) |

- Cont'd -

| Properties ~~Measurements~~ | |
|---|---|
| Phases and phase transition temperatures | Spontaneous polarization ($nC/cm^2$) |
| $Iso \underset{147}{\rightleftarrows} S_A \underset{113}{\rightleftarrows} Sc^* \rightleftarrows K$ | 3.5 |
| $Iso \underset{143}{\rightleftarrows} S_A \underset{115}{\rightleftarrows} Sc^* \underset{82}{\rightleftarrows} K$ | 27 |
| $Iso \underset{143}{\rightleftarrows} S_A \underset{109}{\rightleftarrows} Sc^* \underset{90}{\rightleftarrows} K$ | 27 |
| $Iso \underset{126}{\rightleftarrows} S_A \underset{93}{\rightleftarrows} Sc^* \underset{76}{\rightleftarrows} K$ | 14.5 |
| $Iso \underset{145}{\rightleftarrows} S_A \underset{101}{\rightleftarrows} Sc^* \underset{67}{\longleftarrow} K$; $Sc^* \nearrow S_1 \searrow K$ | 21 |
| $Iso \underset{94}{\rightleftarrows} Ch \underset{86}{\rightleftarrows} Sc^* \underset{76}{\rightleftarrows} K$ | 68 |
| $Iso \underset{113}{\rightleftarrows} Ch \underset{88}{\rightleftarrows} Sc^* \underset{73}{\rightleftarrows} K$ | 27 |

- Cont'd -

Table 1 (Cont'd)

| | | | | | | |
|---|---|---|---|---|---|---|
| 8 | $-C_8H_{17}$, | -O-, | -O-, | -O-, | 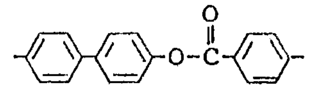 | 8 |
| 9 | $-C_4H_9$, | -O-, | -O-, | -O-, | 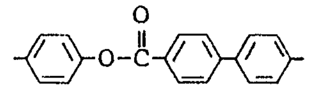 | 6 |
| 10 | $-C_4H_9$, | -O-, | -O-, | -O-, | 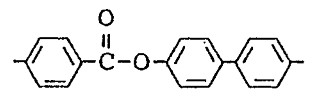 | 7 |
| 11 | $-C_4H_9$, | -O-, | -O-, | -O-, | 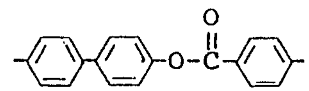 | 27 |
| 12 | $-CH_3$, | -O-, | 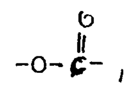 | -O-, | 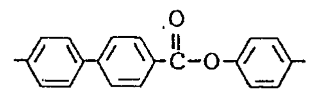 | 11 |
| 13 | $-C_4H_9$, | -O-, | 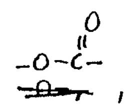 | -O-, | 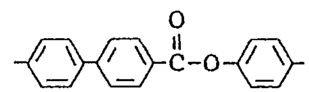 | 15 |
| 14 | $-CH_3$, | -O-, | 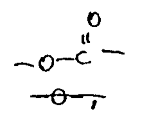 | -O-, | 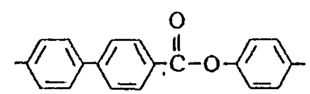 | 13 |
| 15 | $-C_4H_9$, | -O-, | 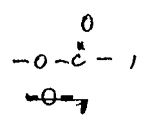 | -O-, | 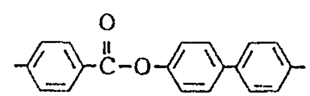 | 17 |

- Cont'd -

| | |
|---|---|
| Iso ⇄ 101 Ch ⇄ 83 Sc* ⇄ 64 K | 33 |
| Iso ⇄ 112 Ch ⇄ 91 Sc* $S_1$ → $S_2$ K ← 53 | 45 |
| Iso ⇄ 120 Ch ⇄ 90 Sc* ⇄ 65 $S_1$ ⇄ 57 K | 26 |
| Iso ⇄ 114.5 Ch ⇄ 89.7 Sc* ⇄ 63.9 ~~86.4~~ 14.0 K | 44 |
| Iso ⇄ 178 Ch ⇄ 165 $S_A$ ⇄ 136 Sc* K 57 50 $S_1$ | 21 |
| Iso ⇄ 163 Ch ⇄ 161 $S_A$ ⇄ 127 Sc* $S_1$ → $S_2$ K ← 61 | 30 |
| Iso ⇄ 159 Ch ⇄ 112 Sc* $S_1$ K ← 74 | 44 |
| Iso ⇄ 151 Ch ⇄ 119 Sc* $S_1$ K ← 70 | 44 |

- Cont'd -

Table 1 (Cont'd)

| | | | | | | |
|---|---|---|---|---|---|---|
| 16 | $-CH_3$, | -O-, | ~~-O-,~~ $-O-\overset{O}{\overset{\|}{C}}-$, | -O-, | 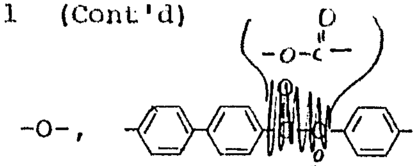 | 12 |
| 17 | $-C_4H_9$, | -O-, | ~~-O-,~~ $-O-\overset{O}{\overset{\|}{C}}-$, | -O-, | 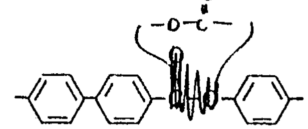 | 16 |
| 18 | $-CH_3$, | -O-, | ~~-O-,~~ $-O-\overset{O}{\overset{\|}{C}}-$, | -O-, | 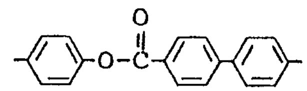 | 14 |
| 19 | $-C_4H_9$, | -O-, | ~~-O-,~~ $-O-\overset{O}{\overset{\|}{C}}-$, | -O-, | 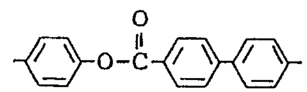 | 18 |
| 20 | $-C_2H_5$, | -O-, | ~~-O-,~~ $-O-\overset{O}{\overset{\|}{C}}-$, | -O-, | 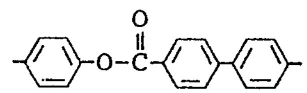 | 24 |
| 21 | $-C_4H_9$, | -O-, | $-\overset{O}{\overset{\|}{C}}-O-$, | -O-, | 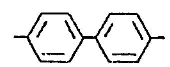 | 19 |
| 22 | $-C_4H_9$, | -O-, | -O-, | -O-, | 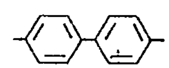 | 20 |
| 23 | $-C_4H_9$, | -O-, | $-\overset{O}{\overset{\|}{C}}-O-$, | $-\overset{O}{\overset{\|}{C}}-O-$, | 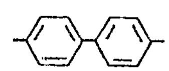 | 21 |

- Cont'd -

| | |
|---|---|
| $\mathrm{Iso} \underset{157}{\rightleftarrows} \mathrm{Ch} \underset{120}{\rightleftarrows} \mathrm{Sc^*} \underset{94}{\rightleftarrows} \mathrm{K}$ | 11 |
| $\mathrm{Iso} \underset{145}{\rightleftarrows} \mathrm{Ch} \underset{121}{\rightleftarrows} \mathrm{Sc^*} \underset{98}{\rightleftarrows} \mathrm{K}$ | 26 |
| $\mathrm{Iso} \underset{155}{\rightleftarrows} \mathrm{Ch} \underset{118}{\rightleftarrows} \mathrm{Sc^*} \nearrow S_1 \searrow \mathrm{K}$, $\mathrm{K} \underset{46}{\longrightarrow}$ ... $\mathrm{Sc^*}$ | 57 |
| $\mathrm{Iso} \underset{145}{\rightleftarrows} \mathrm{Ch} \underset{123}{\rightleftarrows} \mathrm{Sc^*} \underset{75}{\rightleftarrows} \mathrm{K}$ | 45 |
| $\mathrm{Iso} \underset{153}{\rightleftarrows} \mathrm{Ch} \underset{121}{\rightleftarrows} \mathrm{Sc^*} \nearrow S_1 \searrow \mathrm{K}$, $\mathrm{K} \underset{74}{\longrightarrow}$ ... $\mathrm{Sc^*}$ | 73 |
| $\mathrm{Iso} \underset{58}{\rightleftarrows} \mathrm{K}$ | not ~~Not available~~ determined |
| $\mathrm{Iso} \underset{33}{\rightleftarrows} \mathrm{K}$ | not ~~Not available~~ determined |
| $\mathrm{Iso} \underset{45}{\rightleftarrows} \mathrm{K}$ | not ~~Not available~~ determined |

- Cont'd -

Table 1 (Cont'd)

| | | |
|---|---|---|
| 24 | $-C_4H_9$, $-O-$, $-O-\overset{O}{\overset{\|}{C}}-$, $-O-$, 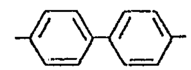 | 22 |
| 25 | $-C_4H_9$, $-O-$, $-O-\overset{O}{\overset{\|}{C}}-$, $-O-$, 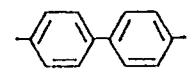 | 23 |
| ~~27~~ 26 | $-C_2H_5$, $-$ , $-O-\overset{O}{\overset{\|}{C}}-$, $-O-$, 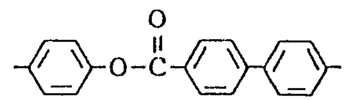 | 26 |
| ~~28~~ 27 | $-C_4H_9$, $-O-$, $-O-\overset{O}{\overset{\|}{C}}-$, $-O-$, 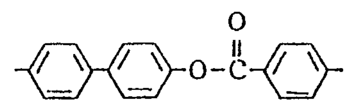 | 28 |
| Optically active compound of general formula $n\text{-}C_3H_7-Q_1-M-Q_2-\overset{*}{C}H(CH_3)-\overset{*}{C}H(CH_3)-Q_3-R_4$ | | |
| ~~26~~ 28 | $-C_2H_5$, $-O-$, $-O-\overset{O}{\overset{\|}{C}}-$, $-O-$, 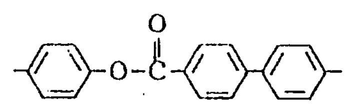 | 25 |

| | |
|---|---|
| Iso $\underset{41}{\rightleftarrows}$ Ch $\underset{36}{\rightleftarrows}$ Sc* $\underset{30}{\rightleftarrows}$ K | 30 |
| Iso $\underset{49}{\rightleftarrows}$ Ch $\underset{46}{\rightleftarrows}$ Sc* $\underset{41}{\leftarrow}$ K; Sc* $\nearrow$ $S_1$ $\searrow$ K | Not ~~available~~ determined |
| Iso $\overset{128.8}{\longleftrightarrow}$ Ch $\overset{101.1}{\longleftrightarrow}$ Sc* $\underset{86.4}{\overset{58.9}{\rightleftarrows}}$ K | 36 |
| Iso $\overset{41.3}{\longleftrightarrow}$ Ch $\overset{83.8}{\longleftrightarrow}$ Sc* $\underset{76.0}{\overset{64.7}{\rightleftarrows}}$ K | Not ~~available~~ determined |
| | |
| Iso $\overset{163.4}{\longleftrightarrow}$ Ch $\underset{135.3}{\overset{117.3}{\rightleftarrows}}$ K | not determined |

Examples 29-90

The compounds of Examples 29-90 were prepared by a similar procedure as that described previously. The structural formulae, phases, phase transition temperatures and elementary analyses of these compounds are shown in Table 2. As to the compounds containing a dichiral side chain component whose

preparation has not been explained above (these compounds are marked with an * asterisk in Table 2), their preparation is described in Reference Examples 1-5 which appear after Table 2. Incidentally, the structural formulae of compounds whose elementary analyses are not given in Table 2 were determined based on various spectral data.

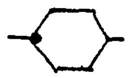

stands for trans-cyclohexyl.

Table 2

| Example No. | Structural formula | Phases and Phase transition temperatures (°C) |
| --- | --- | --- |
| 29 | 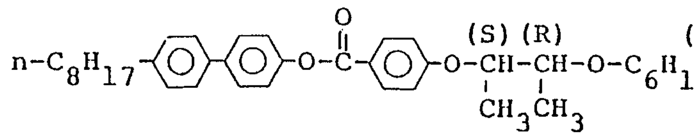 (*1) | K ⇄ (54.2 ←, 65.6 →) Sc* ↔ 85.6 Ch ↔ 107.6 Iso |
| 30 | 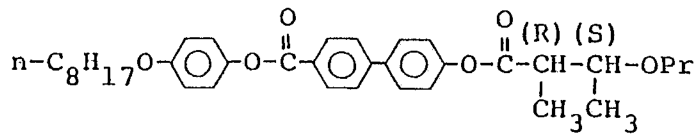 | K ⇄ (53.7 ←, 84.1 →) Sc* ↔ 126.3 Ch ↔ 150.5 Iso |
| 31 | 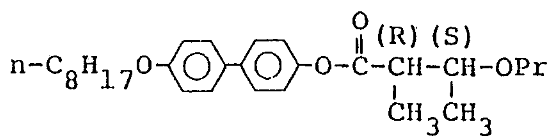 | K ⇄ (33.5 ←, 37.5 →) Sc* ↔ 46.3 Ch ↔ 50.2 Iso |
| 32 | 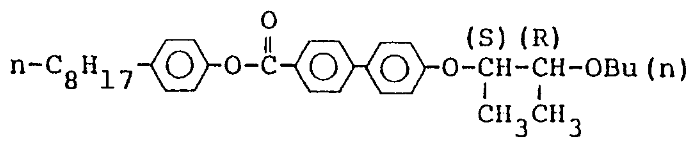 | K ← 25.6 Sc* ← 56.2 Ch; K → 53.0 Ch; Ch ↔ 81.3 Iso |
| 33 | 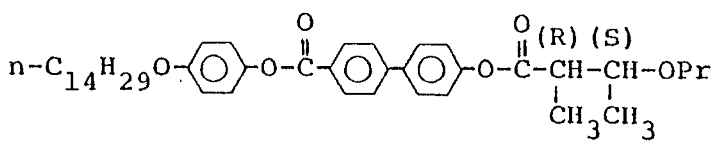 | K ⇄ (64.4 ←, 72.5 →) Sc* ↔ 124.1 Ch ↔ 137.1 Iso |
| 34 | 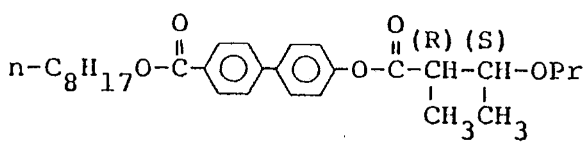 | k → 7.9 $S_1$ → 21.3 Iso; Iso → -8∿-26 k |

*1 See Reference Example 1.

- Cont'd -

| Spontaneous polarization ($nC/cm^2$) | Elementary analysis (Compositional formula, calculated value and observed value) |
| --- | --- |
| 43 | $C_{37}H_{50}O_5$ : C,77.31 ; H, 8.77.<br>: C,77.57 ; H, 8.88. |
| 29 | $C_{35}H_{44}O_6$ : C,74.97 ; H, 7.91.<br>: C,74.98 ; H, 7.69. |
| 92 | $C_{28}H_{40}O_4$ : C,76.33 ; H, 9.15.<br>: C,76.04 ; H, 9.17. |
| 42 | $C_{35}H_{46}O_4$ : C,79.21 ; H, 8.74.<br>: C,79.33 ; H, 8.78. |
| 48 | $C_{41}H_{56}O_6$ : C,76.36 ; H, 8.75.<br>: C,76.22 ; H, 8.73. |
| - | $C_{29}H_{40}O_5$ : C,74.33 ; H, 8.60.<br>: C,74.62 ; H, 8.87. |

- Cont'd -

Table 2 (Cont'd)

| | | |
|---|---|---|
| 35 | 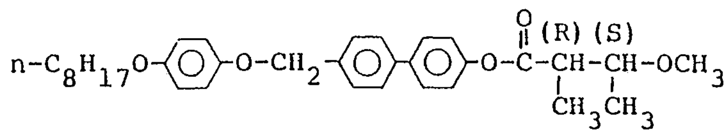 | 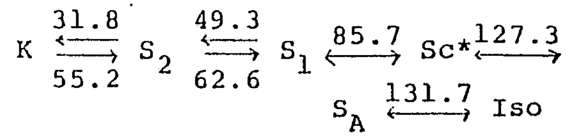 |
| 36 | 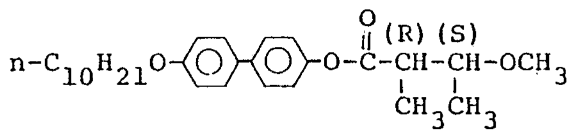 | 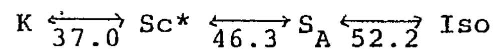 |
| 37 | 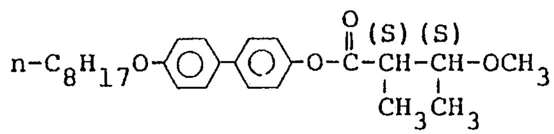 (*3) | 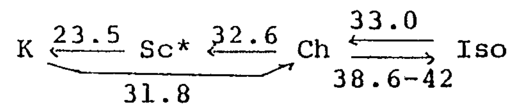 |
| 38 | 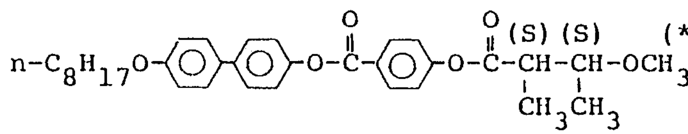 (*3) | 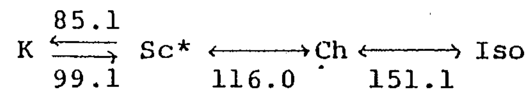 |
| 39 | 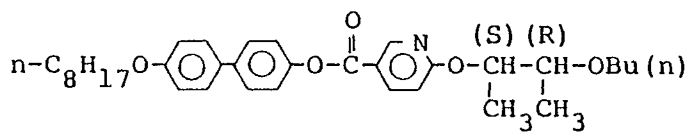 | 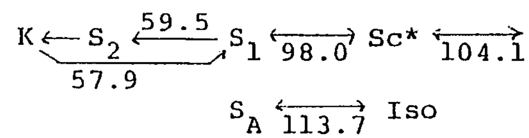 |
| 40 | 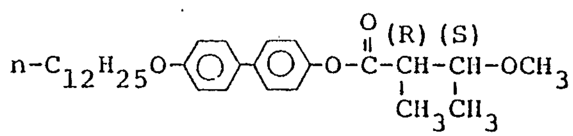 | 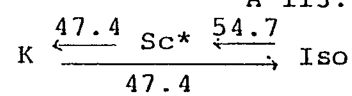 |
| 41 | 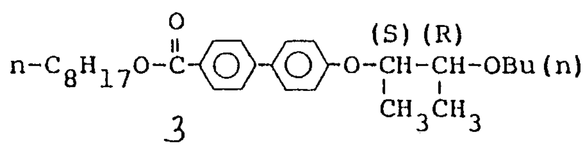 | 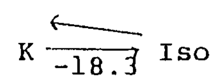 |

*3 See Reference Example 3.

- Cont'd -

| | | |
|---|---|---|
| 32.5 | $C_{33}H_{42}O_5$ | : C,76.42 ; H, 8.16.<br>: C,76.37 ; H, 8.04. |
| 46.4 | $C_{28}H_{40}O_4$ | : C,76.33 ; H, 9.15.<br>: C,76.11 ; H, 9.20. |
| 8 | $C_{26}H_{36}O_4$ | : C,75.69 ; H, 8.80.<br>: C,75.83 ; H, 8.86. |
| 8(-15°C) | $C_{33}H_{40}O_6$ | : C,74.41 ; H, 7.57.<br>: C,74.47 ; H, 7.54. |
| - | $C_{34}H_{45}O_5$ | : C,74.56 ; H, 8.28 ; N, 2.56.<br>: C,74.64 ; H, 8.31 ; N, 2.25. |
| - | $C_{30}H_{44}O_4$ | : C,76.88 ; H, 9.46.<br>: C,76.64 ; H, 9.51. |
| - | $C_{29}H_{42}O_4$ | : C,76.61 ; H, 9.31.<br>: C,76.97 ; H, 9.48. |

- Cont'd -

Table 2 (Cont'd)

| | | |
|---|---|---|
| 42 | 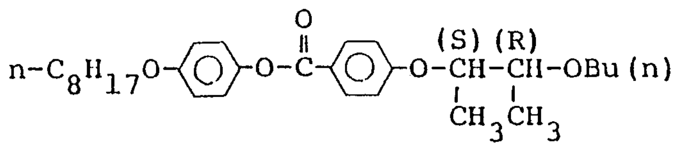 | K ⇄ Iso (18.4 ←; 30.3 →) |
| 43 | 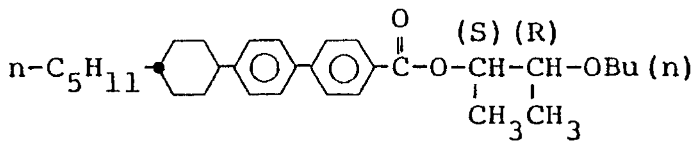 | K ⇄ $S_1$ (64.2) ⇄ $S_2$ (69.6) ⇄ Iso (87.7 ←; 78.3~87.5 →) |
| 44 | 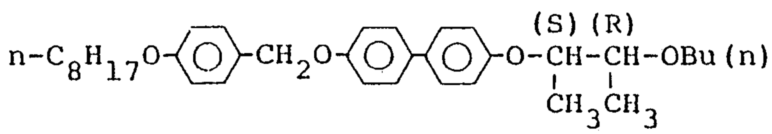 | K ← $S_1$ (26.6); $S_1$ ← Sc* (56.4); K → Sc* (52.3); Sc* ⇄ Iso (110.3) |
| 45 | 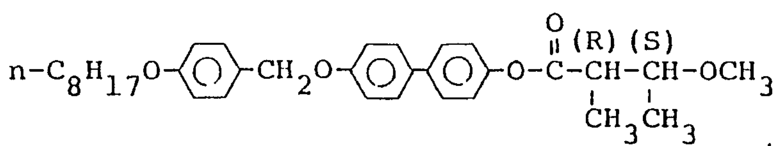 | K ⇄ $S_2$ (51.4 ←; 76.7 →) ⇄ $S_1$ (90.8 ←; 95.1 →) ⇄ Sc* (106.1)<br>Sc* ⇄ Iso (137.0) |
| 46 | 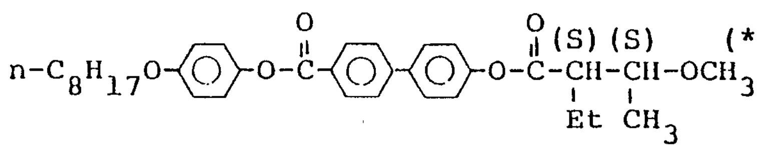 (*4) | K ⇄ Sc* (29.2 ←; 75.0 →) ⇄ Ch (64.2 ←; 77.2 →) ⇄ Iso (85.0-97.0) |
| 47 | 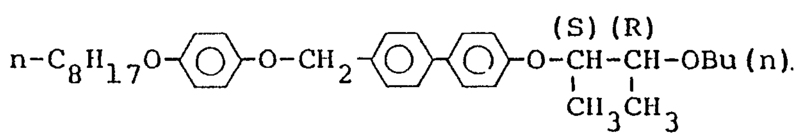 | K ← Sc* (95.9); Sc* ← Iso (104.1); K → Iso (104.1) |

*4 See Reference Example 4.

- Cont'd -

| | | |
|---|---|---|
| – | $C_{29}H_{42}O_5$ | : C,74.01 ; H, 8.99.<br>: C,73.99 ; H, 9.02. |
| – | $C_{32}H_{46}O_3$ | : C,80.29 ; H, 9.69.<br>: C,80.17 ; H, 9.57. |
| 34.5 | $C_{35}H_{48}O_4$ | : C,78.91 ; H, 9.08.<br>: C,79.22 ; H, 9.14. |
| 38 | $C_{33}H_{42}O_5$ | : C,76.42 ; H, 8.16.<br>: C,76.30 ; H, 8.19. |
| small | $C_{34}H_{42}O_6$ | : C,74.70 ; H, 7.74.<br>: C,74.53 ; H, 7.65. |
| 28(99°C) | $C_{35}H_{48}O_4$ | : C,78.91 ; H, 9.08.<br>: C,79.05 ; H, 9.14. |

- Cont'd -

Table 2 (Cont'd)

| | | | |
|---|---|---|---|
| 48 | 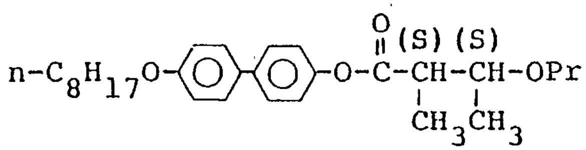 | (*3) | K ←8.0— Sc* ←13.0— $S_A$ ←21.3— Iso<br>K —32.8→ Iso |
| 49 | 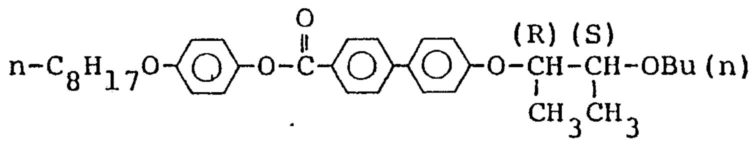 | | K ←11.3— $S_2$ ←22.6— $S_1$ ←35.2— Sc* ←<br>K —54.8→ —90.5→ Ch ←112.8→ Iso |
| 50 | 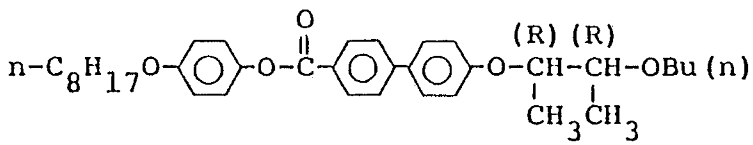 | | K ←13.7 / 54.9→ Sc* ←77.7→ Iso |
| 51 | 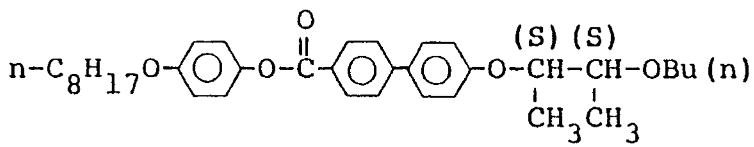 | | K ←13.6 / 54.5→ Sc* ←77.2→ Iso |
| 52 | 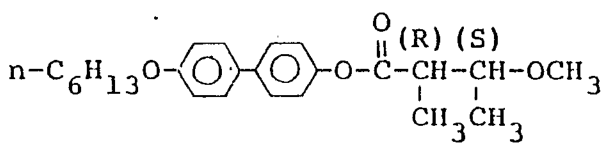 | | K ←39.6 / 42.4→ Sc* ←46.2→ Ch ←46.9→ Iso |
| 53 | 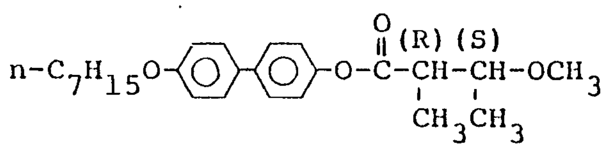 | | K ←26.0— $S_1$ ←28.2— Sc* ←42.8—<br>K —39.4→ —→ Ch ←43.3→ Iso |

*3 See Reference Example 3.

- Cont'd -

| | | |
|---|---|---|
| – | $C_{28}H_{40}O_4$ | : C,76.33 ; H, 9.15.<br>: C,76.38 ; H, 9.28. |
| 35.6 | $C_{35}H_{46}O_5$ | : C,76.89 ; H, 8.48.<br>: C,76.98 ; H, 8.51. |
| – | $C_{35}H_{46}O_6$ | : C,76.89 ; H, 8.48.<br>: C,77.00 ; H, 8.54. |
| small | $C_{35}H_{46}O_5$ | : C,76.89 ; H, 8.48.<br>: C,76.72 ; H, 8.47. |
| 156(42°C) | $C_{24}H_{32}O_4$ | : C,74.97 ; H, 8.39.<br>: C,75.06 ; H, 8.41. |
| 110(40°C) | $C_{25}H_{34}O_4$ | : C,75.34 ; H, 8.60.<br>: C,75.41 ; H, 8.63. |

- Cont'd -

Table 2 (Cont'd)

| | | |
|---|---|---|
| 54 | 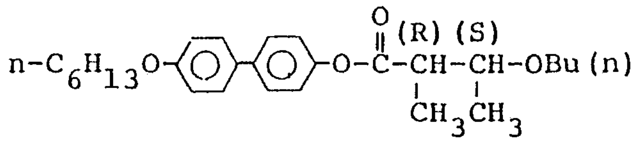 | K (26.3 ←) Sc* (43.7 ←) Ch (48.8 ←) Iso; K (→ 48.8) Iso |
| 55 | 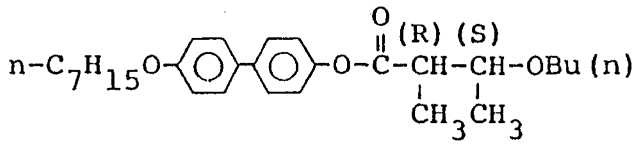 | K (-21.0 ←, → 38.0) Sc* (↔ 41.6) Ch (↔ 44.5) Iso |
| 56 | 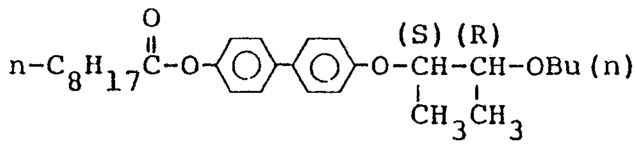 | K (28.2 ←, → 40.4) Iso |
| 57 | 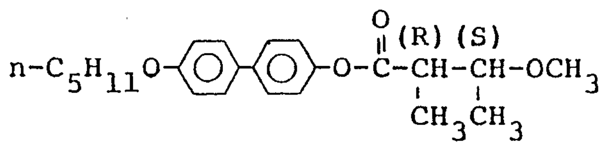 | K (43.7 ←, → 50.3) Iso |
| 58 | 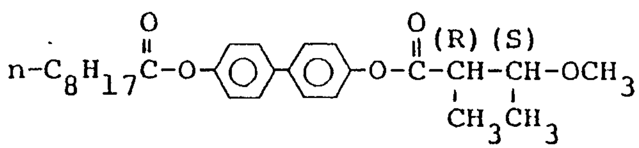 | K (35.9 ←, → 37.5) Sc* (↔ 46.9) Iso |
| 59 | 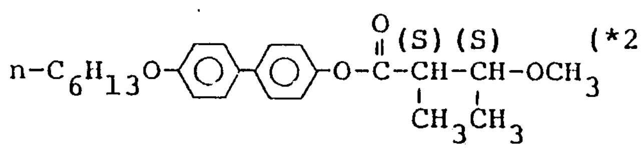 (*2) | K (↔ 33.2) Iso |

\- Cont'd -

*2 See Reference Example 2.

| | | |
|---|---|---|
| 78.8(38°C) | $C_{27}H_{38}O_4$ | : C,76.02 ; H, 8.98.<br>: C,76.25 ; H, 9.08. |
| 82 | $C_{28}H_{40}O_4$ | : C,76.33 ; H, 9.15.<br>: C,76.17 ; H, 9.18. |
| - | $C_{29}H_{42}O_4$ | : C,76.61 ; H, 9.31.<br>: C,76.43 ; H, 9.27. |
| - | $C_{23}H_{30}O_4$ | : C,74.56 ; H, 8.16.<br>: C,74.67 ; H, 8.03. |
| 111 | $C_{27}H_{36}O_5$ | : C,73.61 ; H, 8.24.<br>: C,73.84 ; H, 8.35. |
| - | $C_{24}H_{32}O_4$ | : C,74.97 ; H, 8.39.<br>: C,74.73 ; H, 8.45. |

- Cont'd -

Table 2 (Cont'd)

| | | |
|---|---|---|
| 60 | 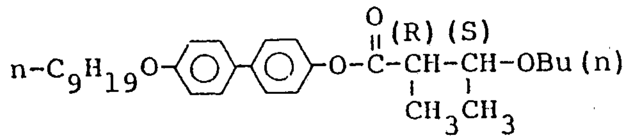 | K $\underset{42.2}{\overset{36.3}{\rightleftarrows}}$ Sc* $\underset{51.2}{\rightleftarrows}$ Ch $\underset{52.4}{\rightleftarrows}$ Iso |
| 61 | 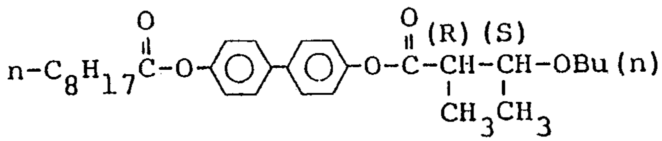 | K $\underset{46.6}{\overset{35.2}{\leftarrow}}$ $S_2$ $\overset{38.1}{\leftarrow}$ $S_1$ $\overline{\ 49.0\ }$ $\longrightarrow$ Sc* $\underset{56.2}{\longrightarrow}$ Iso |
| 62 | 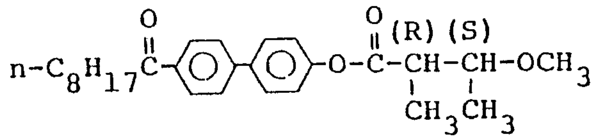 | K $\underset{84.7}{\overset{77.8}{\rightleftarrows}}$ Iso |
| 63 | 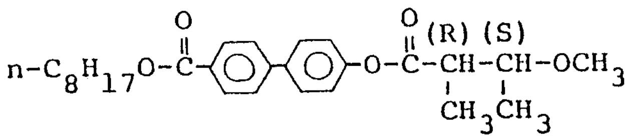 | K $\underset{23.7}{\overset{0.4}{\rightleftarrows}}$ Iso |
| 64 | 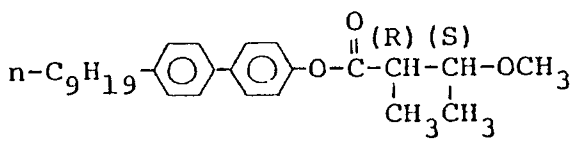 | K $\overset{-7.4}{\leftarrow}$ Sc* $\overset{13.4}{\leftarrow}$ $S_A$ $\overset{20.8}{\leftarrow}$ Iso; K $\underset{31.2}{\longrightarrow}$ Iso |
| 65 | 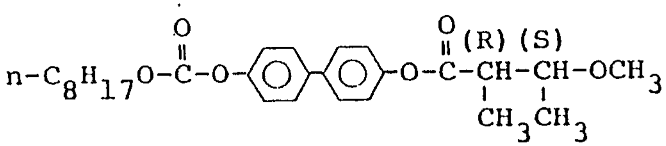 | K $\overset{0.3}{\leftarrow}$ Sc* $\overset{10.5}{\leftarrow}$ Ch $\overset{19.5}{\leftarrow}$ Iso; K $\underset{36.8}{\longrightarrow}$ Iso |

- Cont'd -

| | | |
|---|---|---|
| 60 | $C_{29}H_{44}O_4$ | : C,76.27 ; H, 9.71.<br>: C,76.31 ; H, 9.36. |
| 25(-6°C) | $C_{30}H_{42}O_5$ | : C,74.65 ; H, 8.77.<br>: C,74.40 ; H, 8.48. |
| - | $C_{27}H_{36}O_4$ | : C,76.45 ; H, 8.55.<br>: C,76.50 ; H, 8.70. |
| - | $C_{27}H_{36}O_5$ | : C,73.61 ; H, 8.24.<br>: C,73.83 ; H, 8.21. |
| 90 | $C_{27}H_{38}O_3$ | : C,78.98 ; H, 9.33.<br>: C,79.01 ; H, 9.35. |
| 78(-5°C) | $C_{27}H_{36}O_6$ | : C,71.03 ; H, 7.95.<br>: C,71.13 ; H, 8.09. |

- Cont'd -

Table 2 (Cont'd)

| | | |
|---|---|---|
| 66 | 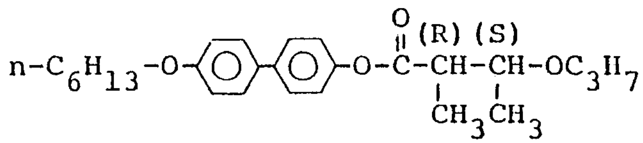 | K ←22.9— $S_2$ ←27.9— $S_1$ ←37.6— Sc* ←; K —35.4→ Sc*; —53.2→ Ch ←56.8→ Iso |
| 67 | 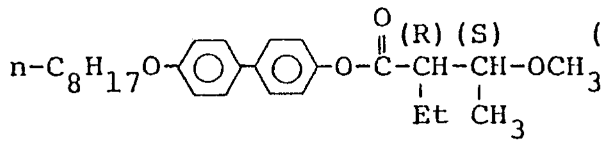 (*4) | K ←30.5→ Iso |
| 68 | 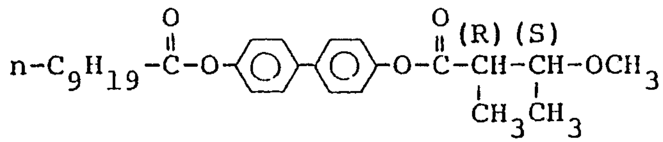 | K ←44.0→ Sc* ←52.0→ Iso |
| 69 | 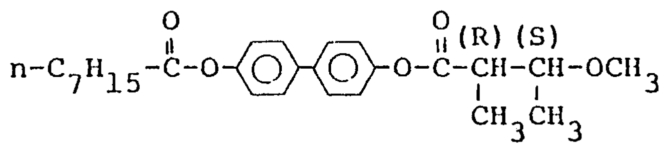 | K ⇄ (31.9 / 36.9) Sc* ←45.5→ Iso |
| 70 | 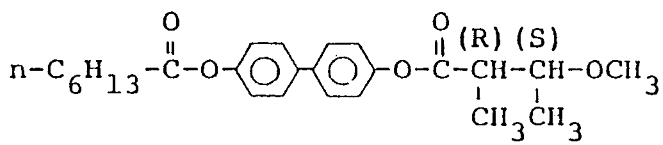 | K ⇄ (16.2 / 23.1) $S_2$ ←25.7→ $S_1$ ←31.6— —→ Sc* ←39.0→ Iso |
| 71 | 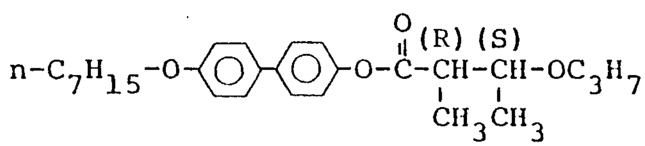 | K ⇄ (20.6 / 27.2) ($S_1$) ⇄ (24.5 / 35.0) Sc* ←58.0→ Iso |

- Cont'd -

*4 See Reference Example 4.

| | | |
|---|---|---|
| 96 | | |
| - | $C_{27}H_{38}O_4$ | : C,76.02 ; H, 8.98.<br>: C,75.98 ; H, 9.17. |
| 78(-5°C) | $C_{28}H_{38}O_5$ | : C,73.98 ; H, 8.43.<br>: C,74.01 ; H, 8.54. |
| 119 | $C_{26}H_{34}O_5$ | : C,73.21 ; H, 8.03.<br>: C,73.25 ; H, 8.18. |
| 143(-5°C) | $C_{25}H_{32}O_5$ | : C,72.79 ; H, 7.82.<br>: C,73.01 ; H, 7.82. |
| 63 | $C_{27}H_{38}O_4$ | : C,76.02 ; H, 8.98.<br>: C,76.36 ; H, 8.94. |

- Cont'd -

Table 2 (Cont'd)

| | | |
|---|---|---|
| 72 | 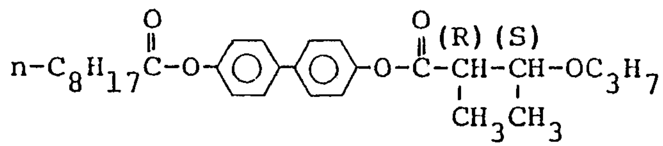 | K $\underset{32.0}{\overset{22.7}{\rightleftarrows}}$ $S_1$ $\underset{50.0}{\leftrightarrow}$ Sc* $\underset{58-64}{\leftrightarrow}$ Iso |
| 73 | 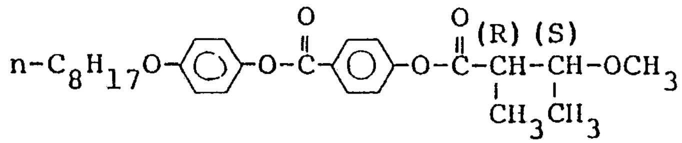 | K $\underset{34.5}{\leftrightarrow}$ Iso |
| 74 | 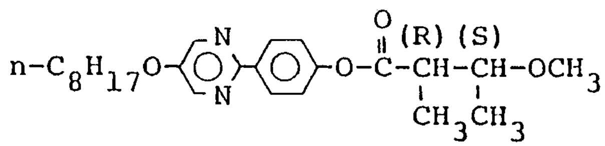 | K $\overset{6.8}{\leftarrow}$ Sc* $\overset{27.0}{\leftarrow}$ Ch $\overset{35.6}{\leftarrow}$ Iso; K $\underset{48-51.6}{\longrightarrow}$ Iso |
| 75 | 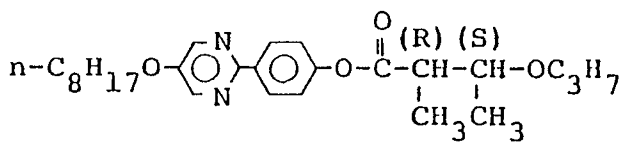 | K $\underset{6.4}{\overset{-2.0}{\rightleftarrows}}$ Sc* $\underset{35.0}{\leftrightarrow}$ $S_1$ $\underset{43.3}{\leftarrow}$ $\longrightarrow$ Ch $\underset{49.5}{\leftrightarrow}$ Iso |
| 76 | 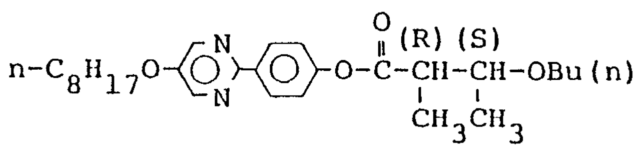 | K $\overset{21.1}{\leftarrow}$ Sc* $\overset{28.2}{\leftarrow}$ Ch $\overset{41.6}{\leftarrow}$ Iso; K $\underset{41.6}{\longrightarrow}$ Iso |
| 77 | 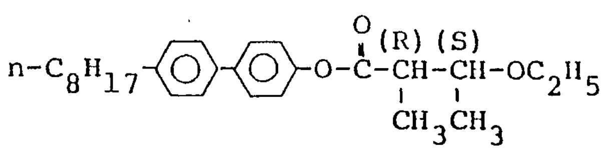 | K $\overset{22.0}{\leftarrow}$ Sc* $\overset{26.2}{\leftarrow}$ Iso; K $\underset{36.3}{\longrightarrow}$ Iso |

- Cont'd -

| | |
|---|---|
| 42 | |
| - | $C_{27}H_{36}O_6$ : C,71.03 ; H, 7.95.<br>: C,71.22 ; H, 8.05. |
| 131 | $C_{24}H_{34}N_2O_4$ : C,69.54 ; H, 8.27 ; N, 6.76.<br>: C,69.82 ; H, 8.37 ; N, 6.79. |
| 54.5 | |
| 49.2(-5°C) | $C_{27}H_{40}N_2O_4$ : C,71.02 ; H, 8.83 ; N, 6.13.<br>: C,71.31 ; H, 8.83 ; N, 6.19. |
| 55(6-7°C) | $C_{27}H_{38}O_3$ : C,78.98 ; H, 9.38.<br>: C,78.81 ; H, 9.42. |

- Cont'd -

Table 2 (Cont'd)

| | | |
|---|---|---|
| 78 | 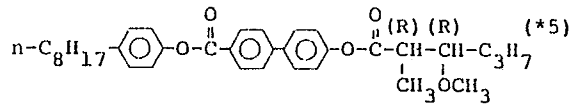 (*5) | K $\underset{45.6}{\overset{2.7}{\rightleftarrows}}$ Sc* $\overset{103.5}{\leftrightarrow}$ Ch $\overset{133.1}{\leftrightarrow}$ Iso |
| 79 | 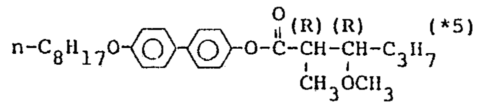 (*5) | K $\xleftarrow{8.3}$ Sc* $\xleftarrow{20.5}$ Ch $\xleftarrow{27.7}$ Iso; K $\xrightarrow{27.7}$ Iso |
| 80 | 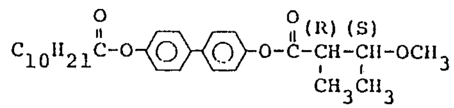 | K $\overset{46.7}{\leftrightarrow}$ Sc* $\overset{54.7}{\leftrightarrow}$ Iso |
| 81 | 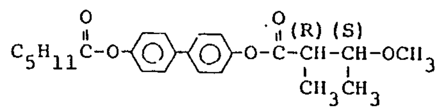 | K $\underset{17.6}{\overset{-5.0}{\rightleftarrows}}$ $S_1$ $\overset{40.2}{\leftrightarrow}$ Sc* $\overset{43.4}{\leftrightarrow}$ Iso |
| 82 | 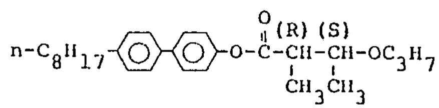 | K $\underset{35.6}{\overset{22.8}{\rightleftarrows}}$ Iso |
| 83 | 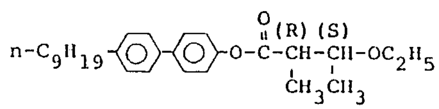 | K $\xleftarrow{-13.2}$ Sc* $\xleftarrow{12.9}$ $S_A$ $\xleftarrow{21.0}$ Iso; K $\xrightarrow{27.7}$ Iso |

- Cont'd -

*5 See Reference Example 5.

| | | |
|---|---|---|
| 7.4 | $C_{35}H_{44}O_6$ | : C,74.97 ; H, 7.91.<br>: C,75.09 ; H, 7.93. |
| 9.7(18°C) | $C_{28}H_{40}O_4$ | : C,76.33 ; H, 9.15.<br>: C,76.35 ; H, 9.23. |
| 61(-5°C) | $C_{29}H_{40}O_5$ | : C,74.33 ; H, 8.60.<br>: C,74.16 ; H, 8.71. |
| 168(41°C) | $C_{24}H_{30}O_5$ | : C,72.34 ; H, 7.59.<br>: C,72.27 ; H, 7.69. |
| – | $C_{28}H_{40}O_3$ | : C,79.20 ; H, 9.49.<br>: C,79.37 ; H, 9.60. |
| 48.2 | $C_{28}H_{40}O_3$ | : C,79.20 ; H, 9.49.<br>: C,79.34 ; H, 9.60. |

- Cont'd -

Table 2 (Cont'd)

| | | |
|---|---|---|
| 84 | 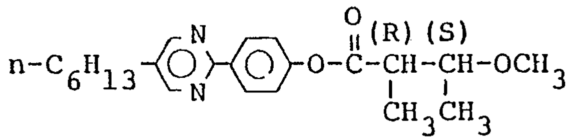 | $K \underset{41.5}{\overset{30.1}{\rightleftarrows}} Iso$ |
| 85 | 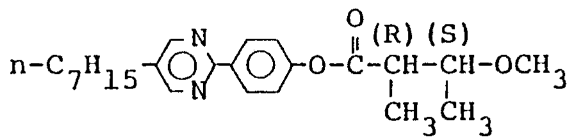 | $K \underset{54.8}{\overset{32.1}{\rightleftarrows}} Iso$ |
| 86 | 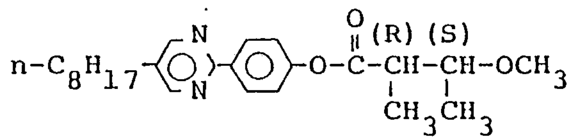 | $K \underset{50.8}{\overset{17.7}{\rightleftarrows}} Iso$ |
| 87 | 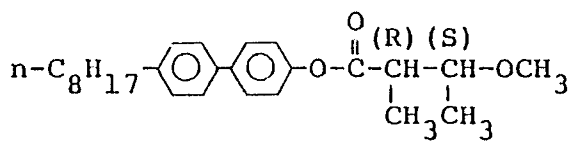 | $K \xleftarrow{-18.9} Sc^* \xleftarrow{10.3} S_A \xleftarrow{14.6} Iso$; K → Iso: 32.2 |
| 88 | 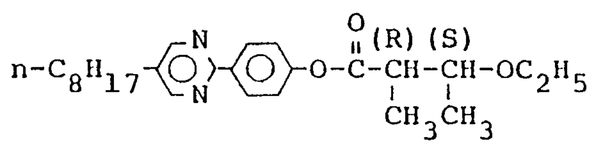 | $K \underset{23.6}{\overset{0.3}{\rightleftarrows}} Iso$ |
| 89 | 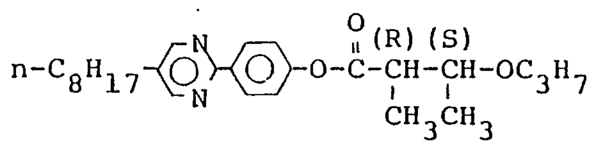 | $K \xleftarrow{-12.8} S_2 \xleftarrow{-8.1} S_1 \xleftarrow{-1.9} Iso$; K → Iso: 16.1 |
| 90 | 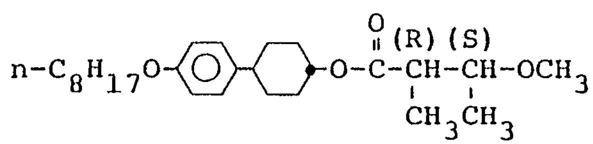 | $K \xleftarrow{-6.9} S_2 \xleftarrow{-4.6} S_1 \xleftarrow{8.3} Iso$; K → Iso: 25.2 |

| | |
|---|---|
| - | |
| - | |
| - | $C_{24}H_{34}N_2O_3$ : C,72.33 ; H, 8.60 ; N, 7.03.<br>: C,72.34 ; H, 8.68 ; N, 6.98. |
| 94 | |
| - | |
| - | |
| - | $C_{26}H_{42}O_4$ : C,74.60 ; H,10.11.<br>: C,74.78 ; H,10.12. |

Note: -: not ~~available~~ determined

Examples 90A (1) - (6)

These compounds were prepared by the procedure similar to that described above. The structures, phases, transition temperatures, values of spontaneous polarization and elemental analysis of these compounds are summarized in Table 3. The chemical structure of the compound of Example No. 96 was determined based on the various spectral data.

Table ~~2-a~~ 3

| Example No. | Structure | Phase Transition Temperature (°C) |
|---|---|---|
| 90A(1) | 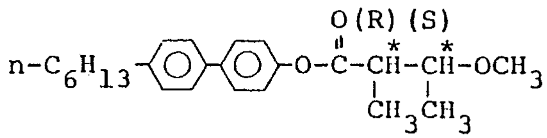 | K ←5.2→ Iso |
| 90A(2) | 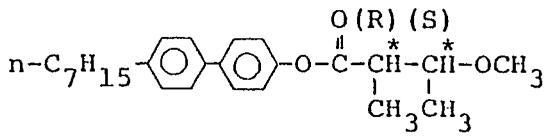 | $S_1$ ←-17.8→ Sc* ←9.0→ $S_A$ ←13.2→ Iso |
| 90A(3) | 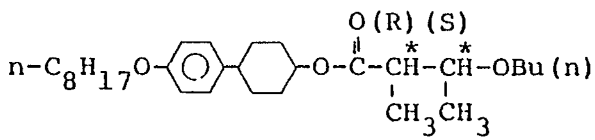 | K ←-35.8 $S_2$ ←-28.5 $S_1$ ←11.8 Iso; K —11.8→ Iso |
| 90A(4) | 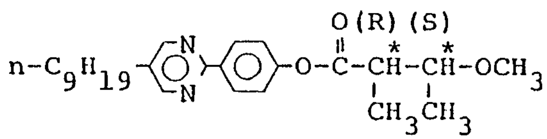 | K ←20.1 / 60.6→ Iso |
| 90A(5) | 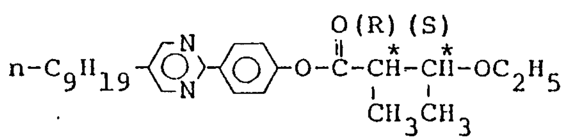 | K ←10.8 / 30.8→ Iso |
| 90A(6) | 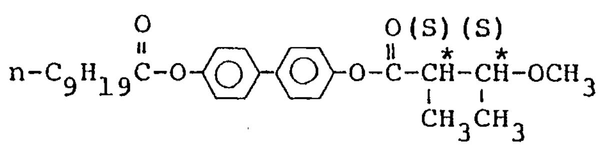 | K ←43.3→ Iso |

- Cont'd -

| Spontaneous Polarization ($nC/cm^2$) | Molecular Formula | Elemental Analysis {Calculated Value / Found Value} |
|---|---|---|
| - | $C_{24}H_{32}O_3$ | : C,78.22 ; H, 8.75.<br>: C,78.44 ; H, 8.87. |
| 127.0 | $C_{25}H_{34}O_3$ | : C,78.49 ; H, 8.96.<br>: C,78.48 ; H, 9.03. |
| - | $C_{29}H_{48}O_4$ | : C,75.61 ; H,10.50.<br>: C,75.37 ; H,10.62. |
| - | $C_{25}H_{36}N_2O_3$ | : C,72.78 ; H, 8.80.<br>: C,72.68 ; H, 8.46. |
| - | $C_{26}H_{38}N_2O_3$ | : C,73.20 ; H, 8.98.<br>: C,73.31 ; H, 9.07. |
| - | | |

The preparation of the novel dichiral compounds used in the above Examples is explained in the following Reference Examples.

Reference Example 1 Preparation of (2R, 3R)-3-hexyloxy-2-butanol

The title compound was obtained in the same procedure as in Example 1 i) except that hexyl aldehyde

was used in place of n-butyl aldehyde.

Reference Example 2 Preparation of (2R, 3S)-2-methyl-3-'propoxybutyric acid

The title compound was obtained by, according to the procedure of Example 15, carrying out etherification using n-propyl iodide in place of n-butyl iodide and then hydrolysis.

Reference Example 3 Preparation of (2S, 3S)-3-methoxy-2-methylbutyric acid

The title compound was obtained in accordance with the process described in a literature [G. Fr á ter et al., Tetrahedron, 40, 1269 (1984)]. This compound was converted to a methyl ether compound and then subjected to hydrolysis according to the procedure of Example 11 i) to obtain a corresponding (2S, 3S)-3-methoxy-2-methylbutyric acid. That is, the title compound was produced by using n-methyl iodide in place of n-butyl iodide and then subjected to hydrolysis according to the procedure of Example 15.

Reference Example 4 Preparation of (2S, 3S)-2-ethyl-3-methoxybutyric acid

Similarly to Reference Example 3, the title compound was obtained by employing the process of the literature [G. Fr á ter et al., Tetrahedron, 40, 1269 (1984)] and using ethyl iodide in place of methyl iodide. This compound was converted to a methyl ether compound and then subjected to hydrolysis according to the procedure of Example 11 to obtain a corresponding (2S, 3S)-2-ethyl-3-methoxybutyric acid.

Reference Example 5 Preparation of (2R, 3R)-3-methoxy-2-methylhexanoic acid

In accordance with the procedure of Example 9, ethyl ester of 3-oxohexanoic acid as a substrate in place of ethyl ester of 2-methyl-3-oxobutyric acid was subjected to reduction by baker's yeast to obtain ethyl ester of (3R)-3-hydroxyhexanoic acid. This compound was subjected to methylation according to the process of the literature [G. Fr á ter et al., Tetrahedron, 40, 1269 (1984)] as in Reference Example 3, to obtain ethyl ester of (2R, 3R)-3-hydroxy-2-methylhexanoic acid. This compound was converted to a methyl ether compound and then subjected to hydrolysis in accordance with the procedure of Example 11 to obtain the title compound.

Example 91 Preparation of 4-[(1R, 3R)-3-butoxy-1-methylbutoxycarbonyl]phenyl ester of 4′-octyloxy-4-biphenylcarboxylic acid (a compound of the general formula [I] in which n is 1, $R_1$ is n-$C_8H_{17}$, $R_2$ and $R_3$ are each $CH_3$, $R_4$ is n-$C_4H_9$, $Q_1$ and $Q_3$ are -O-, $Q_2$ and Y are each -C-O-, X is a single bond, and

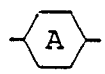 , 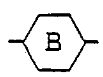 and 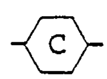 each

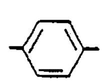 ).

i) Preparation of (2R, 4R)-4-butoxy-2-pentanol

This optically active alcohol was prepared according to the following scheme:

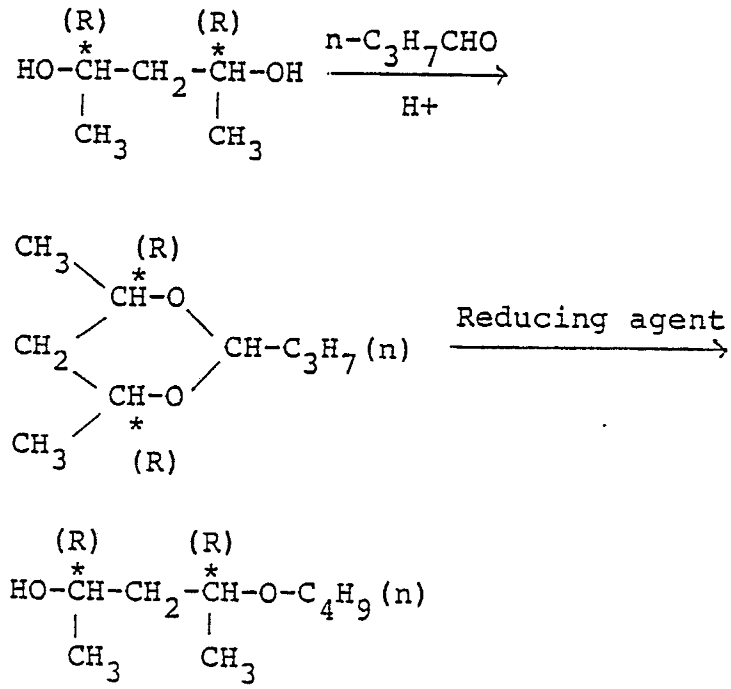


In 50 ml of benzene were dissolved 5.0 g of (2R, 4R)-(-)-2,4-pentanediol and 5.2 g of n-butyl aldehyde. Thereto was added 0.2 g of p-toluenesulfonic acid monohydrate. The mixture was refluxed for 2 hours with removing water, according to a conventional method. After cooling, the reaction mixture was mixed with 80 ml of a 10% aqueous sodium hydrogencarbonate solution. The organic layer was separated, dried and concentrated to obtain 7.7 g of a butylidene form.

12.8 g of aluminum chloride was dissolved in 100 ml of dry ether with ice cooling. Thereto was added 0.9 g of lithium aluminum hydride in small portions. Then, 7.7 g of the butylidene obtained above compound was added with ice cooling. The mixture was stirred overnight at room temperature. To the reaction mixture was added 80 ml of 10% sulfuric acid with stirring under ice cooling. The ether layer was separated and the aqueous layer was extracted with ether twice. The combined ether layer was dried and concentrated. The residue was subjected to column chromatography (Silica gel, developing solvent = chloroform) to obtain 7.10 g of (2R, 4R)-4-butoxy-2-pentanol.

The $^1$H-NMR and IR spectrum of this compound was shown below.

$^1$H-NMR (90MHz, $CDCl_3$)

δ: 0.93 (3H. t), 1.21 (3H, d), 1.22 (3H, d), 1.20-1.76 (6H, m), 2.74 (1H, br, S), 3.20-3.90 (3H, m), 4.10 (1H, m).

$IR\nu_{max}^{neat}$ $cm^{-1}$ : 3150 - 3600.

ii) Esterification

3.20 g of the (2R, 4R)-4-butoxy-2-pentanol produced in the above i) was dissolved in 50 ml of dry tetrahydrofuran. Thereto were added 1.94 g of 1,1,1,3,3,3,-hexamethyldisilazane and one drop of trimethylsilyl chloride, and the mixture was refluxed for 5 hours. The mixture was concentrated and the residue was subjected to distillation under reduced pressure (b.p. 109° C/27 mmHg) to obtain 3.14 g of a trimethylsilyl ether compound.

Separately, 2.23 g of 4-(4′-octyloxy-4-biphenylcarbonyloxy)benzoic acid was mixed with 10 ml of thionyl chloride. The mixture was stirred for 3 hours at 120° C and then excess thionyl chloride was removed by distillation to obtain a corresponding acid chloride. It was dissolved in 50 ml of dry acetonitrile. Thereto were added 1.16 g of the above obtained trimethylsilyl ether of (2R, 4R)-4-butoxy-2-pentanol and 0.07 g of zinc chloride. The mixture was refluxed for 1 hour. The mixture was concentrated and the residue was subjected to column chromatography (silica gel, developing solvent = dichloromethane) and then to recrystallization from hexane to obtain 1.21 g of the title compound. The $^1$H-NMR, IR spectrum, elementary analysis and specific rotatory power are shown below.

$^1$H-NMR (90MHz, $CDCl_3$)

δ : 0.73-1.00 (6H, m), 1.17 (3H, d), 1.37 (3H, d), 1.10-2.00 (18H, m), 3.07-3.70 (3H, m), 4.00 (2H, t), 5.33 (1H,

m), 6.80-8.30 (12H, m).
IR $\nu^{KBr}_{max}$ $cm^{-1}$ : 1730, 1710, 1600, 760.

| Elementary analysis | | | | | |
|---|---|---|---|---|---|
| Calcd. for $C_{37}H_{48}O_6$ | : | C, | 75.48 ; | H, | 8.22 |
| Found | : | C, | 75.50 ; | H, | 8.23 |

$[\alpha]^{29}_{D}$ : -48.20° (c = 0.946, chloroform)

The phase transition temperatures and spontaneous polarizations of the title compound and the compounds obtained in Examples 92-107 described below are shown in Table 3. These values may vary depending upon the purity of the compounds.

Incidentally, phase transition temperature was determined by using both DSC and a polarizing microscope with a hot stage, and spontaneous polarization was determined by the triangular wave voltage application method described in the basic version of "Liquid Crystal" co-compiled by Mitsuji Okano and Shunsuke Kobayashi and published by Baifukan in 1985.

In Table 3, K, Sc*, $S_A$, Ch and Iso are the same as defined previously, and S denotes an unidentified smectic phase.

Example 92 Preparation of 4′-octyloxy-4-biphenyl ester of 4-[(1R, 3R)-3-butoxy-1-methylbutoxycarbonyl]-benzoic acid (a compound of the general formula [I′] in which n is 1 , $R_1$ is n-$C_8H_{17}$, $R_2$ and $R_3$ are each $CH_3$, $R_4$ is n-$C_4H_9$, $Q_1$ and $Q_3$ are each -O-, $Q_2$ is -C(=O)-O-,

X is a single bond, Y is -O-C(=O)-,

and 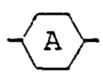 , 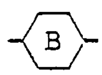 and 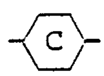 are each 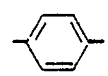 ).

1.16 g of the trimethylsilyl ether derivative of (2R, 4R)-4-butoxy-2-pentanol obtained in Example 91 ii) and 2.23 g of 4′-octyloxy-4-biphenyl ester of 4-carboxybenzoic acid were subjected to the same procedure as in Example 91 ii) to obtain 1.13 g of the title compound.

The $^1$H-NMR, IR spectrum, elementary analysis and specific rotatory power of the compound are shown below.

$^1$H-NMR (90MHz, $CDCl_3$)
δ : 0.70-1.00 (6H, m), 1.15 (3H, d), 1.40 (3H, d), 1.05-1.95 (18H, m), 3.06-3.63 (3H, m), 4.00 (2H, t), 5.38 (1H, m), 6.80-8.33 (12H, m).
IR $\nu^{KBr}_{max}$ $cm^{-1}$ : 1730, 1715, 1500, 720.

| Elementary analysis | | | | | |
|---|---|---|---|---|---|
| Calcd. for $C_{37}H_{48}O_6$ | : | C, | 75.48 ; | H, | 8.22 |
| Found | : | C, | 75.66 ; | H, | 8.19 |

$[\alpha]^{29}_{D}$ : -49.99° (c = 0.932, chloroform)

Example 93 Preparation of 4-[(1S, 3R)-3-butoxy-1-methylbutoxycarbonyl]phenyl ester of 4′-octyloxy-4-biphenylcarboxylic acid (a compound of the general formula [I′] in which n is 1, $R_1$ is n-$C_8H_{17}$, $R_2$ and $R_3$ are each $CH_3$, $R_4$ is n-$C_4H_9$, $Q_1$ and $Q_3$ are each -O-, $Q_2$ and Y are each
-C(=O)-O-, X is a single bond, and

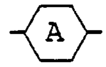 , 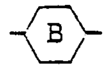 and 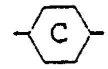

are each 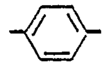 ).

0.40 g of the (2R, 4R)-4-butoxy-2-pentanol obtained in Example 91 i), 1.11 g of 4-(4′-octyloxy-4-biphenylcarboxylic acid and 0.66 g of triphenylphosphine were dissolved in 30 ml of dry tetrahydrofuran. Thereto was dropwise added 0.44 g of diethyl azodicarboxylate. The mixture was stirred for 30 minutes at room temperature. The solvent was removed by distillation. The residue was subjected to column chromatography (silica gel, developing solvent = chloroform) and then to recrystallization from hexane to obtain 0.72 g of the title compound.

The ¹H-NMR, IR spectrum, elementary analysis and specific rotation are shown below.

¹H-NMR (90 MHz, $CDCl_3$)

δ : 0.73-1.00 (6H, m), 1.18 (3H, d), 1.37 (3H, d), 1.10-2.30 (18H, m), 3.16-3.67 (3H, m), 4.00 (2H, t), 5.30 (1H, m), 6.87-8.30 (12H, m).

IR $\nu_{max}^{KBr}$ $cm^{-1}$ : 1735, 1710, 1600, 760.

| Elementary analysis | | | | | |
|---|---|---|---|---|---|
| Calcd. for $C_{37}H_{48}O_6$ | : | C, | 75.48 ; | H, | 8.22 |
| Found | : | C, | 75.66 ; | H, | 8.29 |

$[\alpha]_D^{23}$ : +12.21° (c=0.524, chloroform)

Example 94 Preparation of 4′-octyloxy-4-biphenyl ester of 4-[(1S, 3R)-3-butoxy-1-methylbutoxy]benzoic acid (a compound of the general formula [I′] in which n is 1, $R_1$ is n-$C_8H_{17}$, $R_2$ and $R_3$ are each $CH_3$, $R_4$ is n-$C_4H_9$, $Q_1$, $Q_2$ and $Q_3$ are each -O-, X is a single bond, Y is

$-O-\overset{\overset{O}{\|}}{C}-$, and

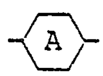 , 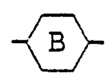 and

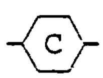 are each 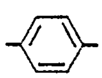 ).

0.16 g of the (2R, 4R)-4-butoxy-2-pentanol obtained in Example 91 i) and 0.21 g of 4′-octyloxy-4-biphenyl ester of 4-hydroxybenzoic acid were subjected to the same procedure as in Example 3 to obtain 0.16 g of the title compound.

The ¹H-NMR, IR spectrum, elementary analysis and specific rotatory power of the compound are shown below.

¹H-NMR (90MHz, $CDCl_3$)

δ : 0.70-1.05 (6H, m), 1.15 (3H, d), 1.40 (3H, d), 1.05-2.30 (18H, m), 3.17-3.70 (3H, m), 4.00 (2H, d), 4.68 (1H, m), 6.80-8.20 (12H, m).

IR $\nu_{max}^{KBr}$ $cm^{-1}$ : 1725, 1600, 840, 800, 760.

| Elementary analysis | | | | | |
|---|---|---|---|---|---|
| Calcd. for $C_{36}H_{48}O_5$ | : | C, | 77.11 ; | H, | 8.63 |
| Found | : | C, | 77.34 ; | H, | 8.66 |

$[\alpha]_D^{28}$ : +6.77° (c=1.078, chloroform)

Example 95 Preparation of 4-[(1S, 3R)-3-butoxy-1-methylbutoxy]phenyl ester of 4′-octyloxy-4-biphenylcarboxylic acid (a compound of the general formula [I′] in which n is 1, $R_1$ is n-$C_8H_{17}$, $R_2$ and $R_3$ are each $CH_3$, $R_4$ is n-$C_4H_9$, $Q_1$, $Q_2$ and $Q_3$ are each -O-, X is a single bond, Y is $-\overset{\overset{O}{\|}}{C}-O-$, and

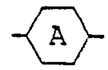 , 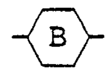

and 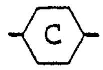 are each 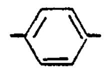 ).

0.80 g of the (2R, 4R)-4-butoxy-2-pentanol produced in Example 91 i) and 1.05 g of 4-hydroxyphenyl ester of 4′-octyloxy-4-biphenylcarboxylic acid were subjected to the same procedure as in Example 93 to obtain 0.59 g of the title compound.

The $^1$H-NMR, IR spectrum, elementary analysis and specific rotation are shown below.

$^1$H-NMR (90MHz, $CDCl_3$)

δ : 0.60-1.03 (6H, m), 1.15 (3H, d), 1.35 (3H, d), 1.03-2.30 (18H, m), 3.15-3.70 (3H, m), 4.00 (2H, t), 4.50 (1H, m), 6.70-8.25 (12H, m).

IR $\nu_{max}^{KBr}$ $cm^{-1}$ : 1725, 1600, 820, 805, 760.

| Elementary analysis | | | | | |
|---|---|---|---|---|---|
| Calcd. for $C_{36}H_{48}O_5$ | : | C, | 77.11 ; | H, | 8.63 |
| Found | : | C, | 77.18 ; | H, | 8.67 |

$[\alpha]_D^{28}$ : +2.23° (c=0.626, chloroform)

Example 96 Preparation of 4′-[(1S, 3R)-3-butoxy-1-methylbutyoxy]-4-biphenyl ester of 4octyloxybenzoic acid (a compound of the general formula [I′] in which n is 1, $R_1$ is n-$C_8H_{17}$, $R_2$ and $R_3$ are each $CH_3$, $R_4$ is $C_4H_9$, $Q_1$, $Q_2$ and $Q_3$ are each -O-, X is $-\overset{\overset{O}{\|}}{C}-O-$, Y is a single bond, and

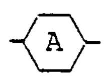 ,

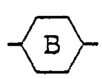 and 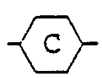 are each 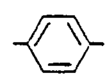 ).

0.80 g of the (2R, 4R)-4-butoxy-2-pentanol obtained in Example 91 i) and 1.05 g of 4-hydroxy-4-biphenyl ester of 4-octyloxybenzoic acid were subjected to the same procedure as in Example 93 to obtain 0.31 g of the title compound.

The $^1$H-NMR, IR spectrum, elementary analysis and specific rotation of the compound are shown below.

$^1$H-NMR (90MHz, $CDCl_3$)

δ : 0.73-1.03 (6H, m), 1.15 (3H, d), 1.35 (3H, d), 1.03-2.30 (18H, m), 3.15-3.70 (3H, m), 4.02 (2H, t), 4.56 (1H, m), 6.75-8.20 (12H, m).

IR $\nu_{max}^{KBr}$ $cm^{-1}$ : 1720, 1600, 760.

| Elementary analysis | | | | | | |
|---|---|---|---|---|---|---|
| Calcd. for $C_{36}H_{48}O_5$ | : | C, | 77.11 ; | H, | 8.63 |
| Found | : | C, | 77.31 ; | H, | 8.70 |

$[\alpha]_D^{23}$ : +2.22° (c=0.630, chloroform)

Example 97 Preparation of 4′octyloxy-4-biphenyl ester of 4-[(1R, 3R)-3-butoxy-1-methylbutoxy]benzoic acid (a compound of the general formula [I′] in which n is 1, $R_1$ is n-$C_8H_{17}$, $R_2$ and $R_3$ are each $CH_3$, $R_4$ is n-$C_4H_9$, $Q_1$, $Q_2$ and $Q_3$ are each -O-, X is a single bond, Y is

$-O-\overset{O}{\overset{\|}{C}}-$. and

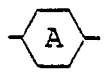 , 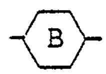 and

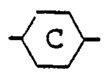 each 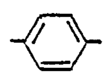 ).

i) Preparation of (2S, 4R)-4-butoxy-2-pentanol

This optically active alcohol was produced by subjecting the (2R, 4R)-4-butoxy-2-pentanol produced in Example 91 i) to the inversion of hydroxyl group according to the following scheme.

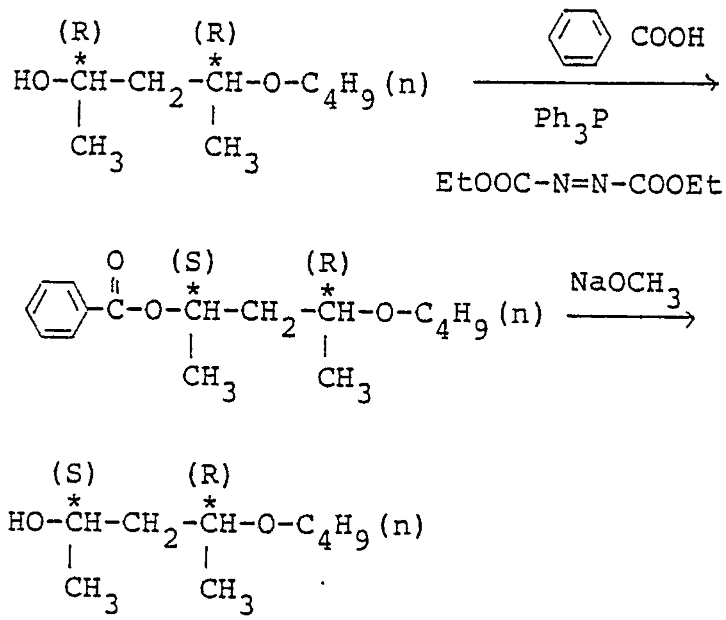


1.74 g of diethyl azodicarboxylate and 1.22 g of benzoic acid were dissolved in 10 ml of dry ether. Thereto was dropwise added a solution of 1.60 g of (2R, 4R)-4-butoxy-2-pentanol and 2.62 g of triphenylphosphine dissolved in 10 ml of dry ether. The mixture was stirred for 2 days at room temperature.

Ether was removed by distillation under reduced pressure. The residue was subjected to column chromatography (silica gel, developing solvent = chloroform) to obtain 0.82 g of (2S, 4R)-4-butoxy-2-pentyl

benzoate.

In 10 ml of methanol were dissolved 0.15 g of sodium hydroxide 0.82 g of the above obtained (2S, 4R)-4-butoxy-2-pentyl benzoate obtained above. The mixture was stirred overnight at room temperature. Methanol was removed by distillation under reduced pressure. The residue was subjected to column chromatography (silica gel, developing solvent = chloroform) to obtain 0.40 g of (2S, 4R)-4-butoxy-2-pentanol.

The $^1$H-NMR and IR spectrum of this compound are shown below.

$^1$H-NMR (90MHz, $CDCl_3$)

δ : 0.80-1.03 (3H, m), 1.14 (6H, d), 1.20-1.80 (6H, m), 3.30 (1H, m), 3.45-3.80 (2H, m), 3.90 (1H, br S), 3.95 (1H, m).

IR $\nu_{max}^{neat}$ cm$^{-1}$ : 3150-3650.

ii) Condensation

0.18 g of the (2S, 4R)-4-butoxy-2-pentanol obtained in the above i) and 0.21 g of 4′-octyloxy-4-biphenyl ester of 4-hydroxybenzoic acid were subjected to the same procedure as in Example 93 to obtain 0.12 g of the title compound. The $^1$H-NMR, IR spectrum, elementary analysis and specific rotatory power are shown below.

$^1$H-NMR (90MHz, $CDCl_3$)

δ : 0.70-1.05 (6H, m), 1.15 (3H, d), 1.33 (3H, d), 1.05-1.90 (18H, m), 3.15 (1H, m), 3.50 (2H, m), 4.00 (2H, t), 4.80 (1H, m), 6.80-8.20 (12H, m).

IR $\nu_{max}^{KBr}$ cm$^{-1}$ : 1725, 1605, 840, 800, 760.

| Elementary analysis | | | | | |
|---|---|---|---|---|---|
| Calcd. for $C_{36}H_{48}O_5$ | : | C, | 77.11 ; | H, | 8.63 |
| Found | : | C, | 76.80 ; | H, | 8.54 |

$[\alpha]_D^{25}$ : -31.25° (c = 0.016, chloroform)

Example 98 Preparation of 4-octyloxyphenyl ester of 4′-[(1S, 3R)-3-butoxy-1-methylbutoxy]-4-biphenylcarboxylic acid (a compound of the general formula [I′] in which n is 1, $R_1$ is n-$C_8H_{17}$, $R_2$ and $R_3$ are each $CH_3$, $R_4$ is n-$C_4H_9$, $Q_1$, $Q_2$ and $Q_3$ are each -O-, X is

$-O-\overset{O}{\overset{\|}{C}}-$, Y is a single bond, and

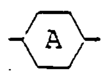 ,

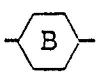 and 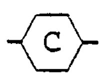 are each 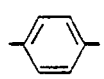 ).

0.40 g of the (2R, 4R)-4-butoxy-2-pentanol obtained in Example 91 i) and 0.52 g of 4-octyloxyphenyl ester of 4′-hydroxy-4-biphenylcarboxylic acid were subjected to the same procedure as in Example 93 to obtain 0.44 g of the title compound.

The $^1$H-NMR, IR spectrum, elementary analysis and specific rotatory power of the compound are shown below.

$^1$H-NMR (90MHz, $CDCl_3$)

δ : 0.75-1.07 (6H, m), 1.18 (3H, d), 1.39 (3H, d), 1.08-2.35 (18H, m), 3.20-3.70 (3H, m), 3.98 (2H, t), 4.63 (1H, m) 6.80-8.30 (12H, m).

IR $\nu_{max}^{KBr}$ cm$^{-1}$ : 1730, 1600, 825, 800, 760.

| Elementary analysis | | | | | |
|---|---|---|---|---|---|
| Calcd. for $C_{36}H_{48}O_5$ | : | C, | 77.11 ; | H, | 8.63 |
| Found | : | C, | 77.08 ; | H, | 8.65 |

$[\alpha]_D^{25}$ : +3.33° (c=0.03, chloroform)

Example 99 Preparation of 4-octyloxyphenyl ester of 4′-[(1S, 3R)-3-ethoxy-1-methylbutoxy-4-biphenylcarboxylic acid (a compound of the general formula [I′] in which n is 1, $R_1$ is n-$C_8H_{17}$, $R_2$ and $R_3$ are each $CH_3$, $R_4$ is $C_2H_5$, $Q_1$, $Q_2$ and $Q_3$ are each -O-, X is

$-O-\overset{O}{\overset{\|}{C}}-$, Y is a single bond, and

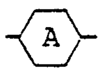 , 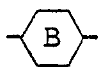 and 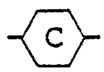 are each 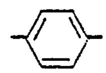 ).

i) Preparation of (2S, 4R)-4-ethoxy-2-pentanol

This optically active alcohol was produced according to the following scheme.

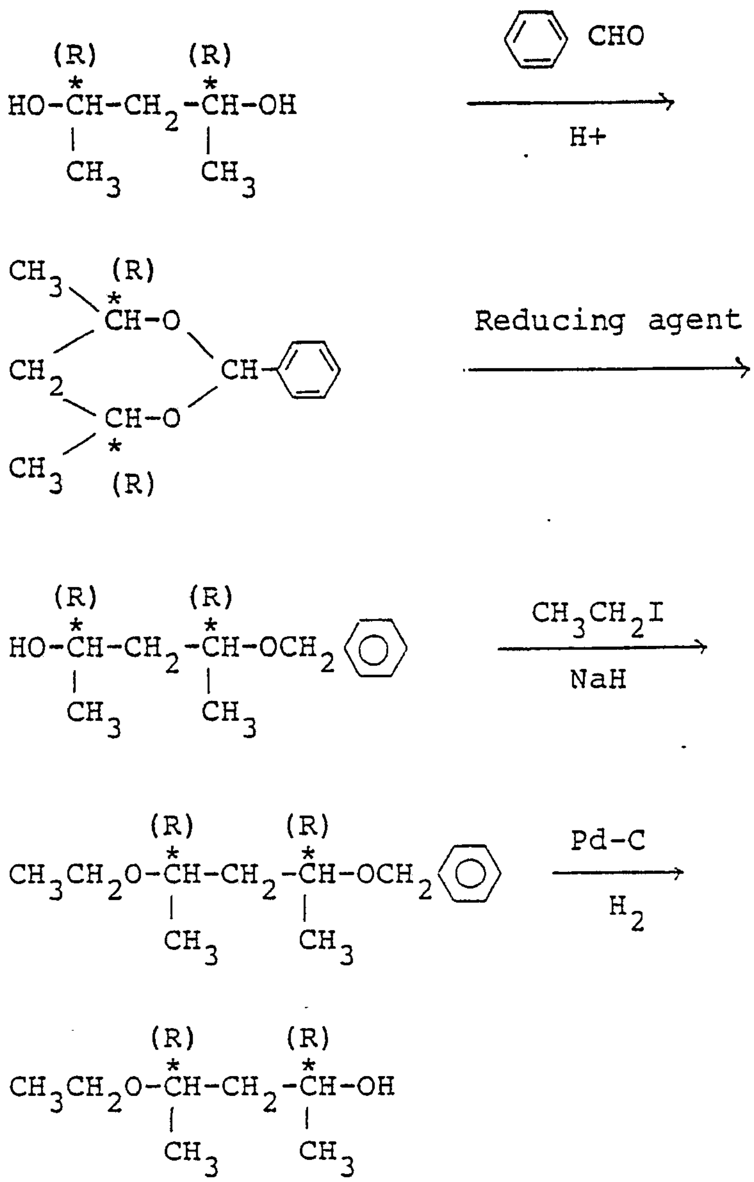


20.0 g of (2R, 4R)-(-)-pentanediol and 20.4 g of benzaldehyde were subjected to the same procedure as in Example 91 i) to obtain 39.9 g of (2R, 4R)-4-benzyloxy-2-pentanol.

In 10 ml of N,N-dimethylformamide were dissolved 2.9 g of the above obtained (2R, 4R)-4-benzyloxy-2-pentanol and 3.0 g of about 60% oily sodium hydride. The mixture was stirred for 2 hours at room temperature. 11.7 g of ethyl iodide was added thereto. The mixture was stirred for 2 hours at room temperature. 100 ml of water and 150 ml of ether were added to the mixture. The ether layer was washed with water, dried and concentrated. The residue was subjected to column chromatography (silica gel, developing solvent = chloroform) to obtain 3.20 g of (2R, 4R)-2-ethoxy-4-benzyloxypentane.

3.20 g of this (2R, 4R)-2-ethoxy-4-benzyloxypentane was dissolved in 50 ml of ethanol. 0.30 g of 10% palladium-carbon and 5 ml of 1 N hydrochloric acid were added, and hydrogenation was carried out at the room temperature at the atmospheric pressure. The catalyst was removed by filtration. The filtrate was concentrated. The residue was subjected to column chromatography (silica gel, developing solvent = chloroform) to obtain 0.91 g of (2R, 4R)-4-ethoxy-2-pentanol.

The $^1$H-NMR and IR spectrum of the compound are shown below.

$^1$H-NMR (90MHz, $CDCl_3$)

$\delta$ : 1.03-1.30 (9H, m), 1.50-1.70 (2H, m), 2.35 (1H, br S), 3.23-4.25 (4H, m).

IR $\nu_{max}^{neat}$ $cm^{-1}$ : 3150-3600.

ii) Condensation

0.33 g of the (2R, 4R)-4-ethoxy-2-pentanol obtained in the above i) and 0.52 g of 4-octyloxyphenyl ester of 4′-hydroxy-4-biphenylcarboxylic acid were subjected to the same procedure as in Example 93 to obtain 0.27 g of the title compound.

The ¹H-MNR, IR spectrum, elementary analysis and specific rotatory power of the compound are shown below.

¹H-NMR (90MHz, $CDCl_3$)

δ : 0.70-1.00 (6H, m), 1.00-2.30 (23H, m), 3.30-3.70 (3H, m), 3.97 (2H, t), 4.50-4.80 (1H, m), 6.83-8.30 (12H, m),

IR $\nu_{max}^{KBr}$ $cm^{-1}$ : 1730, 820, 760.

| Elementary analysis | | | | | |
|---|---|---|---|---|---|
| Calcd. for $C_{34}H_{44}O_5$ | : | C, | 76.66 ; | H, | 8.33 |
| Found | : | C, | 76.69 ; | H, | 8.41 |

$[\alpha]_D^{25}$ : +2.27° (c = 0.264, chloroform)

Example 100 Preparation of 4-octyloxyphenyl ester of 4′-[(1S, 3R)-3-octyloxy-1-methylbutoxy]-4-biphenylcarboxylic acid (a compound of the general formula [I′] in which n is 1, $R_1$ and $R_4$ are each n-$C_8H_{17}$, $R_2$ and $R_3$ are each $CH_3$, $Q_1$, $Q_2$ and $Q_3$ are each -O-, X is $-O-\overset{O}{\overset{\|}{C}}-$, Y is a single bond, and

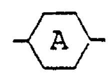 ,

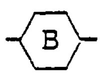 and 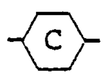 are each 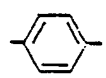 ).

i) Preparation of (2S, 4R)-4-octyloxy-2-pentanol

5.00 g of (2R, 4R)-(-)-pentanediol and 6.16 g of n-octylaldehyde were subjected to the same procedure as in Example 91 i) to obtain 8.70 g of (2R, 4R)-4-octyloxy-2-pentanol.

ii) Condensation

0.54 g of the (2R, 4R)-4-octyloxy-2-pentanol obtained in the above i) and 0.52 g of 4-octyloxyphenyl ester of 4′-hydroxy-4-biphenylcarboxylic acid were subjected to the same procedure as in Example 93 to obtain 0.49 g of the title compound.

The ¹H-NMR, IR spectrum, elementary analysis and specific rotatory power of the compound are shown below.

¹H-NMR (90MHz, $CDCl_3$)

δ : 0.75-1.00 (6H, m), 1.15 (3H, d), 1.38 (3H, d), 1.05-2.30 (26H, m), 3.17-3.70 (3H, m), 3.96 (2H, d), 4.35-4.75 (1H, m), 6.80-8.29 (12H, m),

IR $\nu_{max}^{KBr}$ $cm^{-1}$ : 1735, 1600, 825, 760.

| Elementary analysis | | | | | |
|---|---|---|---|---|---|
| Calcd. for $C_{40}H_{56}O_5$ | : | C, | 77.88 ; | H, | 9.15 |
| Found | : | C, | 78.02 ; | H, | 9.27 |

$[\alpha]_D^{25}$ : +7.92° (c = 0.20, chloroform)

Example 101 Preparation of 4-octyloxyphenyl ester of 4′-[(1S, 3R)-3-pentyloxy-1-methylbutoxy]-4-biphenylcarboxylic acid (a compound of the general formula [I′] in which n is 1, $R_1$ is n-$C_8H_{17}$, $R_2$ and $R_3$ are each $CH_3$, $R_4$ is n-$C_5H_{11}$, $Q_1$, $Q_2$ and $Q_3$ are each -O-, X is

$-O-\overset{\overset{O}{\|}}{C}-$, Y is a single bond, and

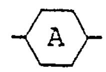 ,

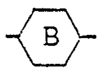 and 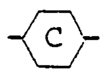 are each 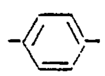 ).

i) Preparation of (2R, 4R)-4-pentyloxy-2-pentanol

2.90 g of the (2R, 4R)-4-benzyloxy-2-pentanol produced in Example 99 i) and 2.90 g of pentane iodide were subjected to the same procedure as in Example 99 i) to obtain 1.10 g of (2R, 4R)-4-pentyloxy-2-pentanol.

ii) Condensation

0.44 g of the (2R, 4R)-4-pentyloxy-2-pentanol obtained in the above i) and 0.52 g of 4-octyloxyphenyl ester of 4′-hydroxy-4-biphenylcarboxylic acid were subjected to the same procedure as in Example 93 to obtain 0.46 g of the title compound.

The elementary analysis and specific rotatory power of the compound are shown below

| Elementary analysis | | | | | |
|---|---|---|---|---|---|
| Calcd. for $C_{37}H_{50}O_5$ | : | C, | 77.31 ; | H, | 8.77 |
| Found | : | C, | 77.56 ; | H, | 8.82 |

$[\alpha]_D^{25}$ : +4.77° (c = 0.31, chloroform)

Example 102 Preparation of 4-propoxyphenyl ester of 4′-[(1S, 3R)-3-octyloxy-1-methylbutoxy]-4-biphenylcarboxylic acid (a compound of the general formula [I′] in which n is 1, $R_1$ is n-$C_3H_7$, $R_2$ and $R_3$ are each $CH_3$, $R_4$ is n-$C_8H_{17}$, $Q_1$, $Q_2$ and $Q_3$ are each -O-, X is

$-O-\overset{\overset{O}{\|}}{C}-$, Y is a single bond, and

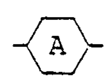 ,

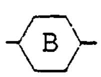 and 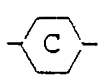 are each 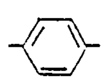 ).

0.54 g of the (2R, 4R)-4-octyloxy-2-pentanol obtained in Example 100 i) and 0.44 g of 4-propoxyphenyl ester of 4′-hydroxy-4-biphenylcarboxylic acid were subjected to the same procedure as in Example 93 to obtain 0.41 g of the title compound.

The elementary analysis and specific rotatory power of the compound are shown below.

| Elementary analysis | | | | | |
|---|---|---|---|---|---|
| Calcd. for $C_{35}H_{46}O_5$<br>Found | :<br>: | C,<br>C, | 76.89 ;<br>76.98 ; | H,<br>H, | 8.48<br>8.42 |

$[\alpha]_D^{25}$ : +5.80° (c=0.22, chloroform)

Example 103 Preparation of 4-octylphenyl ester of 4′-[(1S, 3R)-3-butoxy-1-methylbutoxy]-4-biphenylcarboxylic acid (a compound of the general formula [I′] in which n is 1, $R_1$ and $R_4$ are each n-$C_4H_9$, $R_2$ and $R_3$ are each $CH_3$, $Q_1$ is a single bond, $Q_2$ and $Q_3$ are each -O-, X is -O-C-, Y is a single bond, and

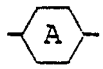 , 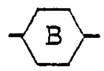

and 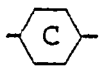 are each 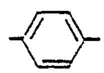 ).

0.40 g of the (2R, 4R)-4-butoxy-2-pentanol obtained in Example 91 i) and 0.42 g of 4-octylphenyl ester of 4′-hydroxy-4-biphenylcarboxylic acid were subjected to the same procedure as in Example 93 to obtain the title compound.

The elementary analysis of this compound is shown below.

| Elementary analysis | | | | | |
|---|---|---|---|---|---|
| Calcd. for $C_{36}H_{48}O_4$<br>Found | :<br>: | C,<br>C, | 79.37;<br>79.47; | H,<br>H, | 8.88<br>8.84 |

Example 104 Preparation of octyl ester of 4′-[(1S, 3R)-3-octyloxy-1-methylbutoxy]-4-biphenylcarboxylic acid (a compound of the general formula [I′] in which n is 1, $R_1$ and $R_4$ are each n-$C_8H_{17}$, $R_2$ and $R_3$ are each $CH_3$, $Q_1$ is

$-O-\overset{\overset{O}{\|}}{C}-$, $Q_2$ and $Q_3$ are each -O-, X is a single bond, and

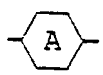 , 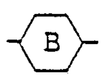 and 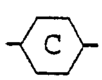 are each 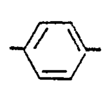 ).

0.54 g of the (2R, 4R)-4-octyloxy-2-pentanol obtained in Example 100 i) and 0.41 g of octyl ester of 4′-hydroxy-4-biphenylcarboxylic acid were subjected to the same procedure as in Example 93 to obtain 0.42 of the title compound.

The elementary analysis of this compound is shown below.

| Elementary analysis | | | | | |
|---|---|---|---|---|---|
| Calcd. for $C_{34}H_{52}O_4$ | : | C, | 77.82 ; | H, | 9.99 |
| Found | : | C, | 78.15 ; | H, | 10.33 |

Example 105 Preparation of 4-octyloxyphenyl ester of 4-[(1S, 3R)-3-octyloxy-1-methylbutoxy]benzoic acid (a compound of the general formula [I′] in which n is 1, $R_1$ and $R_4$ are each n-$C_8H_{17}$, $R_2$ and $R_3$ are each $CH_3$, $Q_1$, $Q_2$ and $Q_3$ are each -O-, X is

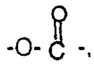 ,

and 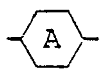 , 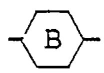 and 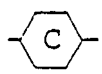 are each

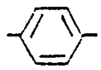 ).

0.54 g of the (2R, 4R)-4-octyloxy-2-pentanol obtained in Example 100 i) and 0.43 g of 4-octyloxyphenyl ester of 4-hydroxybenzoic acid were subjected to the same procedure as in Example 93 to obtain 0.52 g of the title compound.

The elementary analysis of this compound is shown below.

| Elementary analysis | | | | | |
|---|---|---|---|---|---|
| Calcd. for $C_{35}H_{52}O_4$ | : | C, | 75.52 ; | H, | 9.69 |
| Found | : | C, | 75.68 ; | H, | 9.84 |

Example 106 Preparation of 4-octyloxyphenyl ester of 4′-[(1S, 3R)-3-propoxy-1-methylbutoxy]-4-biphenylcarboxylic acid (a compound of the general formula [I′] in which n is 1, $R_1$ is n-$C_8H_{17}$, $R_2$ and $R_3$ are each $CH_3$, $R_4$ is n-$C_3H_7$, $Q_1$, $Q_2$ and $Q_3$ are each -O-, X is

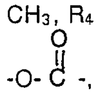 , Y is a single bond, and

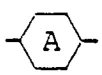 , 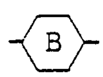 and 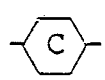 are each 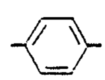 ).

i) Preparation of (2S, 4R)-4-propoxy-2-pentanol

10.0 g of (2R, 4R)-(-)-pentanediol and 7.25 g of propione aldehyde were subjected to the same procedure as in Example 91 i) to obtain 7.44 g of (2R, 4R)-4-propoxy-2-pentanol.

ii) Condensation

0.37 g of the (2R, 4R)-4-propoxy-2-pentanol obtained in the above i) and 0.52 g of 4-octyloxyphenyl ester of 4′-hydroxy-4-biphenylcarboxylic acid were subjected to the same procedure as in Example 93 to obtain 0.45 g of the title compound.

The elementary analysis of this compound is shown below.

| Elementary analysis | | | | | |
|---|---|---|---|---|---|
| Calcd. for $C_{35}H_{46}O_5$ | : | C, | 76.89 ; | H, | 8.48 |
| Found | : | C, | 76.97 ; | H, | 8.53 |

Example 107 Preparation of 4-tetradecyloxyphenyl ester of 4′-[(1S, 3R)-3-butoxy-1-methylbutoxy] 4-biphenylcarboxylic acid (a compound of the general formula [I′] in which n is 1, $R_1$ is n-$C_{14}H_{29}$, $R_2$ and $R_3$ are each $CH_3$, $R_4$ is n-$C_4H_9$, $Q_1$, $Q_2$ and $Q_3$ are each -O-, X is

O
||
-O- C -, Y is a single bond, and

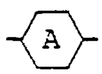 , 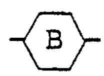 and 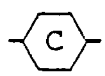 are each 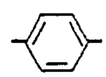 ).

0.40 g of the (2R, 4R)-4-butoxy-2-pentanol obtained in Example 91 i) and 0.63 g of 4-tetradecyloxyphenyl ester of 4′-hydroxy-4-biphenylcarboxylic acid were subjected to the same procedure as in Example 93 to obtain 0.52 g of the title compound.

The elementary analysis of the compound is shown below.

| Elementary analysis | | | | | |
|---|---|---|---|---|---|
| Calcd. for $C_{42}H_{60}O_5$ | : | C, | 78.22 ; | H, | 9.38 |
| Found | : | C, | 78.39 ; | H, | 9.44 |

The optically active compounds obtained in Examples 91-107 were determined for phases, phase transition temperatures and spontaneous polarization. The results of these measurements are shown in Table 4. In Table 4, the arrow mark (↽) denotes a phase transition temperature when cooled. The spontaneous polarization is a value at a temperature which is 10 °C lower than the phase transition temperature of $Sc^*$ - $S_A$ (Ch). The other symbols are the same as defined previously.

Examples 108-114

The compounds of Examples 108-114 were prepared in accordance with the previously described processes. Example Nos., structural formulae, phase transition temperatures and elementary analyses of these compounds are shown in Table 4, together with those of the compounds of Examples 91-107. As to the compounds containing a dichiral side chain component whose preparation has not been explained above (these compounds are marked with an asterisk in Table 4), the preparation of such compounds is described in Reference Examples 6-8 which appear after Table 4.

Table 84

| Example No. | Structural formula |
|---|---|
| 91 | 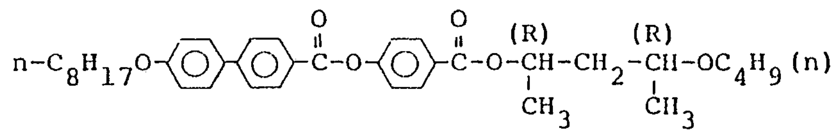 |
| 92 | 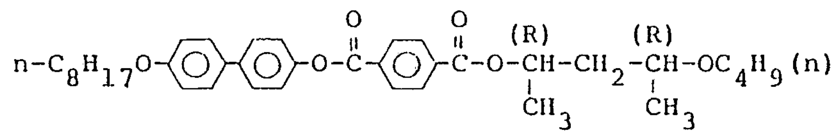 |
| 93 | 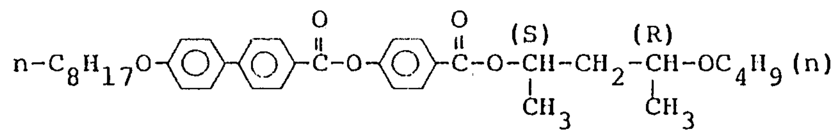 |
| 94 | 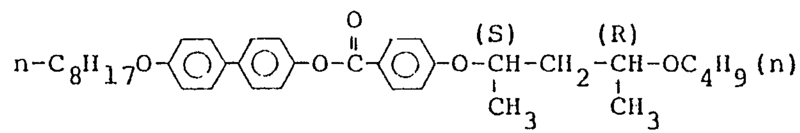 |
| 95 | 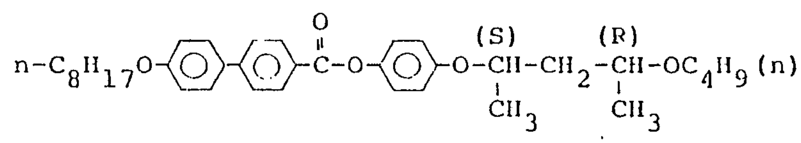 |
| 96 | 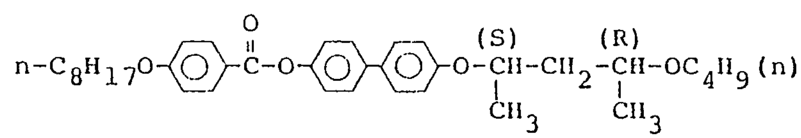 |

- Cont'd -

| Phases and Phase transition temperatures (°C) | Spontaneous Polarization ($nC/cm^2$) |
|---|---|
| $K \xleftrightarrow{68.0} Sc^* \xleftrightarrow{105.4} S_A \xleftrightarrow{126.4} Iso$ | 52 |
| $K \longleftrightarrow S \xleftrightarrow{86.0} Ch \xleftrightarrow{116.6} Iso$ | Not ~~available~~ determined |
| $K \xleftrightarrow{71.0} Sc^* \xleftrightarrow{107.8} S_A \xleftrightarrow{137.0} Iso$ | 54 |
| $K \xleftrightarrow{62.4} S_A \xleftrightarrow{64.0} Iso$ | Not ~~available~~ determined |
| $K \xleftrightarrow{72.0} Sc^* \xleftrightarrow{87.0} S_A \xleftrightarrow{111.8} Iso$ | 34 |
| $K \xleftrightarrow{55.0} S_A \xleftrightarrow{78.0} Iso$ | Not ~~available~~ determined |

- Cont'd -

Table 4 (Cont'd)

| | |
|---|---|
| 97 | 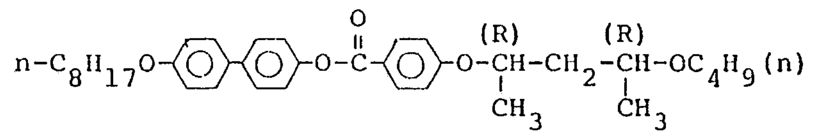 |
| 98 | 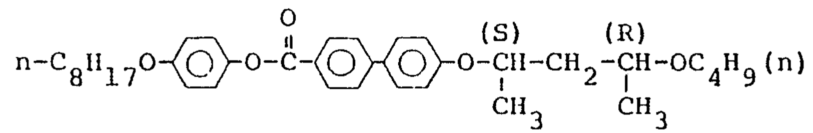 |
| 99 | 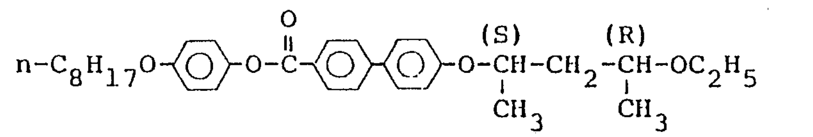 |
| 100 | 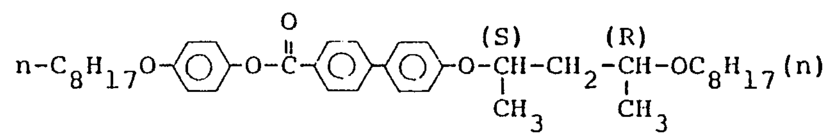 |
| 101 | 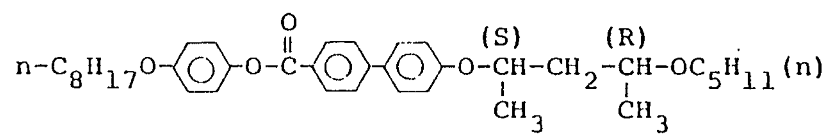 |
| 102 | 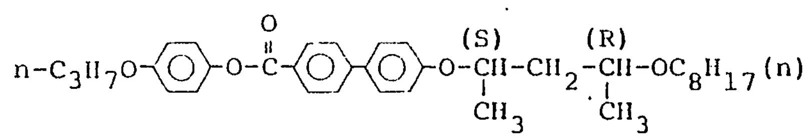 |

- Cont'd -

| | |
|---|---|
| K $\xleftrightarrow{65.1}$ Ch $\xleftrightarrow{80.6}$ Iso | not ~~available~~ determined |
| K $\xleftrightarrow{62.0}$ Iso; Iso $\xrightarrow{56.0}$ Sc* | 93 |
| K $\xleftrightarrow{52.6}$ Sc* $\xleftrightarrow{54.3}$ Ch $\xleftrightarrow{69.6}$ Iso | 74 |
| K $\xleftrightarrow{32.3}$ Sc* $\xleftrightarrow{43.0}$ Ch $\xleftrightarrow{50.6}$ Iso | 48 |
| K $\xleftrightarrow{50.2}$ Ch $\xleftrightarrow{56.4}$ Iso; Ch $\xrightarrow{50.0}$ Sc* | 95 |
| K $\xleftrightarrow{48.9}$ Iso | not ~~available~~ determined |

- Cont'd -

Table ~~3~~ 4 (Cont'd)

| | |
|---|---|
| 103 | 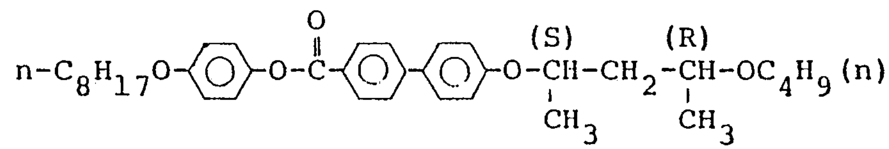 |
| 104 | 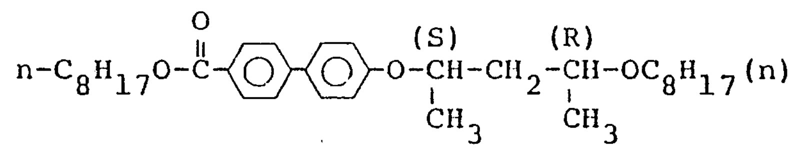 |
| 105 | 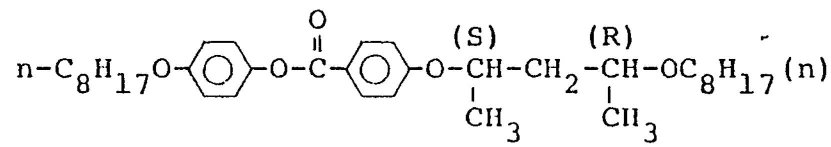 |
| 106 | 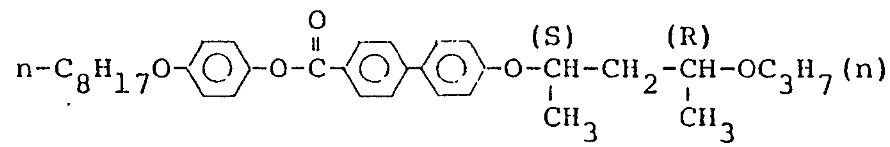 |
| 107 | 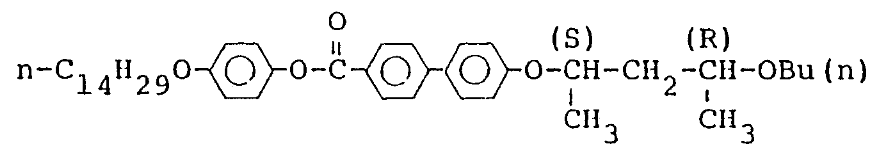 |

- Cont'd -

| | |
|---|---|
| K $\xleftrightarrow{41.9}$ Iso | not determined (Not ~~available~~) |
| K $\xleftrightarrow{9.9}$ Iso | not determined (Not ~~available~~) |
| K $\xleftrightarrow{-3.9}$ Iso | not determined (Not ~~available~~) |
| K $\xleftrightarrow{62.6}$ Ch $\xleftrightarrow{64.5}$ Iso; Ch $\swarrow$ Sc* 57.5 | not determined (Not ~~available~~) |
| K $\xleftrightarrow{64.1}$ Ch $\xleftrightarrow{66.2}$ Iso; Ch $\swarrow$ Sc* 61.9 | 120 |

Table 4 (Cont'd)

| Example No. | Structural formula | Phases and Phase transition temperatures (°C) |
|---|---|---|
| 108 | 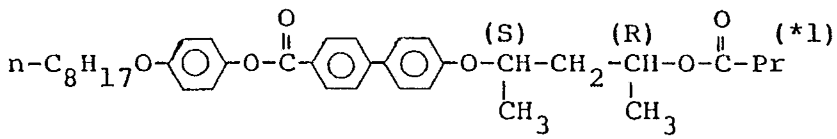 (*1) | K ← Sc* (K → Ch 56.7); Sc* ← Ch 51.8; Ch ⇄ Iso 75.5 |
| 109 | 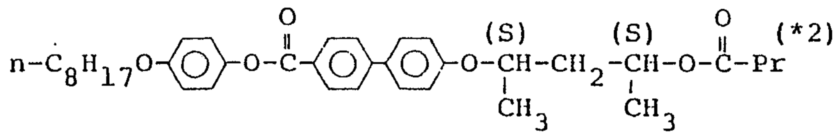 (*2) | K ← Sc* −5.9; Sc* ← Ch 29.4; K → Ch 38.3; Ch ⇄ Iso 79.0 |
| 110 | 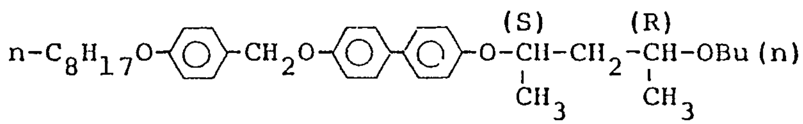 | K ⇄ Sc* 35.2 / 50.3; Sc* ⇄ Iso 75.7 |
| 111 | 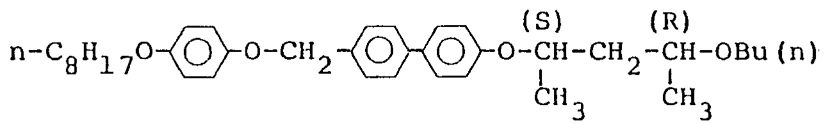 | K ⇄ Sc* 43.1 / 57.9; Sc* ⇄ $S_A$ 69.4; $S_A$ ⇄ Iso 74.7 |
| 112 | 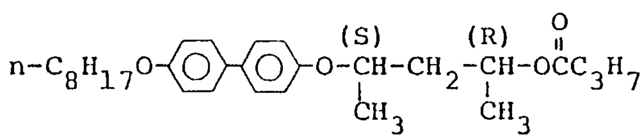 (*1) | K ⇄ Iso 25.0 |

- Cont'd -

*1 See Reference Example 6.

*2 See Reference Example 7.

| Spontaneous polarization ($nC/cm^2$) | Elemental analysis (Compositional formula, calculated value observed value) |
|---|---|
| 50 (46°C) | $C_{36}H_{46}O_6$ : C, 75.23 ; H, 8.07<br>: C, 75.38 ; H, 8.10 |
| Not ~~available~~ determined | $C_{36}H_{46}O_6$ : C, 75.23 ; H, 8.07<br>: C, 75.44 ; H, 8.15 |
| 74 | $C_{36}H_{50}O_4$ : C, 79.08 ; H, 9.22<br>: C, 78.77 ; H, 9.16 |
| 60 | $C_{36}H_{50}O_4$ : C, 79.08 ; H, 9.22<br>: C, 78.94 ; H, 9.30 |
| Not ~~available~~ determined | $C_{29}H_{42}O_4$ : C, 76.61 ; H, 9.31<br>: C, 76.71 ; H, 9.36 |

- Cont'd -

| | | |
|---|---|---|
| 113 | 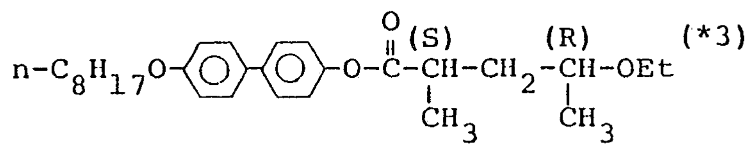 | K $\xleftarrow{5.1}$ $S_1$ $\xleftarrow{7.0}$ Iso, K $\xrightarrow{18.7}$ Iso |
| 114 | 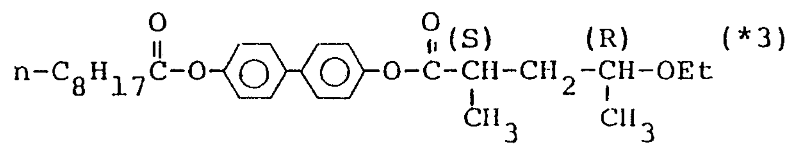 | K $\overset{\longleftarrow}{\underset{7.3}{\longrightarrow}}$ Iso |

- Cont'd -

*3 See Reference Example 3.

| | | |
|---|---|---|
| not ~~available~~ determined | $C_{28}H_{40}O_4$ | : C,76.33 ; H, 9.15<br>: C,76.16 ; H, 9.00 |
| not ~~available~~ determined | $C_{29}H_{40}O_5$ | : C,74.33 ; H, 8.60<br>: C,74.26 ; H, 8.75 |

The preparation of the novel dichiral compounds used in the above Examples is explained in the following Reference Examples.

Reference Example 6 Preparation of (1R, 3R)-3-hydroxy-1-methylbutyl ester of butyric acid

The (2R, 4R)-4-benzyloxy-2-pentanol obtained in Example 99 (a starting material) was subjected to the following scheme to obtain the title compound as a colorless oil.

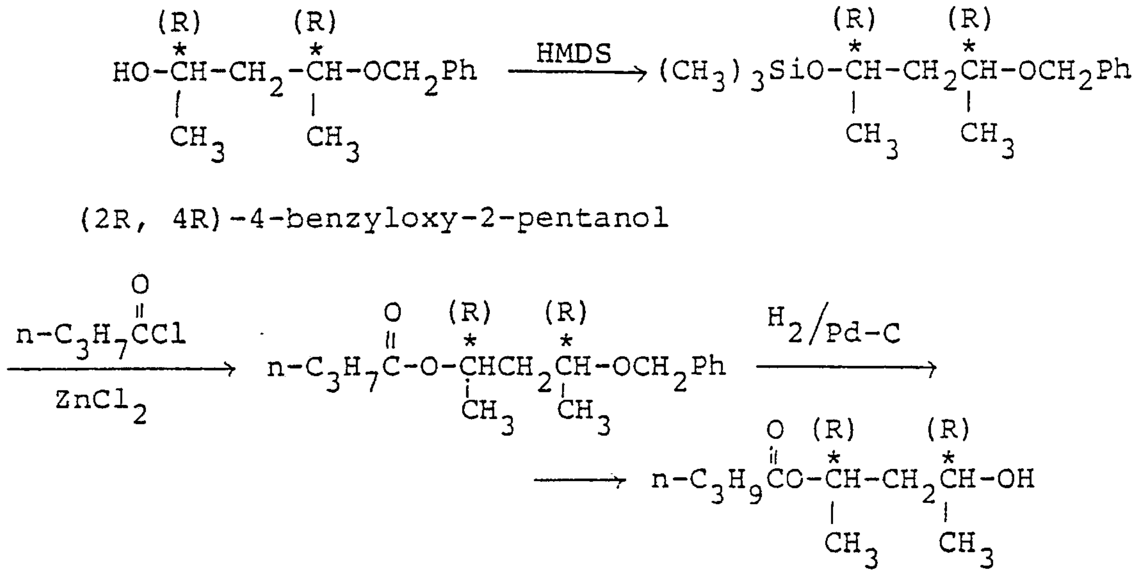


(2R, 4R)-4-benzyloxy-2-pentanol

(1R, 3R)-3-hydroxy-1-methylbutyl ester of butyric acid

Reference Example 7 Preparation of (1S, 3R)-3-hydroxy-1-methylbutyl ester of butyric acid

The (2R, 4R)-4-benzyloxy-2-pentanol obtained in Example 99 (a starting material) was subjected to the following scheme to obtain the title compound as a colorless oil.

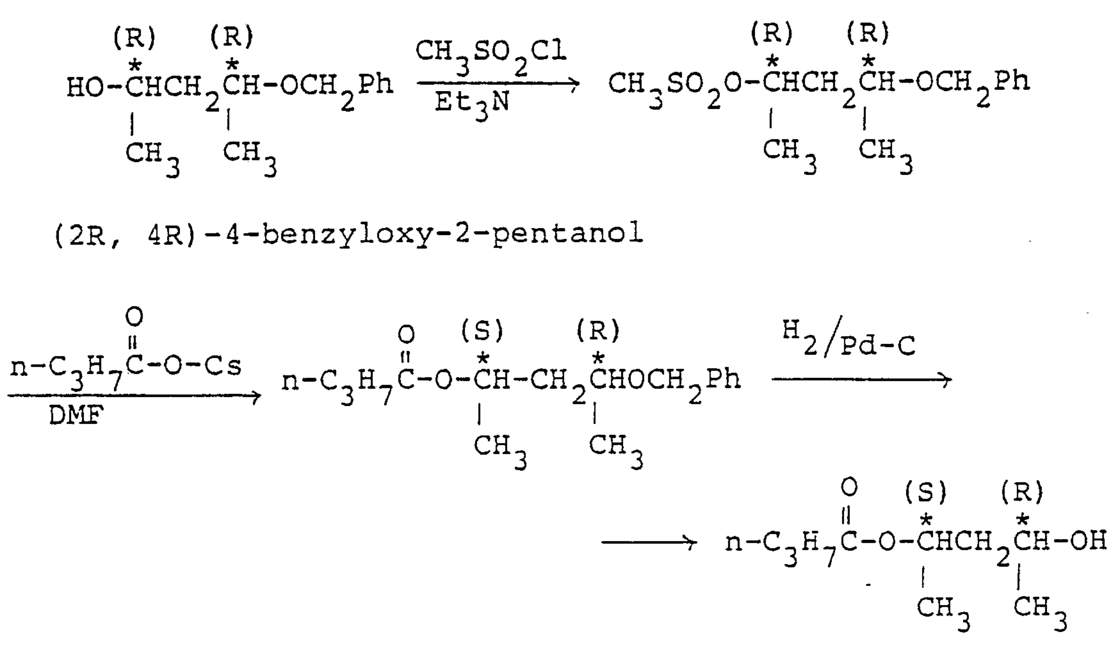


(2R, 4R)-4-benzyloxy-2-pentanol

(1S, 3R)-3-hydroxy-1-methylbutyl ester of butyric acid

Reference Example 8 Preparation of (2S, 4R)-4-ethoxy-2-methylpentanoic acid

The (2R, 4R)-4-ethoxy-2-pentanol obtained in Example 99 (a starting material) was subjected to the following scheme to obtain the title compound as a colorless oil.

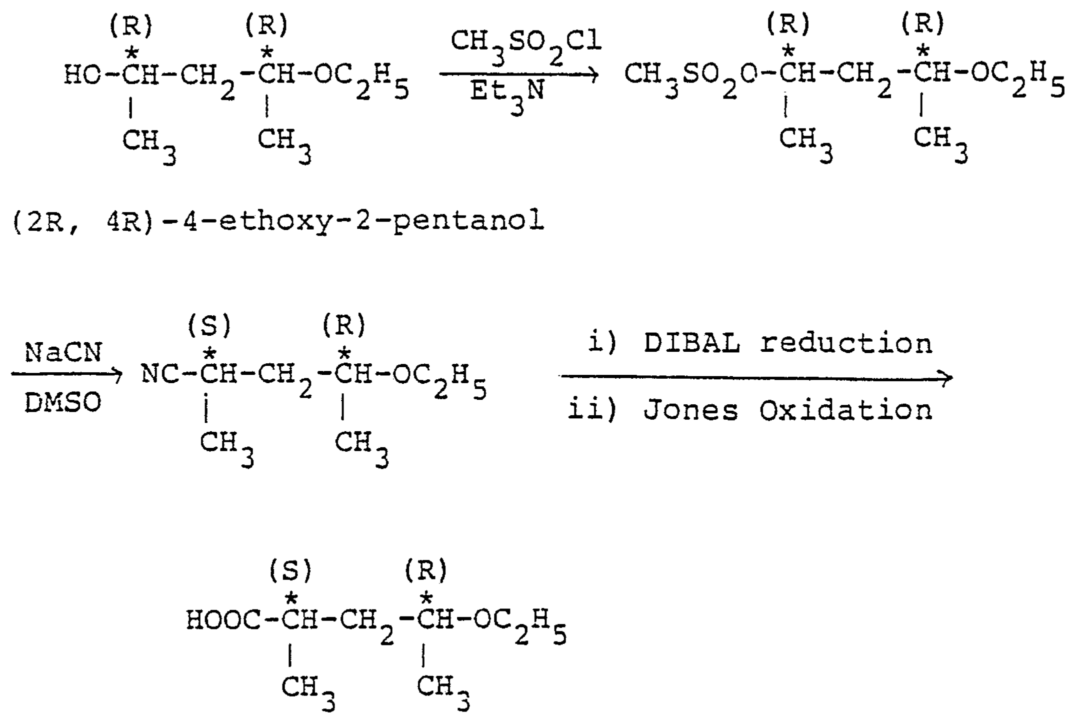


(2R, 4R)-4-ethoxy-2-pentanol

(2S, 4R)-4-ethoxy-2-methylpentanoic acid

Application Example 1

The optically active compounds of the present invention shown in Table 5 were incorporated into conventional, known ferroelectric liquid crystal compositions (hereinafter referred to as "the mother liquid crystal(s)") shown in Table 6, to obtain liquid crystal compositions containing the optically active compounds of the present invention.

Each composition and each mother liquid crystal were measured for spontaneous polarization, and the results are shown in Table 5.

Each spontaneous polarization shown in Table 5 is a value at a temperature which is 10 °C lower than the upper limit of chiral smectic C phase, and was measured according to the Sowyer-Tower method described in Japanese Journal of Applied Physics, 24, 1389-1393 (1985).

Table 5

| Mother liquid crystal | Optically active compound [I] of present invention | | Spontaneous polarization (nC/cm²) |
|---|---|---|---|
| | Example No. | Addition amount (wt.%) | |
| A | - | - | 4 |
| A | 9 | 20 | 8.5 |
| A | 6 | 10 | 9 |
| A | 14 | 10 | 10 |
| A | 18 | 20 | 12.5 |
| B | - | - | 1 |
| B | 13 | 20 | 10 |
| C | - | - | <1 |
| C | 18 | 10 | 7 |
| C | 13 | 20 | 11 |

Table 6

| Mother liquid crystal | Structural formula |
|---|---|
| A | 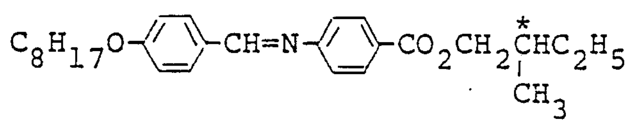 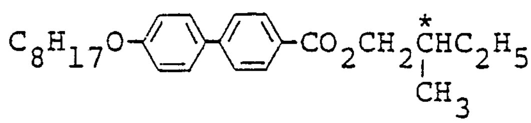 (Equimolar mixture) |
| B | 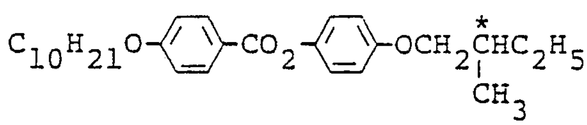 |
| | |

- Cont'd -

| | |
|---|---|
| C | 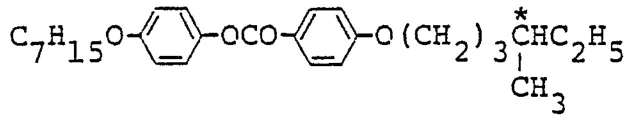 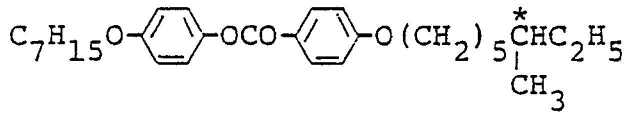 (Equimolar mixture) |

As is clear from Table 5, the incorporation of optically active compounds of the present invention into conventional, known ferroelectric liquid crystal compounds (or mixtures) significantly increased the spontaneous polarization of said liquid crystal compounds (or mixtures).

Liquid crystal devices were prepared by sealing each composition shown in the upper column of Table 5 in a cell constituting by (a) an ITO glass obtained by spin coating of a polyimide and subsequent rubbing and (b) a spacer consisting of a polyethylene terephthalate film of 6 mm in thickness. A rectangular wave (40 Vp-p) was applied to the liquid crystal devices at room temperature, and observation by a polarizing microscope was made. A clear optical contrast was observed. Similar observation was made to a liquid crystal device using the mother liquid crystal C alone, but no optical contrast was observed.

As is clear from the above, optical modulators such as display, printer head and the like can be produced by using a liquid crystal composition containing the optically active compounds of the present invention.

Application Example 2

The optically active compounds of the present invention shown in Table 7 were incorporated into a mother liquid crystal shown below, and the resulting compositions were measured for spontaneous polarization. The results are shown in Table 7. Incidentally, the spontaneous polarization is a value at a temperature which is 10 °C lower than the upper limit of Sc* phase.

Table 67

| Mother liquid crystal | Optically active compound [I] of present invention | | Spontaneous polarization (nC/cm²) |
|---|---|---|---|
| | Example No. | Addition amount (wt.%) | |
| A | 1 | 10 | 7 |
| A | 3 | 20 | 11 |
| A | 5 | 30 | 15 |
| A | 8 | 10 | 10 |
| A | 8 | 30 | 31 |
| A | 10 | 20 | 10 |
| A | - | - | <1 |

Mother liquid crystal A:

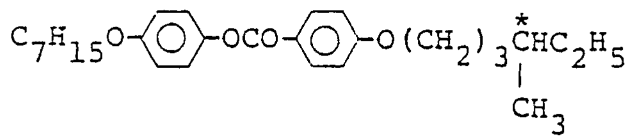

Liquid crystal devices were prepared by sealing each composition shown in the upper column of Table 7 in a cell constituted by (a) a glass with transparent electrodes obtained by spin coating of a polyimide and subsequent rubbing and (b) a spacer consisting of a polyethylene terephthalate film of 6 µm in thickness. A rectangular wave (40 Vp-p, 10 Hz) was applied to the liquid crystal devices, and observation by a polarizing microscope was made. An optical response was observed. Similar observation was made to a liquid crystal device using the mother liquid crystal A, but no optical response was obtained even by the application of a higher voltage of 50 Vp-p.

Examples of Composition Preparation

The following liquid compositions were prepared using the compound of Example 1 or the compound of Example 91. Using these compositions, liquid crystal devices as shown in Fig. 1 were prepared. All of these devices showed a good optical response when a rectangular wave (40 Vp-p, 10 Hz) was applied at room temperature. Further, each composition was mixed with 3% by weight of an anthraquinone type or azo type dichroic dye, and guest host type liquid crystal devices were prepared in the same manner as above. All of these devices showed a good optical response of guest host type. Thus, it was found that the compounds of the present invention are useful as a component of liquid crystal compositions.

Liquid crystal composition 1

| Component | Amount (mole%) |
|---|---|
| 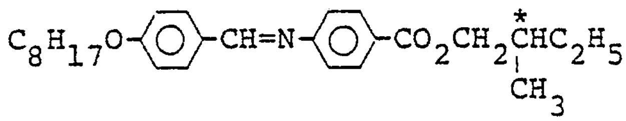 | 18 |
| 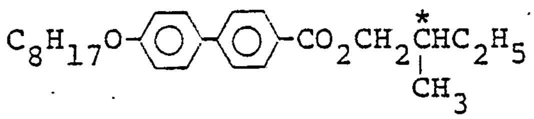 | 20 |
| 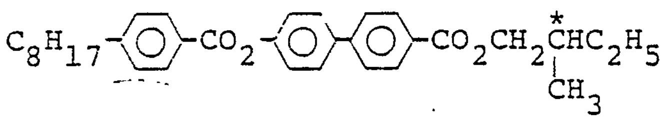 | 26 |
| 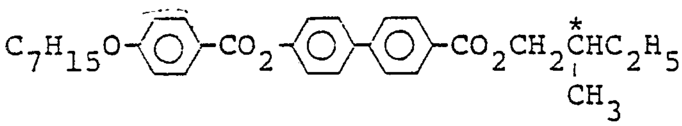 | 27 |
| Compound of Example 1 | 9 |

Liquid crystal composition 2

| Component | Amount (mole%) |
|---|---|
| 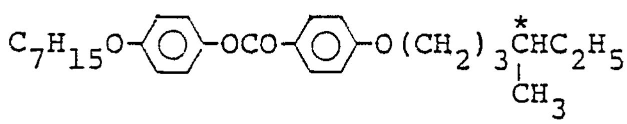 | 32 |
| 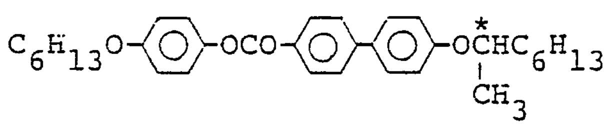 | 33 |
| 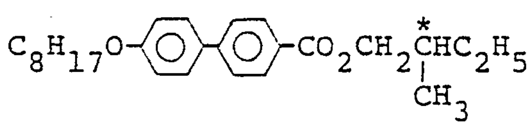 | 15 |
| Compo~~sition~~und of Example 91 | 20 |

Liquid crystal composition 3

| Component | Amount (mole%) |
|---|---|
| 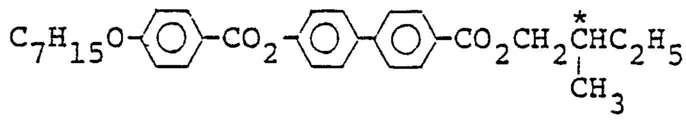 | 15 |
| Compound of Example 1 | 25 |
| 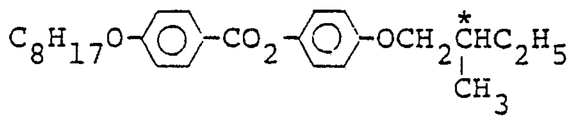 | 35 |
| 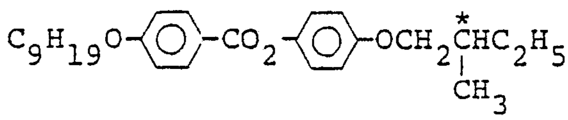 | 25 |

Liquid crystal composition 4

| Component | Amount (mole%) |
|---|---|
| 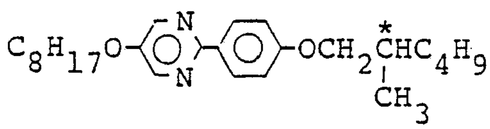 | 30 |
| 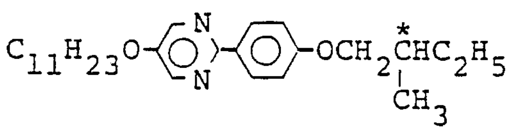 | 30 |
| 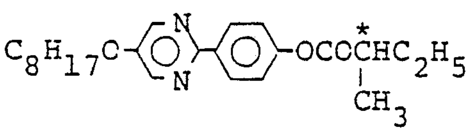 | 30 |
| Compound of Example 91 | 10 |

As described in the above Examples and Application Examples, the present invention provides ferroelectric liquid crystal compounds which have a spontaneous polarization of at least 30 $nC/cm^2$ and are stable physically and chemically. Further, the use of these liquid crystal compounds as components of liquid crystal compositions leads to liquid crystal compositions with very high spontaneous polarization.

## Claims

1. Optically active compounds represented by the general formula I,

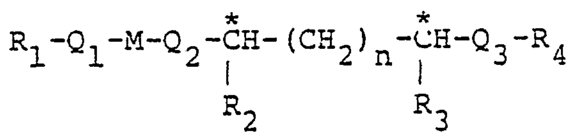

(I),

wherein are:
n 0 or 1;
$R_1$ an alkyl group of 3-14 carbon atoms;
$R_2$ and $R_3$ independently a lower alkyl group of 1-3 carbon atoms;
$R_4$ an alkyl group of 1-10 carbon atoms;
$Q_1$, $Q_2$ and $Q_3$ independently a single bond, an ether group, a carboxylic acid ester group, a carbonyl group or a carbonyldioxy group,

and M 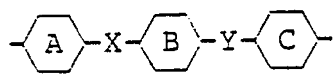 or 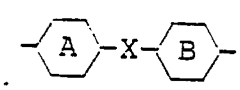 ,

wherein X and Y are independently a single bond, a carboxylic acid ester group, a methyleneoxy group or an ethylene group, and

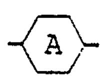 , 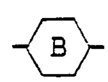 and 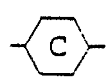

are independently a homocyclic or heterocyclic six-membered ring-1,4-diyl group which may contain 1-2 oxygen or nitrogen atoms as ring-forming atoms.

2. Optically active compounds of the general formula I according to Claim 1, wherein n is 0.

3. Optically active compounds of the general formula I according to Claim 1 or 2, wherein $Q_1$ is a single bond, an ether group or a carboxylic acid ester group, and $Q_2$ and $Q_3$ are independently an ether group or a carboxylic acid ester group.

4. Optically active compounds of the general formula I according to Claim 1, wherein n is 1.

5. Optically active compounds of the general formula I according to Claim 4, wherein $Q_1$ is a single bond, an ether group, a carboxylic acid ester group or a carbonyl group, and $Q_2$ and $Q_3$ are independently an ether group, a carboxylic acid ester group or a carbonyl group.

6. Optically active compounds of the general formula I according to one of Claims 1 to 5, wherein

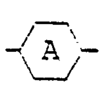 , 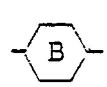 and 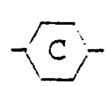 ,

which may be the same or different, are independently p-phenylene, 1,4-cyclohexylene, 2,5-(1,3-dioxane)-diyl, 2,5-pyridinediyl, 2,5-pyrimidinediyl, 2,5-(1,5-pyrazine)-diyl or 3,6-(1,2-pyridazine)-diyl.

7. Optically active compounds of the general formula I according to one of Claims 1 to 6, wherein M is

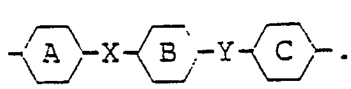 .

8. Optically active compounds of the general formula I according to one of Claims 1 to 7, wherein one of X and Y is a single bond and the other is a carboxylic acid ester group, and

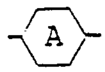 , 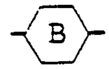 and 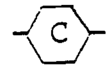

are p-phenylene groups.

9. Optically active compounds of the general formula I according to one of Claims 1 to 6, wherein M is

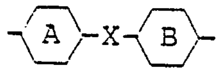 .

10. Optically active compounds of the general formula I according to Claim 9, wherein X is a single bond, and

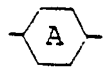 and 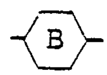

are p-phenylene groups.

11. Use of the optically active compounds of the general formula I according to one of claims 1 to 10 for electrooptical liquid crystal display devices, switching device and optical modulators.

12. Liquid crystal compositions consisting of or comprising at least one optically active compound of the general formula I according to one of Claims 1 to 10.

13. Liquid crystal optical modulators comprising a liquid crystal composition according to Claim 12.

14. Liquid crystal display devices comprising a liquid crystal composition according to Claim 12.

15. A method for preparing the optically active compounds of formula I according to one of Claims 1 to 10, characterized by the following reactions:

(A) when $Q_2$ is a

$-\overset{O}{\overset{\|}{C}}-O-$ group: esterification of a carboxylic acid of the general formula II,

$R_1-Q_1-M-COOH$ (II),

wherein $R_1$, $Q_1$ and M are as in claim 1, or a reactive derivative thereof

with an optically active dichiral secondary alcohol of the general formula III,

$$HO-\underset{R_2}{\overset{*}{C}H}-(CH_2)_n-\underset{R_3}{\overset{*}{C}H}-Q_3-R_4 \qquad (III),$$

wherein $R_2$, $R_3$, $Q_3$, $R_4$ and n are as in Claim 1,

or a reactive derivative thereof;

or

(B) when $Q_2$ is an ether group -O- :

etherification of an alcohol or a phenolic hydroxy compound of the general formula IV;

$R_1-Q_1-M-OH$ (IV),

wherein $R_1$, $Q_1$ and M are as in Claim 1,

or a reactive derivative thereof

with an optically active dichiral secondary alcohol of the above general formula III

or a reactive derivative thereof;

or

(C) when $Q_2$ is a

$-O-\overset{O}{\overset{\|}{C}}-$ group:

esterification of an optically active dichiral carboxylic acid of the general formula VI,

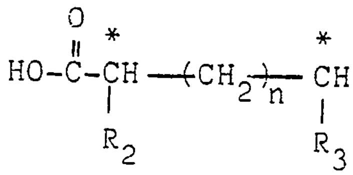 (VI),

wherein $R_2$, $R_3$ and n are as in Claim 1, or a reactive derivative thereof with a compound of the above general formula IV or a reactive derivative thereof.

16. Optically active dichiral secondary alcohols of the general formula III as defined in Claim 15.

17. Optically active dichiral carboxylic acids of the general formula VI as defined in Claim 15.

B 3719

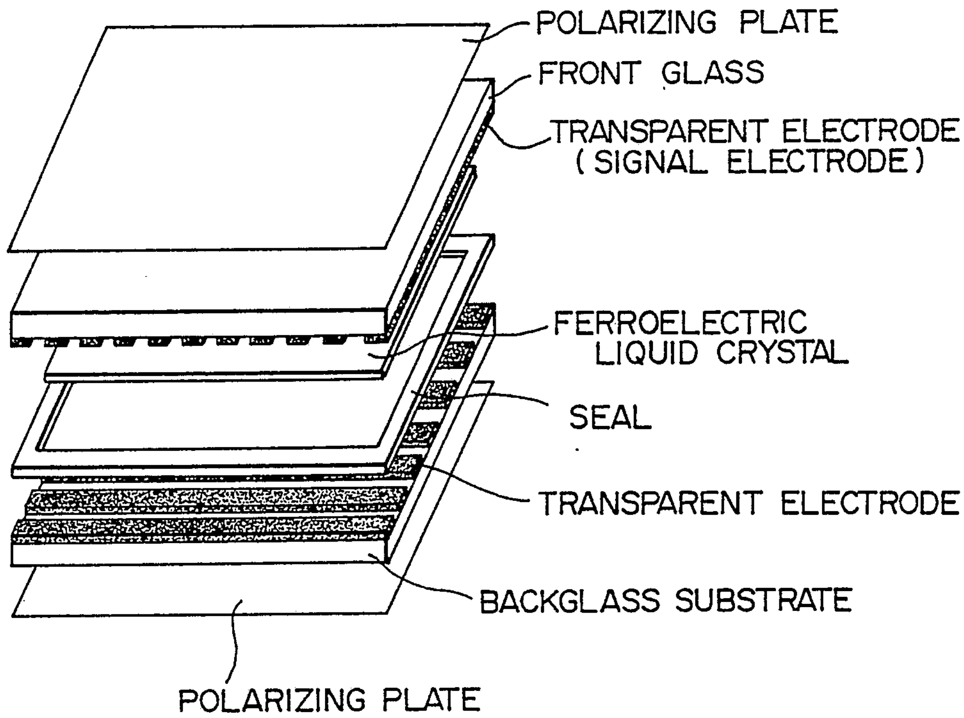
POLARIZING PLATE
FRONT GLASS
TRANSPARENT ELECTRODE
( SIGNAL ELECTRODE )
FERROELECTRIC
LIQUID CRYSTAL
SEAL
TRANSPARENT ELECTRODE
BACKGLASS SUBSTRATE
POLARIZING PLATE